# EUROPEAN PATENT SPECIFICATION

(11) **EP 1 222 289 B1**
(45) Date of publication and mention of the grant of the patent: **16.04.2008**
(21) Application number: 00986821.7
(22) Date of filing: 20.10.2000
(51) Int. Cl.: C12N 15/62, C12N 15/31, C12N 15/12, C12N 15/27, C12N 15/37, C12N 15/86, C12N 5/10, C07K 19/00, C07K 14/35, C07K 14/475, C07K 14/21, C07K 14/025, C07K 14/535, A61K 48/00, A61K 38/18, A61K 38/19, A61K 39/04, A61K 39/104, A61K 39/12

(54) **CHIMERIC IMMUNOGENIC COMPOSITIONS AND NUCLEIC ACIDS ENCODING THEM**
CHIMÄRE IMMUNOGENE ZUSAMMENSETZUNGEN UND DAFÜR KODIERENDE NUKLEINSÄURE
COMPOSITIONS CHIMERIQUES IMMUNOGENES ET ACIDES NUCLEIQUES CODANT POUR DE TELLES COMPOSITIONS

(30) Priority: 20.10.1999 US 421608; 09.02.2000 US 501097
(43) Date of publication of application: 17.07.2002
(73) Proprietor: THE JOHNS HOPKINS UNIVERSITY SCHOOL OF MEDICINE, Baltimore, MD 21205 (US)
(72) Inventor: WU, Tzyy-Choou, Stevenson, MD 21153 (US); HUNG, Chien-Fu, Brookeville, MD 20833 (US)
(74) Representative: Wright, Simon Mark
(86) International application number: PCT/US2000/041422
(87) International publication number: WO 2001/029233

(56) References cited:
- WO-A-94/04696
- WO-A-98/23735
- WO-A-99/07860
- WO-A-99/42472
- SUZUE K ET AL: "ADJUVANT-FREE HSP70 FUSION PROTEIN SYSTEM ELICITS HUMORAL AND CELLULAR IMMUNE RESPONSES TO HIV-1" JOURNAL OF IMMUNOLOGY,US,THE WILLIAMS AND WILKINS CO. BALTIMORE, vol. 156, 1996, pages 873-879, XP002070468 ISSN: 0022-1767
- CHU N R ET AL: "CANCER IMMUNOTHERAPY USING ADJUVANT-FREE, FUSION PROTEIN ENCODING M.BOVIS BCG HSP65 AND HPV16 E7" FASEB JOURNAL,FED. OF AMERICAN SOC. FOR EXPERIMENTAL BIOLOGY, BETHESDA, MD,US, vol. 12, no. 5, 20 March 1998 (1998-03-20), page A909 XP000960840 ISSN: 0892-6638
- DAVIDOFF A M ET AL: "IMMUNE RESPONSE TO P53 IS DEPENDENT UPON P53/HSP70 COMPLEXES IN BREAST CANCERS" PROCEEDINGS OF THE NATIONAL ACADEMY OF SCIENCES OF USA,US,NATIONAL ACADEMY OF SCIENCE. WASHINGTON, vol. 89, no. 8, 15 April 1992 (1992-04-15), pages 3439-3442, XP000602191 ISSN: 0027-8424
- ELSAGHIER A ET AL: "LOCALISATION OF LINEAR EPITOPES AT THE CARBOXY-TERMINAL END OF THE MYCOBACTERIAL 71 KDA HEAT SHOCK PROTEIN" MOLECULAR IMMUNOLOGY,US,ELMSFORD, NY, vol. 29, no. 9, 1992, pages 1153-1156, XP000573737 ISSN: 0161-5890
- CHIEN-HUNG CHEN ET AL.: "Enhancement of DNA vaccine potency by linkage of antigen gene to an HSP70 gene" CANCER RESEARCH, vol. 60, no. 4, 15 February 2000 (2000-02-15), pages 1035-1042, XP002163582 MD US
- CHU N R ET AL: "IMMUNOTHERAPY OF A HUMAN PAPILLOMAVIRUS (HPV) TYPE 16 E7-EXPRESSINGTUMOUR BY ADMINISTRATION OF FUSION PROTEIN COMPRISING MYCOBACTERIUMBOVIS BACILLE CALMETTE-GUERIN (BCG) HSP65 AND HPV16 E7" CLINICAL AND EXPERIMENTAL IMMUNOLOGY,OXFORD,GB, vol. 121, no. 2, August 2000 (2000-08), pages 216-225, XP000965423 ISSN: 0009-9104

## Description

### TECHNICAL FIELD

This invention generally relates to immunology and medicine. This invention provides compositions and methods for enhancing antigen-specific immune responses, e.g., cytotoxic T lymphocyte (CTL) responses, using chimeric molecules comprising a heat shock protein (HSP), and an antigenic polypeptide, such as a tumor-associated antigen or an antigen derived from a pathogen. Thus, one embodiment of the invention relates to DNA vaccines.

### BACKGROUND

Antigen-specific immunotherapy has recently emerged as an approach for controlling cancer because it is capable of developing specific immunity against neoplastic cells while not attacking normal cells. DNA vaccination differs from traditional vaccination in that DNA encoding an antigen (not the antigen itself) is injected into the subject. The production of the antigen, i.e., expression of the antigen encoded by DNA in the vaccine, takes place in the body of the vaccinated individual. However, conventional DNA vaccines have limitations. For example, a major drawback of most DNA vaccines is their potency. WO 99/42472 discloses heat shock proteins in fusion DNA constructs with one or more epitopes for stimulating an immune response in an animal.

### SUMMARY

The invention provides an immunogenic composition comprising a nucleic acid which encodes a chimeric polypeptide comprising (a) a first polypeptide domain, that is a carboxy terminal fragment or domain of a heat shock protein (HSP) which has chaperone activity; (b a second polypeptide domain comprising an antigenic polypeptide; wherein the nucleic acid encoding the second polypeptide domain is 5' to the nucleic acid encoding the first polypeptide domain, and the nucleic acid can stimulate or enhance a cellular immune response to the antigenic polypeptide on administration to a mammal. The invention relates to a nucleic acid encoding a chimeric polypeptide comprising a first polypeptide domain comprising a carboxy terminal fragment of a heat shock protein (HSP), and a second polypeptide domain comprising an antigenic polypeptide. The fusion protein product of the chimeric nucleic acid can also have several domains, including a carboxy terminal fragment of an HSP domain.

Other domains can also be added, e.g., for aiding in purifying the fusion polypeptide or identifying the polypeptide *in situ,* and the like. The nucleic acid encoding the second polypeptide domain is 5' to the nucleic acid encoding the first polypeptide domain. Thus, the fusion protein product of this chimeric nucleic acid has the antigenic polypeptide amino terminal to the first polypeptide domain.

In one embodiment, the carboxy terminal fragment of the heat shock protein (HSP) has chaperone activity, such as peptide chaperone activity. The heat shock protein (HSP) (or its nucleic acid coding sequence) can be derived from any organism, including, e.g., human or bacteria HSPs. The HSP can comprise a heat shock protein 70 (HSP 70), such as an HSP70 derived from a *Mycobacterium,* such as a *Mycobacterium tuberculosis.* The heat shock protein (HSP) can comprise a sequence as set forth by residues 312 to 625 of SEQ ID NO:9, or a sequence as set forth by about residue 517 to about residue 625 of SEQ ID NO:9, or a functional equivalent thereof.

In one embodiment, the antigenic polypeptide comprises an MHC Class I-binding peptide epitope. The MHC class 1-binding peptide epitope can be between about 8 amino acid residues and about 11, or about 15, or about 25, or about 50, amino acid residues in length. The antigenic polypeptide can be derived from any pathogen or microorganism. In alternative embodiments, antigenic polypeptide is derived from a virus, such as a human virus, e.g., a human papovavirus. The papovavirus can be a human papilloma virus (HPV), such as a human papilloma virus-16 (HPV-16). The antigenic polypeptide can comprise a human papilloma virus E6 polypeptide or human papilloma virus E7 polypeptide. The virus can also be a lentivirus, such as a human immunodeficiency virus (HIV), such as HIV-1.

Other pathogens from which the antigenic polypeptide can be derived include malaria, bovine viral diarrhea virus, cytomegalovirus, encephalitis virus, hepatitis virus, herpes simplex virus or influenza virus, to name just a few.

The antigenic polypeptide also can be a tumor-specific or tumor-associated polypeptide. The tumor-specific or tumor-associated polypeptide can comprise an antigen expressed on the surface of a tumor cell. The tumor-specific or tumor-associated polypeptide can be a mutant p53, a MAGE-1 or a MAGE-3.

In one embodiment, the nucleic acid encodes a chimeric polypeptide further comprising a third polypeptide domain comprising a cytoplasmic translocation polypeptide domain. Thus, the invention provides a nucleic acid encoding a chimeric polypeptide comprising a first polypeptide domain comprising a carboxy terminal fragment of a heat shock protein (HSP), a second polypeptide domain comprising an antigenic polypeptide, and a third polypeptide domain comprising a cytoplasmic translocation polypeptide domain. As noted above, the domains can be present in the chimeric nucleic acid or polypeptide in any order, with the proviso that the nucleic acid encoding the second polypeptide domain is 5' to the nucleic acid encoding the first polypeptide domain.

The cytoplasmic translocation polypeptide domain can be derived from any source, e.g., human, bacterial, and the like. The cytoplasmic translocation polypeptide domain can comprise a cytoplasmic translocation domain of a *Pseudomonas* exotoxin A (ETA). The cytoplasmic translocation domain of a *Pseudomonas* exotoxin A (ETA) can comprise a sequence as set forth by about residue 253 to about residue 364 of SEQ ID NO: 3. The cytoplasmic translocation polypeptide domain can comprise a cytoplasmic translocation domain of a pathogen toxin, such as bacterial toxin, e.g., a toxin derived from *Diptheria, Clostridium, Botulinum, Bacillus, Yersinia, Vibrio cholerae,* or *Bordetella pertussis.* The nucleic acid encoding the third polypeptide domain can be located 5' to the nucleic acid encoding the first or the second domain, or is located between the nucleic acids encoding the first or the second domains, or is located 3' to the nucleic acid encoding the first or the second domain.

The nucleic acid can further comprise a regulatory nucleic acid sequence, such as a transcriptional (e.g., promoter or enhancer) or a translational regulatory sequence.

The invention relates to an expression cassette (e.g., a plasmid, a vector, a virus) comprising a chimeric nucleic acid of the invention, including, e.g., a nucleic acid encoding a chimeric polypeptide comprising a first polypeptide domain comprising a carboxy terminal fragment of a heat shock protein (HSP); and, a second polypeptide domain comprising an antigenic polypeptide. The expression cassette comprises at least a promoter and a coding sequence. The promoter can be constitutive, inducible, tissue specific, and the like. The expression cassette can also comprise a coding sequence for a cytoplasmic translocation domain. The expression cassette can also comprise a self-replicating RNA replicon, such as a Sindbis virus self-replicating RNA vector, e.g., a self-replicating RNA vector SINrep5.

The invention relates to a transformed cell comprising a nucleic acid encoding a chimeric polypeptide comprising a first polypeptide domain comprising a carboxy terminal fragment of a heat shock protein (HSP) and, a second polypeptide domain comprising an antigenic polypeptide. This nucleic acid can also comprise a coding sequence for a cytoplasmic tranlocation domain.

The invention relates to a chimeric polypeptide comprising a first polypeptide domain comprising a carboxy terminal fragment of a heat shock protein (HSP) and, a second polypeptide domain comprising an antigenic polypeptide. The fusion protein can be multi-domained, analogous to the chimeric nucleic acid of the invention. The fusion protein can also comprise a cytoplasmic tranlocation domain. The first polypeptide domain can be non-covalently linked to the second polypeptide domain. Alternatively, the first polypeptide domain can be covalently linked to the second polypeptide domain, e.g., it can be a recombinant protein. If the chimeric polypeptide is multi-domained, any combination of linking/ fusion proteins schemes be devised.

The invention relates to a pharmaceutical composition comprising a nucleic acid encoding a chimeric polypeptide (a chimeric nucleic acid of the invention), an expression cassette (e.g., a plasmid, vector, virus) comprising a nucleic acid encoding a chimeric polypeptide of the invention, or a chimeric polypeptide, wherein the chimeric polypeptide comprises a carboxy terminal fragment of a heat shock protein (HSP) and, a second polypeptide domain comprising an antigenic polypeptide; and, a pharmaceutically acceptable excipient. The fusion protein can also comprise a cytoplasmic tranlocation domain.

The pharmaceutical composition can comprise any formulation, e.g., it can be formulated with phospholipids to form liposomes. It can be suitable, e.g., for oral or parenteral administration or ballistic administration and the like. It can be in any appropriate dosage or form, e.g., a tablet, a liquid, an inhalant, a particle for injection/ ballistics and the like.

The invention relates to a DNA vaccine comprising a nucleic acid encoding a chimeric polypeptide, a vector comprising a nucleic acid encoding a chimeric polypeptide, or a chimeric polypeptide, wherein the chimeric polypeptide comprises a carboxy terminal fragment of a heat shock protein (HSP); and, a second polypeptide domain comprising an antigenic polypeptide; and, a pharmaceutically acceptable excipient. The fusion protein can also comprise a cytoplasmic tranlocation domain.

The invention relates to a particle comprising a nucleic acid encoding a chimeric polypeptide or a vector comprising a nucleic acid encoding a chimeric polypeptide, wherein the chimeric polypeptide comprises a carboxy terminal fragment of a heat shock protein (HSP); and, a second polypeptide domain comprising an antigenic polypeptide. The fusion protein can also comprise a cytoplasmic tranlocation domain. The particle can comprise any material suitable for particle bombardment, e.g., gold.

The invention relates to a method of inducing an immune response, the method comprising administering an effective amount of a composition comprising a nucleic acid encoding a chimeric polypeptide, a vector comprising a nucleic acid encoding a chimeric polypeptide, or a chimeric polypeptide, wherein the chimeric polypeptide comprises a carboxy terminal fragment of a heat shock protein (HSP); and, a second polypeptide domain comprising an antigenic polypeptide, or a DNA vaccine comprising the nucleic acid, the vector, the chimeric polypeptide or a combination thereof, wherein administration of the composition in the effective amount induces an immune response. The fusion protein can also comprise a cytoplasmic tranlocation domain. The method can induce an immune response comprising a cellular response, such as a predominantly T cell response, e.g., a predominantly cytotoxic T cell response. In this method, the nucleic acid or vector encoding the chimeric polypeptide can be administered as a naked DNA molecule. The nucleic acid of the invention, e.g., the naked DNA, can be administered by a gene gun or equivalent. Alternatively, in the method, the composition can be administered as a liposomal formulation. The composition can be administered intradermally, by inhalant, and the like.

The invention also relates to a method of vaccinating a mammal against infection by a pathogen, the method comprising administering an effective amount of a composition comprising a nucleic acid encoding a chimeric polypeptide, a vector comprising a nucleic acid encoding a chimeric polypeptide, or a chimeric polypeptide, wherein the chimeric polypeptide comprises a carboxy terminal fragment of a heat shock protein (HSP); and, a second polypeptide domain comprising an antigenic polypeptide derived from a pathogen, or a DNA vaccine comprising the nucleic acid, the vector, the chimeric polypeptide or a combination thereof, wherein administration of the composition induces an immune response to the pathogen.

The invention also relates to a method of vaccinating a mammal against a tumor antigen, the method comprising administering an effective amount of a composition comprising a nucleic acid encoding a chimeric polypeptide, a vector comprising a nucleic acid encoding a chimeric polypeptide, or a chimeric polypeptide, wherein the chimeric polypeptide comprises a carboxy terminal fragment of a heat shock protein (HSP); and, a second polypeptide domain comprising a tumor antigen, or a DNA vaccine comprising the nucleic acid, the vector, the chimeric polypeptide or a combination thereof, wherein administration of the composition induces an immune response to the tumor antigen.

The invention also provides a use of a nucleic acid, a composition or a particle in the manufacture of a medicament for inducing an immune response. The medicament comprises a nucleic acid encoding a chimeric polypeptide, a vector comprising a nucleic acid encoding a chimeric polypeptide, a chimeric polypeptide, or a combination thereof, wherein the chimeric polypeptide comprises a carboxy terminal fragment of a heat shock protein (HSP); and, a second polypeptide domain comprising a tumor antigen, and a pharmaceutically acceptable excipient.

The details of one or more embodiments of the invention are set forth in the accompanying drawings and the description below. Other features, objects, and advantages of the invention will be apparent from the description and drawings, and from the claims.

### DESCRIPTION OF DRAWINGS

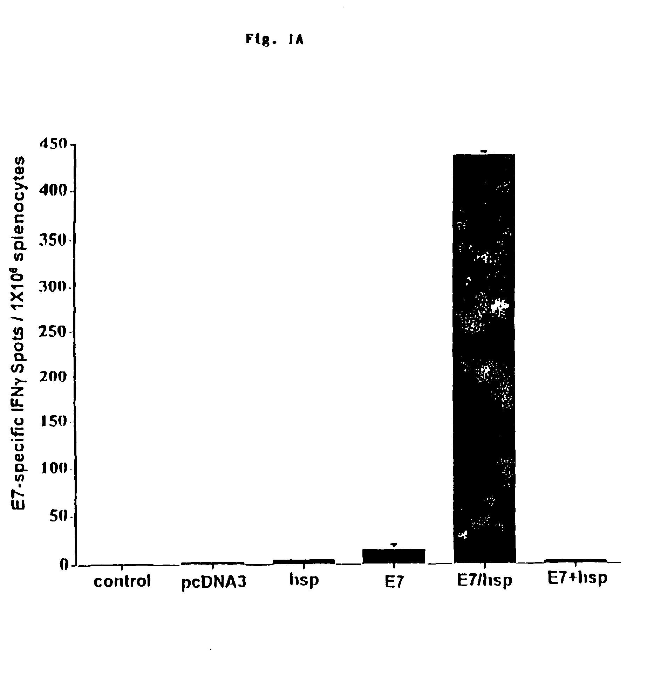

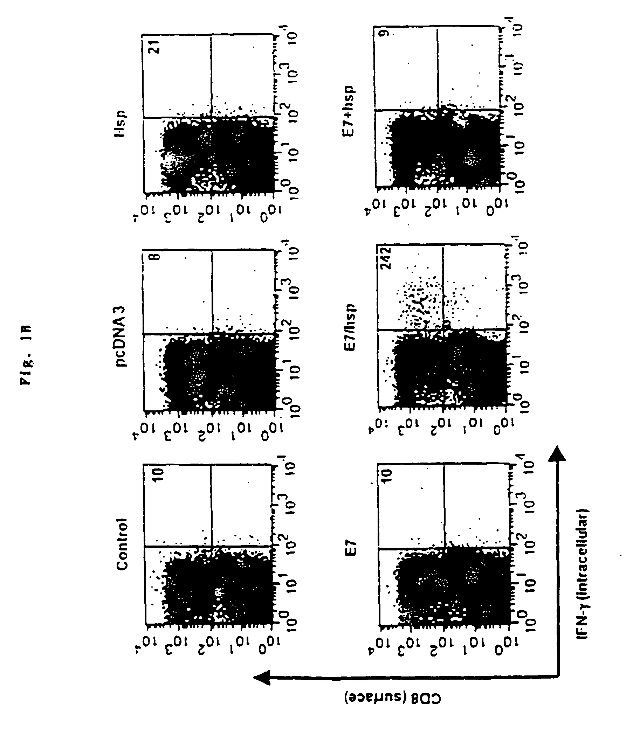

Figure 1A is a bar graph showing the results of an ELISPOT assay. The number of IFN-γ producing E7-specific CD8⁺ T cell precursors was determined using the ELISPOT assay. The spot numbers were the mean of triplicates SE in each vaccinated group. Mice vaccinated with E7-HSP70 DNA generated the highest IFN-γ⁺ spot numbers. The data shown represent E7-specific spot-forming cells (subtracting the spot numbers detected in the absence of the E7 CTL peptide). Figure 1B is a diagram showing the results of a flow cytometry analysis. Splenocytes from vaccinated mice were cultured *in vitro* with an E7 peptide (residues 49-57; RAHYNIVTF; SEQ ID NO:5) overnight and were stained for both CD8 and intracellular IFN-γ. The number of IFN-γ secreting CD8⁺ T cell precursors in mice immunized with various recombinant DNA vaccines was analyzed by standard flow cytometry. Mice vaccinated with E7-HSP70 DNA generated the highest IFN-γ⁺ CD8⁺ double positive T cells. The numbers of CD8⁺ IFN-+γ⁺ double positive T cell in 3 x 10⁵ splenocytes are indicated in the upper right corner. The data shown in Figs. 1A-B indicate the frequency of E7-specific CD8⁺ T cell precursors in C57BL/6 mice immunized with E7-HSP70 DNA vaccines. C57BL/6 mice were immunized with empty plasmid (pcDNA3), HSP70 DNA (HSP), E7 DNA (E7), E7-HSP70 DNA (E7/HSP) or E7 DNA mixed with HSP70 DNA (E7+HSP) via gene gun, or received no vaccination. For vaccinated mice, 2 µg DNA /mouse was given twice. Splenocytes were harvested 10 days after the last DNA vaccination.

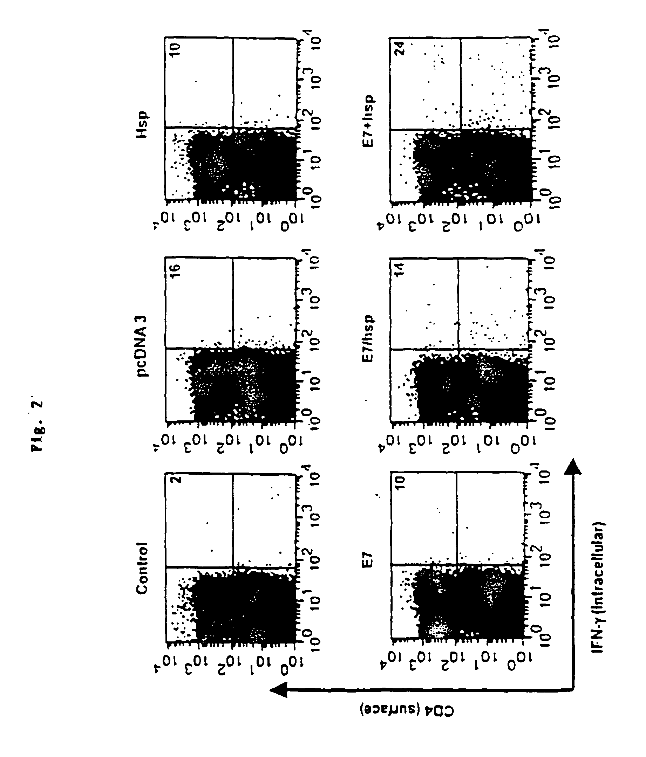

Figure 2 is a diagram of a flow cytometry analysis of IFN-γ secreting E7-specific CD4⁺ cells in mice vaccinated with various recombinant DNA vaccines. C57BL/6 mice were immunized as described in Figs. 1A-1B. Splenocytes from vaccinated mice were cultured in vitro with E7 peptide (residues 30-67; DSSEEEDEIDGPAGQAEPDRAHYN IVTFCCKCDSTLRL; SEQ ID NO:6) overnight and were stained for both CD4 and intracellular IFN-γ. The number of IFN-γ secreting CD4⁺ T cells was analyzed by flow cytometry. Mice vaccinated with E7-HSP70 DNA generated comparable CD4⁺ IFN-γ ⁺ double positive cells compared to mice vaccinated with wild-type E7 DNA. The numbers of CD4⁺ IFN-γ⁺ double positive T cell in 3×10⁵ splenocytes are indicated in the upper right corner.

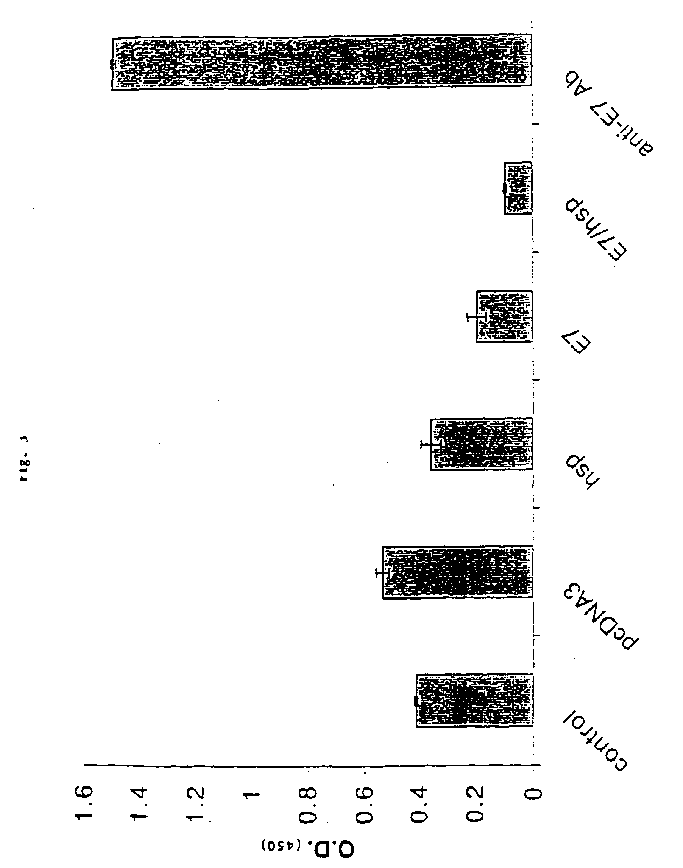

Figure 3 is a bar graph showing E7-specific antibody responses in C57BL/6 mice immunized with various recombinant DNA vaccines. C57BL/6 mice were immunized with control plasmid (no insert), wild-type HSP70, wild-type E7, or E7-HSP70 DNA via gene gun. Serum samples were obtained from immunized mice 14 days after vaccination. The presence of the E7-specific antibody was detected by ELISA using serial dilution of sera. The results from the 1:80 dilution are shown, showing mean absorbance (O.D.) at 450 nm SE.

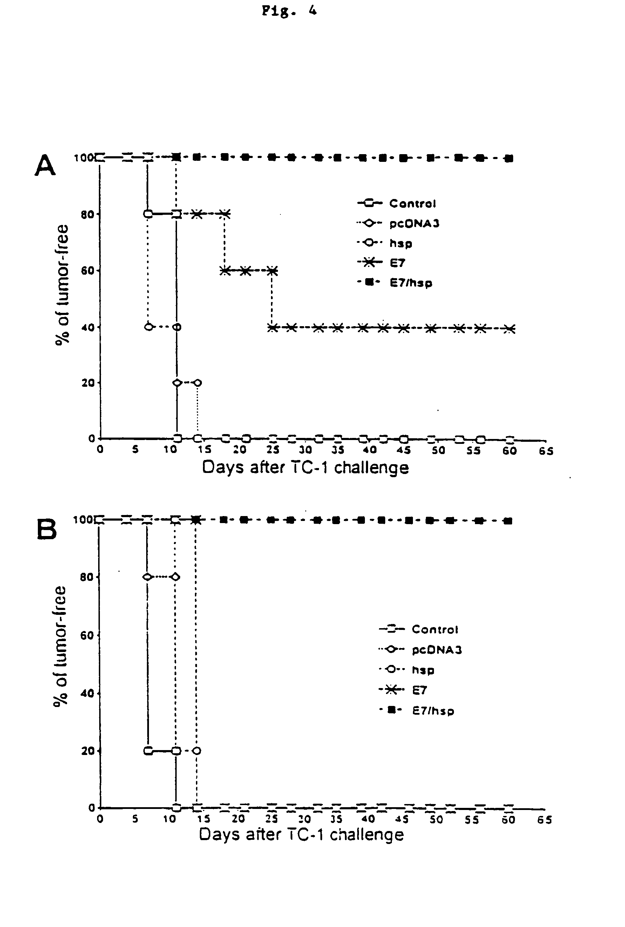

Figures 4A and 4B are line graphs showing that vaccination with E7-HSP70 DNA protects mice against the growth of TC-1 tumors. C57BL/6 mice were immunized with empty plasmid (pcDNA3), HSP70 DNA (HSP), E7 DNA (E7), or E7-HSP70 DNA (E7/HSP) via gene gun. One week after the last vaccination, mice were challenged with 5×10⁴ TC-1 cells per mouse subcutaneously in the right leg. The mice were monitored for evidence of tumor growth by palpation and inspection twice a week. At day 60 after TC-1 challenge, tumor-free mice were re-challenged with 5×10⁴ TC-1 cells per mouse subcutaneously in the left leg. To generate the data shown in Fig. 4A, each mouse received 2 µg DNA vaccine. One week later, the mice were re-vaccinated with the same type and amount of DNA as the first vaccination. To generate the data shown in Fig. 4B, each mouse received only one injection of 2 µg DNA vaccine without further booster.

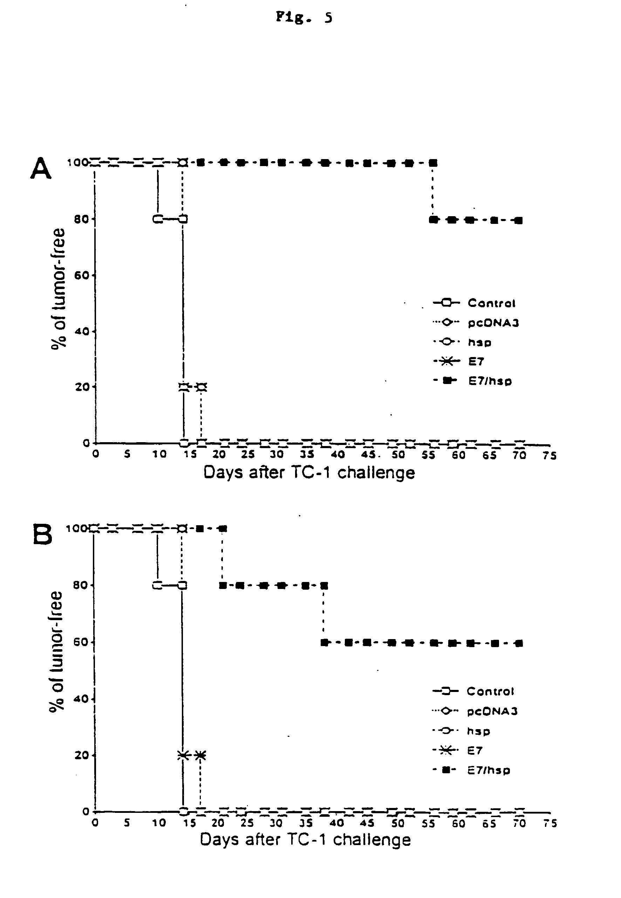

Figures 5A and 5B are line graphs showing that vaccination with E7-HSP70 DNA eradicates pre-existing TC-1 tumor cells. Each mouse was initially challenged with 2×10⁴ TC-1 cells subcutaneously, followed by DNA vaccination with empty plasmid (pcDNA3), HSP70 DNA (HSP), E7 DNA (E7), or E7-HSP70 DNA (E7/HSP) via gene gun. The mice were monitored for evidence of tumor growth by palpation and inspection twice a week. To generate the data shown in Fig. 5A, each mouse received 2 µg of DNA vaccine 3 days and 10 days after tumor challenge, and for Fig. 5B, each mouse received 2 µg of DNA vaccine only 3 days after tumor challenge. No further booster was given.

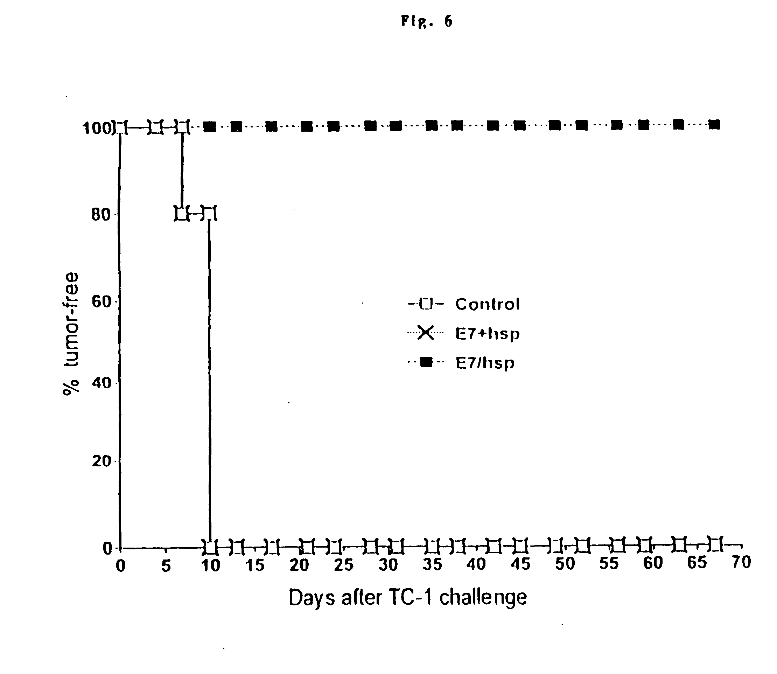

Figure 6 is a line graph showing that the antitumor immunity generated by E7-HSP70 DNA requires the fusion of HSP70 DNA sequences to E7 gene sequences. C57BL/6 mice were immunized with E7-HSP70 DNA(E7/HSP), or E7 DNA mixed with HSP70 DNA (E7+HSP) via gene gun, or received no vaccination. For vaccinated mice, 2 µg DNA /mouse was given only once. One week after the vaccination, mice were challenged with 5×10⁴ TC-1 cells/ mouse subcutaneously in the right leg. The mice were monitored for evidence of tumor growth by palpation and inspection twice a week.

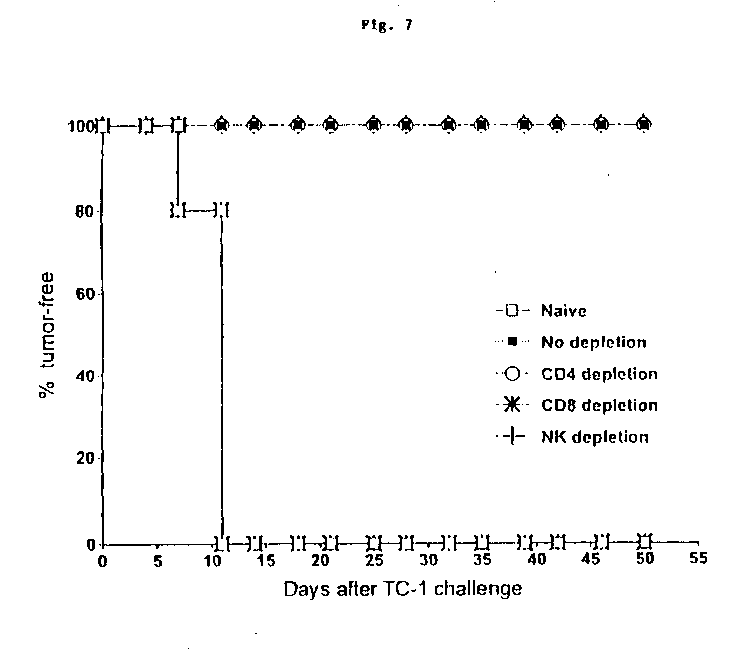

Figure 7 is a line graph showing the effect of lymphocyte subset depletions on the potency of E7-HSP70 DNA vaccine. C57BL/6 mice were immunized with 2 µg E7-HSP70 DNA via gene gun and boosted with 2 µg E7-HSP70 DNA one week later. Two weeks after the last vaccination, mice were challenged with 5×10⁴ TC-1 cells/ mouse subcutaneously in the right leg. Cell depletions were initiated one week prior to tumor challenge and lasted 40 days after tumor challenge. MAb GK1.5 was used for CD4 depletion, MAb 2.43 was used for CD8 depletion, and MAb PK136 was used for NK1.1 depletion. Flow cytometry analysis revealed that the greater than 95% of the appropriate lymphocytes subset were depleted with normal level of other subsets. The mice were monitored for evidence of tumor growth by palpation and inspection twice a week.

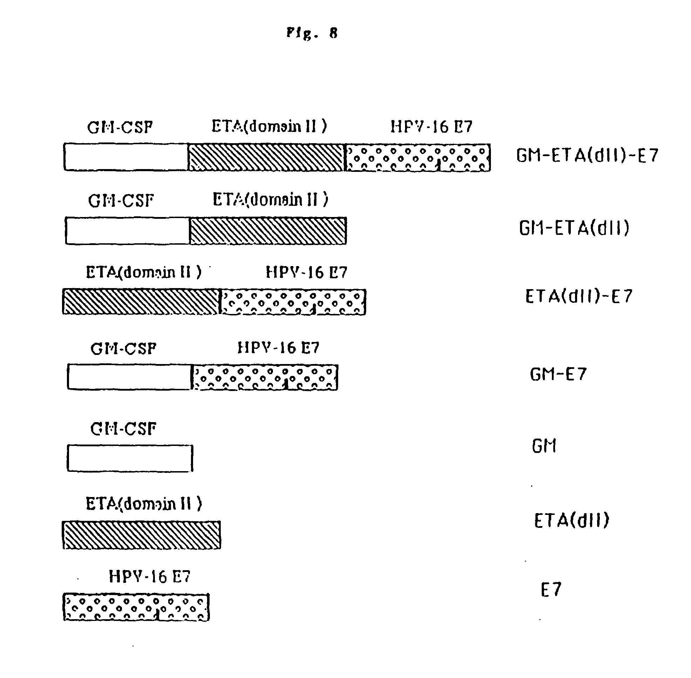

Figure 8 is a diagram of constructs of GM-ETA(dII)-E7, GM-ETA(dII), ETA(dII)-E7, GM-E7, GM-CSF, ETA(dII), E7.

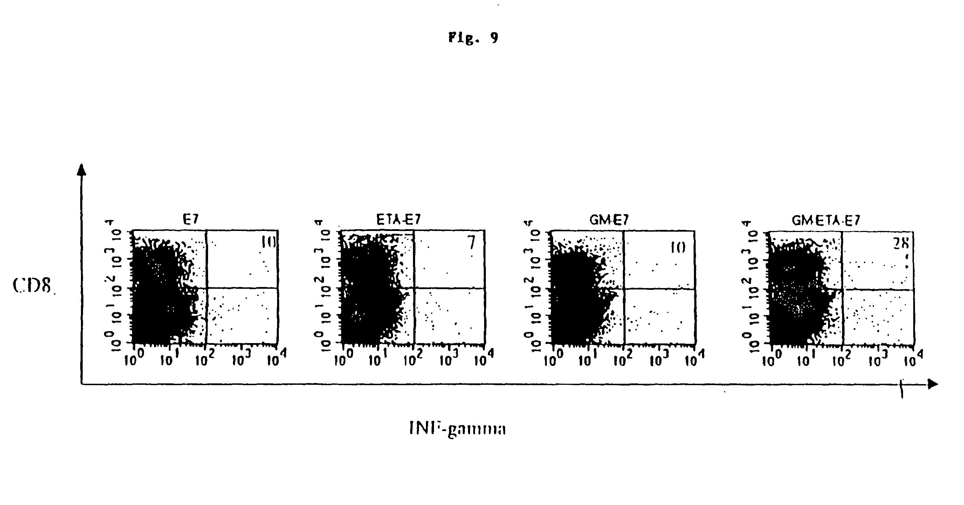

Figure 9 is a diagram of a flow cytometry analysis showing intracellular cytokine staining which was carried out to determine E7-specific CD8⁺ T cell precursors in C57BL/6 mice immunized with GM-ETA(DII)-E7 DNA vaccines. C57BL/6 mice were immunized with Plasmid DNA with GM, ETA(dII), E7, GM-ETA(dII), ETA(DII)-E7 , GM-E7 or GM-ETA(DII)-E7 gene insert or the "empty" plasmid vector intradermally via gene gun, or received no vaccination. For vaccinated mice, 2 µg DNA /mouse was given twice. Splenocytes were harvested 10 days after the last DNA vaccination. Splenocytes from vaccinated mice were cultured in vitro with E7 peptide (residues 49-57; SEQ ID NO:5) overnight and were stained for both CD8 and intracellular IFN-γ. The number of IFN-γ secreting CD8⁺ T cell precursors in mice immunized with various recombinant DNA vaccines was analyzed by flow cytometry. Mice vaccinated with GM-ETA(DII)-E7 DNA generated the highest IFN-γ⁺ CD8+ double positive T cells.

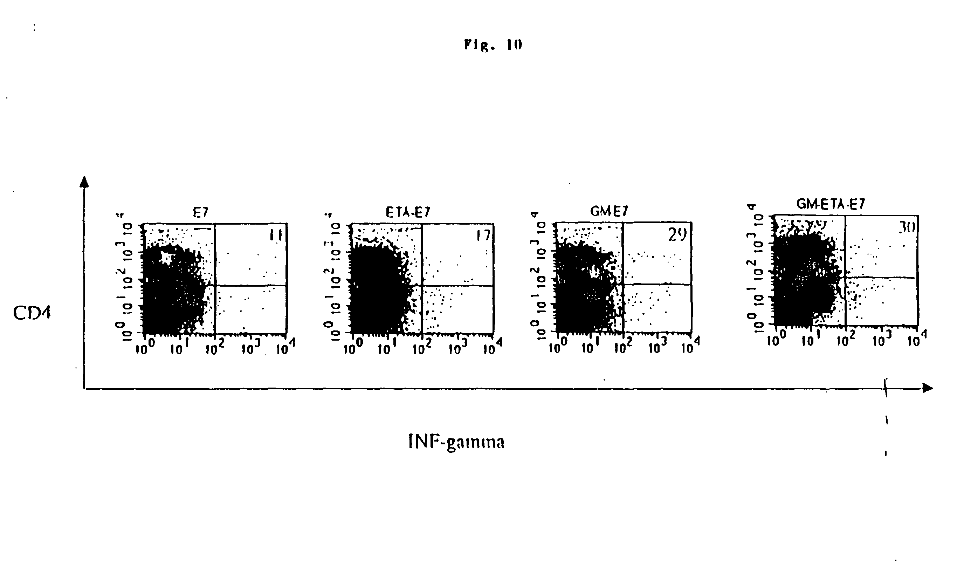

Figure 10 is a diagram showing flow cytometry analysis of IFN-γ secreting E7-specific CD4⁺ cells in mice vaccinated with various recombinant DNA vaccines. C57BL/6 mice were immunized as described above for Fig. 2. Splenocytes from vaccinated mice were cultured in vitro with E7 peptide (residues 30-67; SEQ ID NO:6) overnight and were stained for both CD4 and intracellular IFN-γ. The number of IFN-γ secreting CD4⁺ T cells was analyzed by flow cytometry. Mice vaccinated with GM-ETA(DII)-E7 DNA generated comparable CD4⁺ IFN-γ ⁺ double positive cells when compared to mice vaccinated with GM-E7 DNA. Mice vaccinated with GM-ETA (dII)-E7 generated greater number E7-specific CD4⁺ IFN-γ⁺ double positive cells than mice immunized with empty plasmid (pcDNA3), GM-ETA(dII), ETA(dII)-E7, GM-E7, GM-CSF, ETA(dII), or E7 DNA.

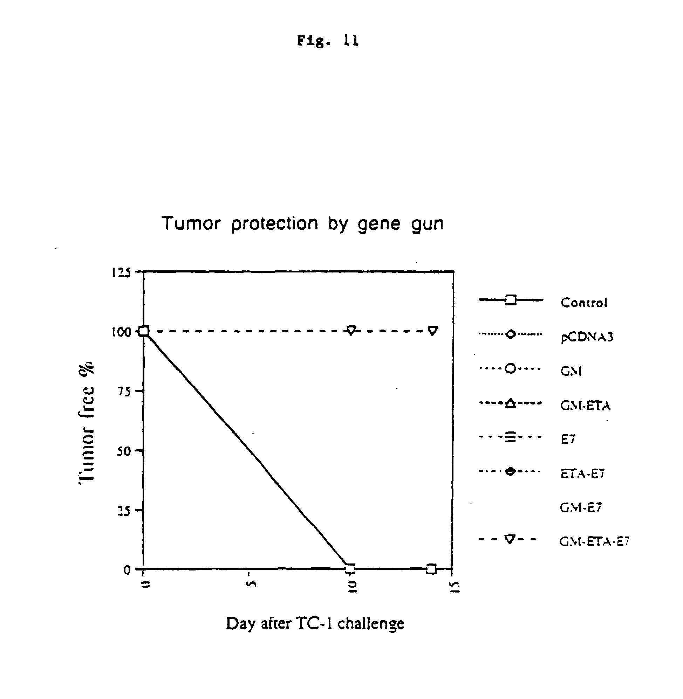

Figure 11 is a line graph showing that vaccination with GM-ETA(DII)-E7 DNA protects mice against the growth of TC-1 tumors. C57BL/6 mice were immunized with 2 µg/mouse of empty plasmid (pcDNA3), GM-ETA(dII), ETA(dII)-E7, GM-E7, GM-CSF, ETA(dII), E7 DNA, or GM-ETA(dII)-E7 DNA via gene gun. One week later, the mice were re-vaccinated with the same type and amount of DNA as the first vaccination. One week after the last vaccination, mice were challenged with 5×10⁴ TC-1 cells per mouse subcutaneously in the right leg. The mice were monitored for evidence of tumor growth by palpation and inspection twice a week. At day 60 after TC-1 challenge, tumor-free mice were re-challenged with 5×10⁴ TC-1 cells/mouse subcutaneously in the left leg.

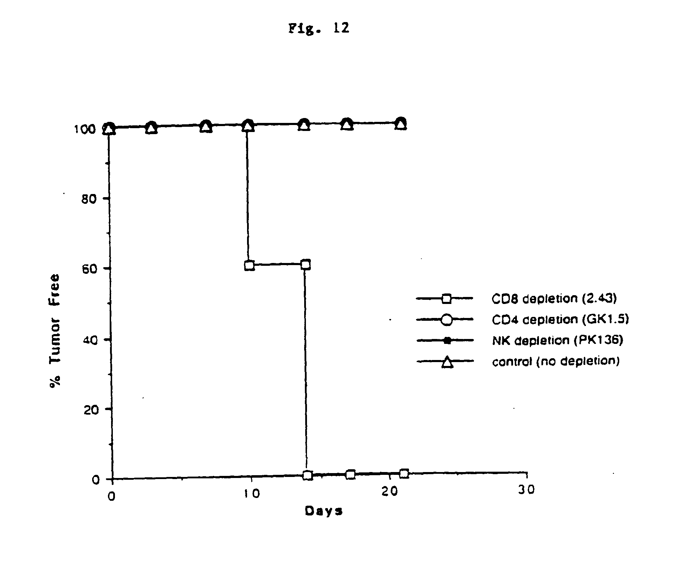

Figure 12 is a line graph showing the effect of lymphocyte subset depletions on the potency of GM-ETAdII-E7 DNA vaccine. C57BL/6 mice were immunized with 2 µg GM-ETAdII-E7 DNA via gene gun and boosted with 2 µg GM-ETAdII-E7 DNA one week later. Two weeks after the last vaccination, mice were challenged with 5×10⁴ TC-1 cell per mouse subcutaneously in the right leg. Depletions were initiated one week prior to tumor challenge and lasted the indicated number of days after tumor challenge. MAb GK1.5 (Dialynas et al., 1983, J. Immunol. 131:2445-2451) was used for CD4 depletion, MAb 2.43 (Sarmiento et al., 1980, J. Immunol. 125:2665-2672) was used for CD8 depletion, and MAb PK136 (Koo et al., 1986, J. Immunol. 137:3742-3747) was used for NK1.1 depletion. Flow cytometry analysis revealed that the >95% of the appropriate lymphocytes subset were depleted with normal level of other subsets. The mice were monitored for evidence of tumor growth by palpation and inspection twice a week.

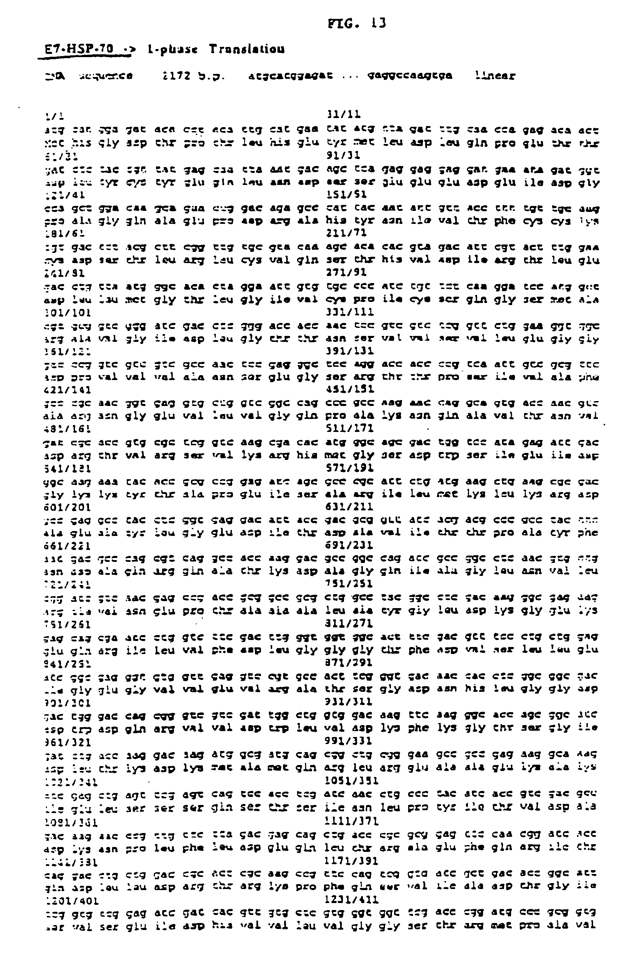

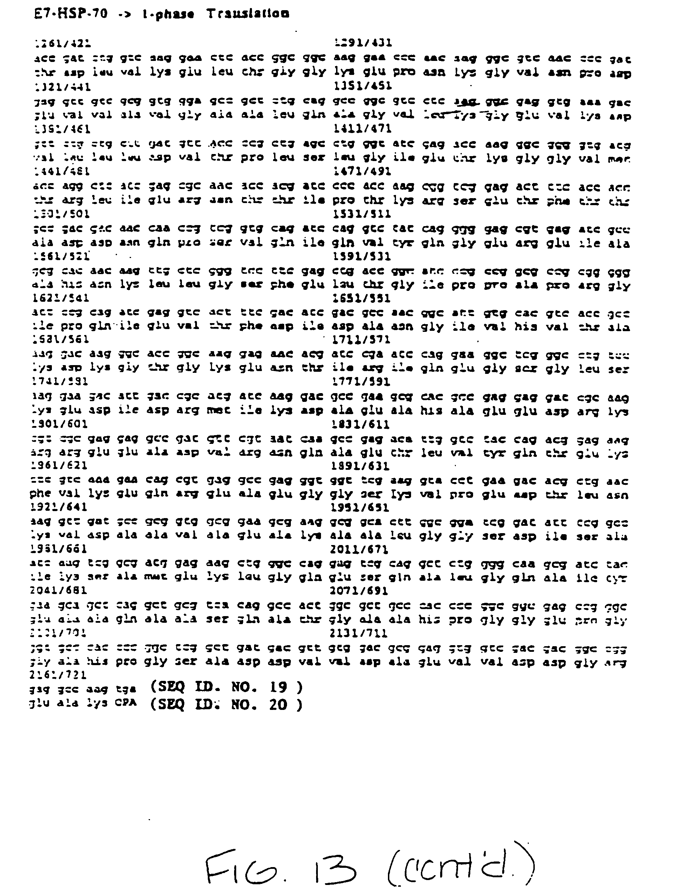

Figure 13 is a diagram of the amino acid and nucleotide sequence of the E7-HSP70 immunogenic construct.

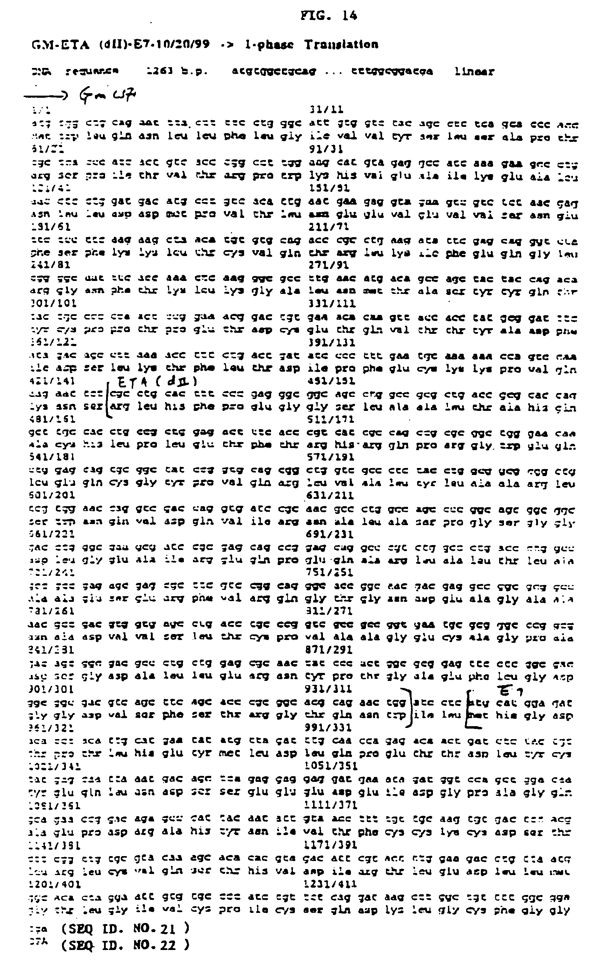

Figure 14 is a diagram of the amino acid and nucleotide sequence of the GM-ETA(dII)-E7 immunogenic construct. The GM-CSF portion, ETA(dII), and E7 portions of the construct are indicated with brackets.

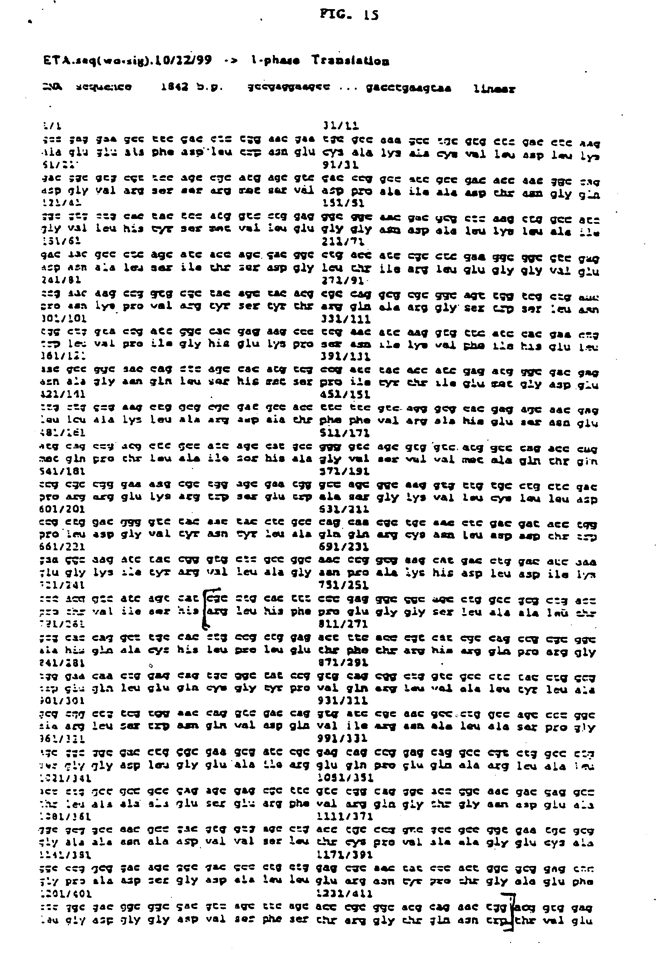

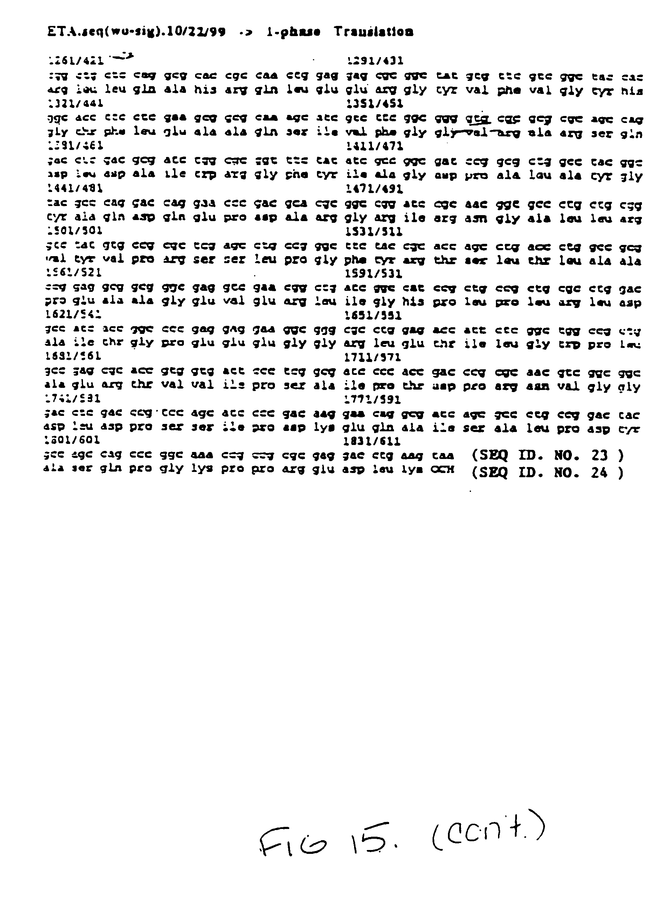

Figure 15 is a diagram of the amino acid and nucleotide sequence of ETA (without the signal sequence). Residues 247-417 (and corresponding nucleotide sequences) used in making the immunogenic construct are bracketed.

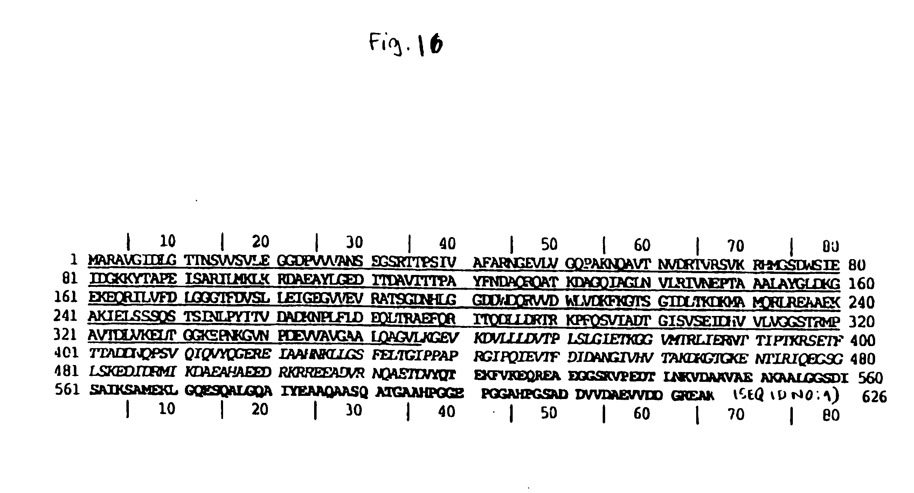

Figure 16 is a diagram showing the domain structure of full-length Mycobacterium tuberculosis HSP70 protein (SEQ ID NO:9). The ATPase domain (residues 1-356) is underlined; the substrate binding domain (SD; residues 357-516) is in italics, and C-terminal domain (CD: residues 517-625) is in bold type.

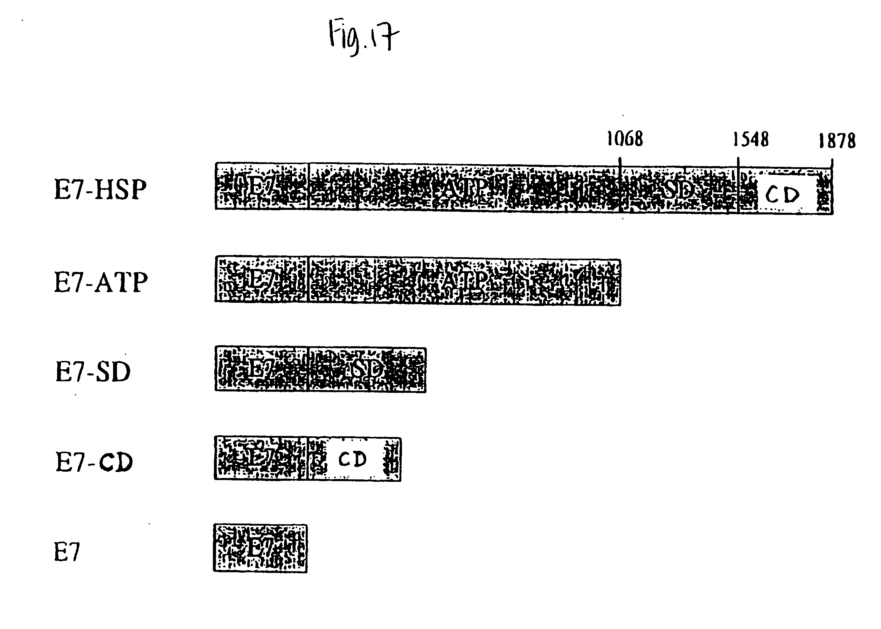

Figure 17 is a diagram of plasmid constructs in a mammalian cell expression vector. All of the chimeric E7/heat shock protein constructs (E7-HSP70, E7-ATP, E7-SD, E7-CD, and E7) were cloned into multiple cloning sites of an expression vector downstream of the cytomegalovirus promoter.

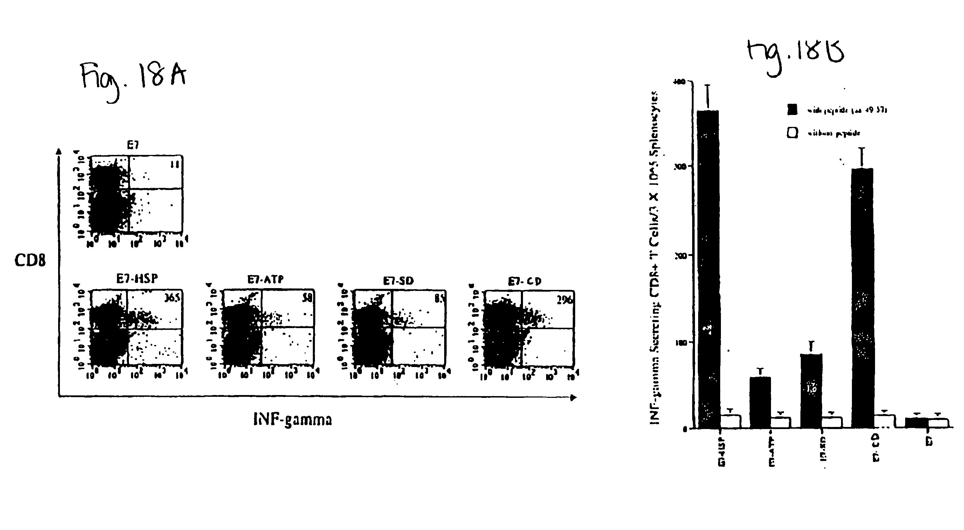

Figure 18A is a diagram of a flow cytometry analysis of IFN-γ E7-specific CD8+ cells in mice immunized with recombinant DNA vaccines containing nucleic acid constructs encoding Hsp fragment-antigen chimeras (E7-HSP70, E7-ATP, E7-SD, E7-CD, and E7). Figure 18B is a bar graph showing mean number of IFN-γ-producing E7-specific CD8+T cells.

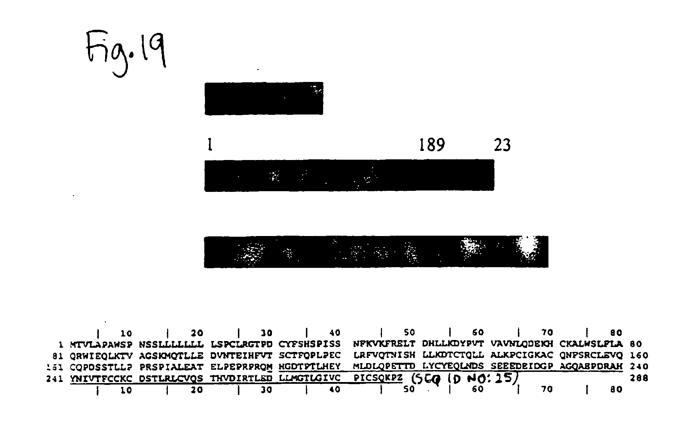

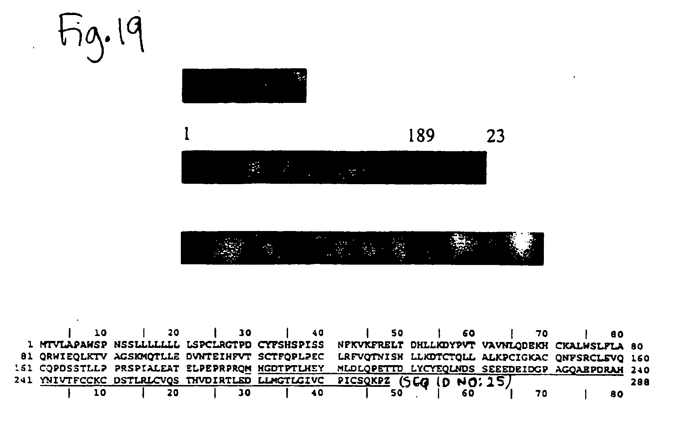

Figure 19 is a diagram of plasmid constructs in a mammalian cell expression vector.

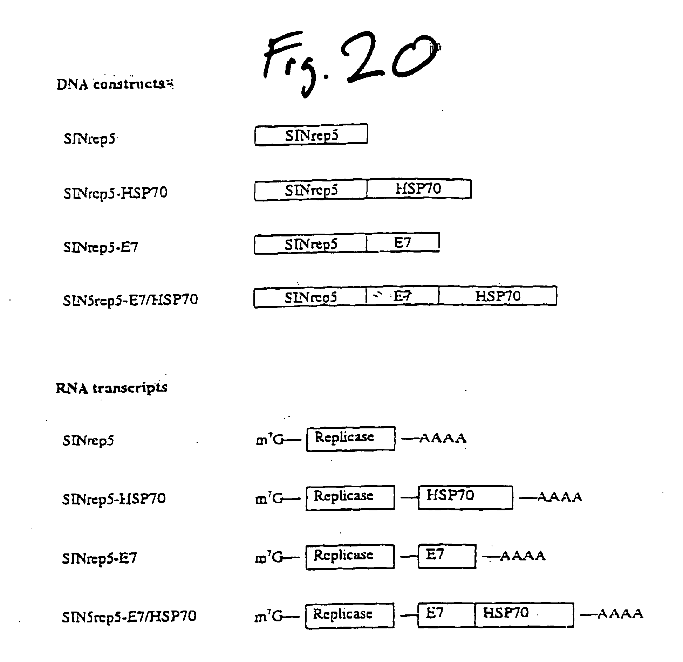

Figure 20A shows a schematic diagram of SINrep5, SINrep5-HSP70, SINrep5-E7, SINrep5-E7/HSP70 DNA constructs. Figure 20B shows a schematic diagram of RNA transcript derived from these DNA constructs using SP6 RNA polymerase as described in detail in Example 6, below.

Like reference symbols in the various drawings indicate like elements.

### DETAILED DESCRIPTION

The invention relates to novel chimeric nucleic acids encoding a chimeric polypeptide, constructs for expressing these polypeptides both *in vitro* and *in vivo,* isolated chimeric polypeptides, pharmaceutical compositions and methods of making and using these compositions. These compositions and methods are particularly useful for stimulating or enhancing the immunogenicity of a selected antigen or stimulating or enhancing a cellular immune response specific for that antigen. The nucleic acid of the invention comprises a first polypeptide domain comprising a carboxy terminal fragment of a heat shock protein (HSP), and a second polypeptide domain comprising an antigenic polypeptide.

One embodiment of the invention is based on the discovery that the immunogenicity of a target antigen encoded by a chimeric nucleic acid, e.g., a DNA vaccine, is enhanced by the presence in the chimeric nucleic acid of DNA comprising a first domain encoding a carboxy-terminal portion of a heat shock protein, such as *Mycobacterium tuberculosis* heat shock protein 70 (HSP70) (see, e.g., SEQ ID NO:9), and, a second domain encoding an antigen, e.g., an antigenic peptide from a pathogen or a tumor. Accordingly, the invention provides compositions and methods of vaccination that significantly enhance the potency, and thus clinical efficacy, of DNA vaccines.

In one embodiment, the chimeric nucleic acid of the invention encodes an immunogenic composition comprising a chimeric polypeptide comprising a first domain (encoded by a first domain DNA) comprising a carboxy-terminal domain (CD) fragment of a heat shock protein (HSP), e.g., HSP70, and a second domain comprising an antigen (encoded by a second domain DNA), such as a major histocompatibility complex (MHC) class I-restricted antigen, e.g., an MHC Class I-binding peptide epitope. In one embodiment, a carboxy-terminal fragment of a protein is a polypeptide or peptide that is at least 10 amino acids in length and is derived from the carboxy terminal half of a naturally-occurring protein. For example, a carboxy-terminal fragment of HSP70 is a peptide the amino acid sequence of which is derived from a portion of HSP70 spanning from about residues 312 to about 625 of SEQ ID NO:9; the peptide is encoded by DNA spanning the coding region of those residues. The carboxy-terminal domain (CD) fragment can also be a peptide having an amino acid sequence from about residues 517 to about 625 of SEQ ID NO:9.

For example, the immunogenic composition can comprise a chimeric polypeptide comprising a first domain comprising a DNA encoding a polypeptide containing the amino acid sequence of residues 517 to 625 of SEQ ID NO: 9 and (operably linked to) a second domain comprising a second DNA encoding a MHC class I restricted antigen. The invention therefore can include a papillomavirus E7-HSP70-CD fusion polypeptide as well as a chimeric nucleic acid (DNA) encoding the fusion polypeptide. Optionally, the composition includes DNA encoding a polypeptide containing residues derived from the amino-terminal fragment of HSP70, e.g., residues 161-370 of SEQ ID NO: 9. The order in which the DNA components, e.g., first and second (and optionally, third), DNAs are operably linked (i.e., arranged on domains on a recombinant protein) can be altered without affecting immunogenicity with the proviso that the nucleic acid encoding the second polypeptide domain is 5' to the nucleic acid encoding the first polypeptide domain. For example, the HSP-encoding DNA sequences are located 5' to the target antigen-encoding sequences. In one embodiment, these polypeptide-encoding nucleic acid domains are in-frame (i.e., the DNA is operably linked) so that the DNA construct encodes a recombinant polypeptide in which the antigen (e.g., MHC class I restricted antigen) is located amino-terminal to the HSP-derived residues.

As is discussed below, the antigen (e.g., MHC class I restricted antigen) can be derived from a pathogen, such as a virus, or from a cancer tissue, e.g., a tumor-specific or tumor-associated polypeptide. The DNA vaccine of the invention can comprise an expression cassette, e.g., an expression or a plasmid vector comprising a first DNA encoding a carboxy-terminal fragment of a HSP and (operably linked to) to a second DNA encoding an antigen (e.g., MHC class I restricted antigen). Therapeutic methods of the invention include methods of inducing a cytotoxic T cell response to an antigen in a mammal by administering to the mammal the immunogenic compositions described herein.

In alternative embodiments, a chimeric nucleic acid or immunogenic composition of the invention contains the following components: (a) a first DNA comprising a sequence encoding a carboxy terminal fragment of an HSP; (b) a second DNA comprising a sequence encoding a cytoplasmic translocator polypeptide (e.g., comprising ETA dII), and (c) a third DNA comprising a sequence encoding an antigen, e.g., an MHC class I restricted antigen. The first, second, and third DNAs can be operably linked. By "operably linked" is meant that a DNA sequence encoding one polypeptide is joined in frame to another DNA encoding another polypeptide to generate a recombinant chimeric, or "fusion," protein. Translation of operably linked DNA messages yields a chimeric polypeptide or fusion gene product. The invention includes chimeric nucleic acids and polypeptides in which the domains linked in all possible combinations, i.e., the order in which the first, second, and third DNAs are operably linked is irrelevant, with the proviso that the nucleic acid encoding the second polypeptide domain is 5' to the nucleic acid encoding the first polypeptide domain.

The construct (e.g., expression cassette, vector, and the like) may also contain regulatory sequences, such as transcriptional or translation regulatory sequences. A polypeptide coding sequence and a regulatory sequence(s) are connected in such a way as to permit gene expression when the appropriate molecules (e.g., transcriptional activator proteins) are bound to the regulatory sequence(s).

In one embodiment, the first nucleic acid domain of the immunogenic composition encodes a fragment of an HSP, e.g., *Mycobacterium tuberculosis* heat shock protein 70 (HSP70) (SEQ ID NO: 9). A "fragment" of a given protein is a polypeptide that is shorter in length than the reference polypeptide. For example, the polypeptide is at least 9 or 10 amino acids in length but less than the total number of residues of the mature reference protein or polypeptide. For example, a fragment of *M. tuberculosis* HSP70 is less than 70 kDa in molecular mass. A fragment of HSP70 has an amino acid sequence that is at least 50% identical to the amino acid sequence of a naturally-occurring HSP70. The length of an HSP70 fragment is less than 625 amino acids. The fragment can have a biological activity of the reference protein. In some embodiments, the second DNA encodes a fragment of an ETA (dII) (e.g., SEQ ID NO: 3).

The second domain (or third domain, if a cytoplasmic translocation domain is also present) of DNA (of the immunogenic composition) can encode an antigenic epitope, such as an MHC class I-restricted antigen. In one embodiment, the antigen is derived from a virus such as a human papovavirus, e.g., a cervical cancer-associated human papillomavirus (HPV). The viral antigen can be all or a part of E6 or E7 antigen derived from HPV-16. Other viral antigens that can be expressed by the chimeric nucleic acid of the invention include the E2 antigen of bovine viral diarrhea virus; ppUL83 or pp89 of cytomegalovirus; prM/E of encephalitis virus SLE; HBV surface antigen or HBV core antigen of hepatitis B virus; HIV-1 antigens such as gp160; ICP27, gD2, glycoprotein B, or glycoprotein B of herpes simplex virus. Alternatively, the antigen can be a cancer (e.g., a tumor-associated) antigen, such as a mutant p53; MAGE-1 or MAGE-3 associated with melanoma cells, e.g. a cancer-associated antigen expressed on the surface of a tumor cell. Other antigens include malaria peptide (NANP)40, HIV-1 p24, or influenza nucleoprotein.

The DNA encoding the second polypeptide domain can encode a translocation domain sequence, such as that of a *Pseudomonas* exotoxin A (ETA), e.g., domain II (dII) of ETA (e.g., spanning residues from about 253 to about 364 of SEQ ID NO:3). A translocation domain is a polypeptide that induces translocation of protein or polypeptide to which it is linked into the cytosol of a cell. For example, the second DNA can encode a polypeptide derived from a *Diptheria, Clostridium (Botulinum,* e.g., tetanus), *Bacillus anthracis* (anthrax), *Yersinia, Vibrio cholerae* (cholera), or *Bordetella pertussis* toxin. While the invention is not limited by any particular mechanisms, presence of DNA encoding a translocation domain in the immunogenic composition can enhance MHC class I presentation of the antigen encoded by the composition through translocation of antigen from the endosomal/ lysosomal compartment to the cytosol. Preferably, the toxic domain of the gene encoding the toxin is mutated or deleted.

The immunogenic composition need not contain a first, second, and third DNA, as described above. In alternative embodiments, the DNA encoding a third domain (a target antigen to which immunity is desired) is operably linked to a DNA encoding a carboxy terminal fragment of a HSP (see, e.g., SEQ ID NO:9), in another embodiment, the chimeric nucleic acid further encodes a DNA encoding a translocator (e.g., cytoplasmic translocator) polypeptide. For example, a chimeric nucleic acid of the invention can encode a fusion polypeptide containing E7-HSP70.

The chimeric nucleic acid of the invention can also encode an immunogenic composition comprising an E7-HSP70 fusion polypeptide.

The invention also relates to a DNA vaccine. The DNA vaccine composition can comprise a plasmid or other expression vector which includes (a) a first DNA encoding a carboxy terminal fragment of a heat shock protein (HSP), and (b) a second DNA encoding an antigen, e.g., an MHC class I restricted antigen. The vaccine can comprise a third domain comprising a DNA encoding a cytoplasmic translocator polypeptide. The polypeptide domain coding sequences (DNAs) are cloned into the expression (e.g., plasmid) vector in such a way that the first, second, and, if appropriate, third DNAs are "in-frame" (operably linked). When transcribed and translated in the cell in which the expression (plasmid) vector has been taken up, the vector directs production of a chimeric polypeptide that includes the above-described domains (e.g., an APC-binding portion, a cytoplasmic translocator portion, and an epitope of a class 1-restricted antigen).

In alternative embodiment, the chimeric nucleic acids (DNAs) (polynucleotides), expression cassettes, and polypeptides of the invention are isolated. By "isolated" is meant, e.g., that a nucleic acid molecule that is free of the genes which, in the naturally-occurring genome of the organism, flank the gene sequence of interest. The term therefore includes, for example, a recombinant DNA which is incorporated into, e.g., a vector; into an autonomously replicating plasmid or virus; or into the genomic DNA of a prokaryote or eukaryotic; or which exists as a separate molecule (e.g., a cDNA or a genomic or cDNA fragment produced by PCR or restriction endonuclease digestion) independent of other sequences. It also includes a recombinant DNA that is part of a hybrid gene encoding additional polypeptide sequence. The term excludes large segments of genomic DNA, e.g., such as those present in cosmid clones, which contain a given DNA sequence flanked by one or more other genes that naturally flank it in a naturally-occurring genome. See also definition of "isolated" herein.

Nucleic acid molecules (or polynucleotides) include both RNA and DNA, including cDNA, genomic DNA, and synthetic (e.g., chemically synthesized) DNA. Where single-stranded, the nucleic acid molecule may be a sense strand or an antisense strand. The term therefore includes, for example, a recombinant DNA which is incorporated into a vector, into an autonomously replicating plasmid or virus, or into the genomic DNA of a prokaryote or eukaryotic at a site other than its natural site; or which exists as a separate molecule (e.g., a cDNA or a genomic or cDNA fragment produced by polymerase chain reaction (PCR) or restriction endonuclease digestion) independent of other sequences. It also includes a recombinant DNA that is part of a hybrid gene encoding additional polypeptide sequence.

Relating to the invention are chimeric nucleic acids (DNAs) encoding the immunogenic compositions (the chimeric polypeptides, or "fusion proteins"), including the coding sequences in the expression cassettes, vectors, DNA vaccines of the invention, contain a strand which has the nucleotide sequence of a given reference sequence (e.g., a carboxy-terminal portion of *Mycobacterium tuberculosis* HSP70 (SEQ ID NO:9), cytoplasmic translocation domain of *Pseudomonas* ETA dII (SEQ ID NO:3)), or that has a sufficiently high sequence identity to have biologically equivalent activity, or, that hybridizes at high stringency to a strand of DNA haying a reference sequence, or the complement thereof. For example, a nucleic acid relating to the invention includes a DNA that has at least 50% sequence identity to a reference sequence, and encodes a polypeptide having a specified biological activity, e.g., cytoplasmic translocation domain of *Pseudomonas* ETA dII. Further examples include: the biological activity of the peptide encoded by the second DNA domain can be a cytoplasmic translocation domain, and the biological activity of the peptide encoded by the third DNA can bind to a MHC class I molecule. The DNA can have at least about 75% identity, about 85% identity, about 90% identity, about 95% identity, or about 99% identity, or 100% identity to the reference sequence.

To calculate sequence identity, nucleotide and amino acid comparisons are carried out using the Lasergene software package (DNASTAR, Inc., Madison, WI). The MegAlign module used was the Clustal V method (Higgins et al., 1989, CABIOS 5(2):151-153). The parameter used were gap penalty 10, gap length penalty 10.

Alternatively, related to the invention are chimeric nucleic acids (DNAs) encoding the immunogenic compositions that hybridize under high stringency conditions to a strand of DNA having the reference sequence (noted above), or the complement thereof and encode a polypeptide with a specific biological activity. Hybridization is carried out using standard techniques, such as those described in Ausubel et al. (Current Protocols in Molecular Biology, John Wiley & Sons, 1989). "High stringency" refers to nucleic acid hybridization and wash conditions characterized by high temperature and low salt concentration, i.e., wash conditions of 65°C at a salt concentration of approximately 0.1 X SSC. "Low" to "moderate" stringency refers to DNA hybridization and wash conditions characterized by low temperature and high salt concentration, i.e., wash conditions of less than 60°C at a salt concentration of at least 1.0 X SSC. For example, high stringency conditions may include hybridization at about 42°C, and about 50% formamide; a first wash at about 65°C, about 2X SSC, and 1% SDS; followed by a second wash at about 65°C and about 0.1% x SSC. Lower stringency conditions suitable for detecting DNA sequences having about 50% sequence identity to a reference gene or sequence are detected by hybridization at about 42□C in the absence of formamide; a first wash at about 42°C, about 6X SSC, and about 1% SDS; and a second wash at about 50°C, about 6X SSC, and about 1% SDS.

The invention relates to a method of inducing a cytotoxic T cell response to an antigen in a mammal by administering to the mammal a chimeric nucleic acid encoding the immunogenic composition, or the chimeric polypeptide, described above. In one embodiment, the composition is administered as naked DNA. The composition (e.g., DNA, polypeptides, vectors, etc.) can also administered in the presence of agents that enhance uptake of the DNA or polypeptide by target cells, such as a phospholipid formulation, e.g, a liposome.

The method is useful to vaccinate a mammal against infection by a pathogen. In one embodiment, the chimeric polypeptides of the invention (and the chimeric nucleic acids that encode them) comprise an antigen derived from said pathogen, such as a virus or bacteria. The method is also useful to prevent the development of cancer or treat an existing cancer in a mammal. Mammals at risk of developing a certain type of cancer are identified using known methods, e.g. genetic screening. Individuals at risk of developing a cancer or suffering from cancer are treated (the condition is "ameliorated") by administering the composition in which the antigen domain of the (e.g., third) DNA domain encodes a cancer-(tumor-) associated antigen, such as HPV E7 which is associated with cervical cancer.

The invention also relates to polynucleotides which, when directly introduced into a mammal *in vivo* induce the expression of an encoded polypeptide(s) within the animal and, in turn, a CD8+ immune response specific for the encoded polypeptide. The polynucleotide can be a nucleic acid that comprises essential regulatory elements (e.g., transcriptional or translational regulatory elements, e.g., promoters) such that upon introduction into a living vertebrate cell, the cell is able to produce translation products encoded by the polynucleotide. For example, the polynucleotide can be a poly-deoxyribonucleic acid (DNA) encoding a carboxy terminal fragment of a heat shock protein (HSP).

The chimeric nucleic acid (DNA) encoding an antigenic polypeptide to which an immune response is desired can operatively linked to a transcriptional promoter. In some cases, the antigen that is encoded by the polynucleotide is does not normally occur in that animal; it is present in the animal only in pathological conditions (e.g., a heterologous protein associated with a virus). In other cases, the antigen can be an autologous polypeptide, e.g., a tumor-associated antigen, that is misregulated in a disease state or other condition. The polypeptides encoded by the chimeric nucleic acid (e.g., DNA vaccine) are synthesized *in vivo* (i.e., produced by the animals' own tissues); the expressed chimeric proteins can be processed by the cell and expressed by MHC molecules, e.g., class I MHC polypeptides.

The chimeric nucleic acids encoding the antigen-comprising polynucleotides can be ligated into a suitable construct for *in vivo* administration, e.g., an expression vector which has been specifically optimized for polynucleotide vaccinations. The chimeric polypeptide-encoding sequences are operably linked to regulatory elements, such as transcriptional promoters, enhancers, immunogenic epitopes, and additional coding sequences (e.g., cistrons) encoding immuno-enhancing or immuno-modulatory genes, which can have their own regulatory elements (e.g., promoters, transcriptional terminators); the construct can also comprise a bacterial origin of replication and/or antibiotic resistance gene. The vector may also contain internal ribosome entry sites (IRES) for the expression of polycistronic mRNA. Transcriptional promoters include such powerful RNA polymerase promoters as the T7 or SP6 promoters.

The polynucleotide and DNA vaccines described herein elicit protective immunity against the antigens encoded. For example, the injection of a DNA expression vector encoding antigen E7 results death of tumor cells or a decrease in tumor cell growth. Antigen-specific CTLs against the tumor cells are produced following vaccination.

A number of advantages are associated with the *in vivo* administration vaccination strategy of the chimeric nucleic acids and expression systems of the invention. A drawback of many known DNA vaccines is their limited potency. In contrast to standard DNA vaccines, the chimeric nucleic acids (polynucleotides) and the methods of vaccination of the invention yield potent antigen-specific immunotherapy. The polynucleotides and DNA vaccines of the invention can induce a cellular immune response, such as a cytotoxic T lymphocyte (CTL) response, that is at least about 2-fold, at least about 10-fold, at least about 20-fold, at least about 30-fold, or at least about 40-fold more potent than conventional DNA vaccines (e.g., those that do not contain the chimeric nucleic acids of the invention, e.g., sequences encoding an chimeric polypeptide comprising a first polypeptide domain comprising a carboxy terminal fragment of a heat shock protein (HSP); and, a second polypeptide domain comprising an antigenic polypeptide.

Vaccine potency is determined by methods known in the art, e.g., by measuring CD8⁺ T cell precursors induced after vaccination or by measuring a reduction in pathogen (e.g., virus) or tumor load after vaccination, as described herein. While the invention is not limited by any particular mechanism of action, polypeptides encoded in a DNA vaccine may have the potential to enter the MHC class I pathway and evoke a cytotoxic T cell response. In one embodiment, the chimeric nucleic acid (e.g., DNA vaccine) of the invention is specifically designed to direct the immunogen into the MHC class I antigen-processing pathway by encoding a cytoplasmic translocation polypeptide domain (a "cytoplasmic translocator domain"). As a result, elicitation of a cytotoxic T cell response is optimized.

Other advantages of DNA vaccines include safety and simplicity of large scale production. The vaccines of the present invention are safe and useful for administration to domesticated or agricultural animals, as well as humans. For example, naked plasmid DNA is safe, has low immunogenicity, and can be repeatedly administered. DNA vaccines are easily prepared in large scale with high purity and are highly stable relative to proteins and other biologic polymers. See, e.g., U.S. Patent Nos. 5,580,859; 5,589,466; 5,910,488.

The amount of expressible DNA or transcribed RNA to be introduced into a vaccine recipient may vary depending on the strength of the transcriptional and translational promoters used. In addition, the magnitude of the immune response may depend on the level of protein expression and on the immunogenicity of the expressed gene product. In general, an effective dose ranges between about 1 ng to 5 mg, 100 ng to 2.5 mg, or between about 1 µg to about 750 µg, or between about 10 µg to about 300 µg of DNA is administered directly into a bodily tissue, such as muscle or dermal tissue. One dosage for intravenous administration (IV) of DNA is approximately 10⁶ to 10²² copies of the DNA molecule. Subcutaneous injection (SC), intradermal introduction, impression through the skin, and other modes of administration such as intraperitoneal, intravenous, or inhalation delivery are also suitable. For example, DNA can be administered by "biolistics" such as using a "gene gun." Booster vaccinations can be administered in the same manner. See, e.g., U.S. Patent Nos: 6,004,287; 5,753,477; 5,179,022.

DNA vaccines also can be formulated to be delivered as part of a *Listeria monocytogenes* vaccine using known methods, e.g., those described in Pan et al., 1995, Cancer Res. 55(21):4776-4779 and Pan et al., 1995, Nature Med. 1(5):471-7. Following vaccination with a polynucleotide immunogen, the immune response may also be boosted by administering the corresponding polypeptide immunogen.

The chimeric nucleic acid, vectors or other polynucleotide may be naked, that is, unassociated with any proteins, adjuvants or other agents that affect the recipients' immune system. Naked DNA is administered in a physiologically acceptable solution, such as sterile saline or sterile buffered saline.

Alternatively, the DNA may be associated with liposomes, such as lecithin liposomes or as a DNA-liposome mixture. Agents that assist in the cellular uptake of DNA (i.e., transfection facilitating agents), such as calcium ions may also be used. Microprojectiles coated with a polynucleotide are also useful as a means of administering the vaccine, e.g., gold particles. Small particle liposome or lipid complex aerosol compounds can also be used to deliver the nucleic acids of the invention, see, e.g., U.S. Patent No. 6,090,407.

*Functional components of the immunogenic composition:* The chimeric nucleic acid of the invention comprises a contiguous nucleic acid sequences capable of being expressed to produce a chimeric gene product with two or three or more functional domains: a first polypeptide domain comprising a carboxy terminal fragment of a heat shock protein (HSP); and, a second polypeptide domain comprising an antigenic polypeptide (e.g., a MHC Class I-restricted epitope). The chimeric nucleic acid can further comprise a cytoplasmic translocation domain-encoding sequence. The encoded gene product generates a potent immune response, particular a cytotoxic T cell response. The response is more potent than administering the chimeric gene product and more potent than administering a DNA vaccine which encodes an antigen (e.g., MHC class I-restricted epitope) without a carboxy terminal fragment of a heat shock protein (HSP) or, as in one embodiment, a cytoplasmic translocation portion.

*Domains of HSP70 Which Confer Enhancement of Antigen-Specific Immune Responses:* The domain structure of *Mycobacterium tuberculosis* HSP70 was determined, as described in detail in Example 5, below. At least three functional domains were identified: an ATPase domain (containing residues 1-356 of SEQ ID NO:9), substrate binding domain (SD; containing residues 357-516 of SEQ ID NO:9) and a carboxyterminal domain (CD; containing residues 517-625 of SEQ ID NO:9) (Fig. 16). The contribution of each domain of this HSP70 on the potency of naked DNA vaccines was determined. DNA encoding each domain was linked in frame, i.e., operably linked, to DNA encoding the papillomavirus antigen E7. E7-specific CD8 T cell immune responses were measured.

The results indicated that when incorporated into a DNA vaccine construct, DNA encoding a cytoplasmic domain (CD) of HSP70 increases the potency of the DNA vaccine in generating immunity to the target antigen encoded by the vaccine. Moreover, the cytoplasmic domain accounts for most of the enhancement of antigen specific MHC class I restricted CD8+ T cell immune responses in DNA constructs that contain full length HSP70.

### DEFINITIONS

Unless defined otherwise, all technical and scientific terms used herein have the meaning commonly understood by a person skilled in the art to which this invention belongs. As used herein, the following terms have the meanings ascribed to them unless specified otherwise.

The terms "antigen" or "immunogen" as used herein refers to a compound or composition comprising a peptide, polypeptide or protein which is "antigenic" or "immunogenic" when administered (or expressed *in vivo* by an administered nucleic acid, e.g., a DNA vaccine) in an appropriate amount (an "immunogenically effective amount"), *i.e.,* is capable of eliciting, augmenting or boosting a cellular and/or humoral immune response (which can also be immunosuppressive, e.g., as is the case in the induction of suppression), either alone or in combination or linked or fused to another substance (which can be administered at once or over several intervals).

The term "expression cassette" as used herein refers to a nucleotide sequence that is capable of affecting expression of a structural gene (i.e., a protein coding sequence) in a host compatible with such sequences. Expression cassettes include at least a promoter operably linked with the polypeptide coding sequence; and, optionally, with other sequences, e.g., transcription termination signals. Additional factors necessary or helpful in effecting expression may also be used, e.g., enhancers. "Operably linked" as used herein refers to linkage of a promoter upstream from a DNA sequence such that the promoter mediates transcription of the DNA sequence. Thus, expression cassettes also include plasmids, expression vectors, recombinant viruses, any form of recombinant "naked DNA" vector, and the like. A "vector" comprises a nucleic acid that can infect, transfect, transiently or permanently transduce a cell. It will be recognized that a vector can be a naked nucleic acid, or a nucleic acid complexed with protein or lipid. The vector optionally comprises viral or bacterial nucleic acids and/or proteins, and/or membranes (e.g., a cell membrane, a viral lipid envelope, etc.). Vectors include, but are not limited to replicons (e.g., RNA replicons (see Example 6, below), bacteriophages) to which fragments of DNA may be attached and become replicated. Vectors thus include, but are not limited to RNA, autonomous self-replicating circular or linear DNA or RNA (e.g., plasmids, viruses, and the like, see, e.g., U.S. Patent No. 5,217,879), and include both the expression and nonexpression plasmids. Where a recombinant microorganism or cell culture is described as hosting an "expression vector" this includes both extrachromosomal circular and linear DNA and DNA that has been incorporated into the host chromosome(s). Where a vector is being maintained by a host cell, the vector may either be stably replicated by the cells during mitosis as an autonomous structure, or is incorporated within the host's genome.

The terms "linked" or "chemically linked" refers to any chemical bonding of two moieties, e.g., as in one embodiment of the invention, where an ER chaperone polypeptide is chemically linked to an antigenic peptide. Such chemical linking includes the peptide bonding of a recombinantly or *in vivo* generated fusion protein.

The term "chimeric protein" or "fusion protein" refers to a composition comprising at least one polypeptide or peptide domain that is associated with a second polypeptide or peptide domain. For example, in one embodiment, the invention provides an isolated or recombinant nucleic acid molecule encoding a fusion protein (a chimeric polypeptide) comprising at least two domains, a first polypeptide domain comprising a carboxy terminal fragment of a heat shock protein (HSP) and a second polypeptide domain comprising an antigenic polypeptide. Additional domains can comprise a polypeptide, peptide, polysaccharide, or the like. The "fusion" can be an association generated by a peptide bond, a chemical linking, a charge interaction (e.g., electrostatic attractions, such as salt bridges, H-bonding, etc.) or the like. If the polypeptides are recombinant, the "fusion protein" can be translated from a common message. Alternatively, the compositions of the domains can be linked by any chemical or electrostatic means. The chimeric molecules of the invention can also include additional sequences, *e.g.,* linkers, epitope tags, enzyme cleavage recognition sequences, signal sequences, secretion signals, and the like. Alternatively, a peptide can be linked to a carrier simply to facilitate manipulation or identification/ location of the chimeric polypeptide.

The terms "antigenic" or "immunogen" or "immunogenic composition" refers to a compound or composition comprising a peptide, polypeptide or protein which is "immunogenic," *i.e.,* capable of eliciting, augmenting or boosting a cellular and/or humoral immune response, either alone or in combination or linked or fused to another substance. An immunogenic composition can be a peptide of at least about 5 amino acids, a peptide of 10 amino acids in length, a fragment 15 amino acids in length, a fragment 20 amino acids in length or greater. The immunogen can comprise a "carrier" polypeptide and a hapten, *e.g.,* a fusion protein or a carrier polypeptide fused or linked (chemically or otherwise) to another composition. The immunogen can be recombinantly expressed in an immunization vector, which can be simply naked DNA comprising the immunogen's coding sequence operably linked to a promoter, e.g., a simple expression cassette. The immunogen includes antigenic determinants, or epitopes, to which antibodies or, when bound to an MHC molecule's peptide binding site, T lymphocyte receptors (TCRs) bind; these epitopes are typically 3 to 10 amino acids in length.

The term "isolated" as used herein, when referring to a molecule or composition, such as, e.g., a chimeric nucleic acid or polypeptide of the invention, means that the molecule or composition is separated from at least one other compound, such as a protein, other nucleic acids (e.g., RNAs), or other contaminants with which it is associated *in vivo* or in its naturally occurring state. Thus, a composition is considered isolated when it has been isolated from any other component with which it is "naturally" associated or associated because it was generated recombinantly *in vitro* or *in vivo*, *e.g.,* cell membrane, as in a cell extract. An isolated composition can, however, also be substantially pure. An isolated composition can be in a homogeneous state and can be in a dry or an aqueous solution. Purity and homogeneity can be determined, for example, using analytical chemistry techniques such as polyacrylamide gel electrophoresis (SDS-PAGE) or high performance liquid chromatography (HPLC). Thus, the isolated compositions of this invention do not contain materials normally associated with their *in situ* environment. Even where a protein has been isolated to a homogenous or dominant band, there can be trace contaminants which co-purify with the desired composition.

The phrase "HPV polypeptide" including HPV-16 E7 polypeptides describes polypeptides well known in the art; e.g., for HPV-16 E7, see, e.g., GenBank Accession No. AF125673 (June 01, 1999) describing the complete HPV-16 genome and the HPV-16 E7 protein protein The terms "polypeptide," "protein," and "peptide" include compositions dating to the invention that also include "analogs," or "conservative variants" and "mimetics" or "peptidomimetics" with structures and activity that substantially correspond to the polypeptide from which the variant was derived, including, a carboxy terminal fragment of a heat shock protein (HSP).

The term "pharmaceutical composition" refers to a composition suitable for pharmaceutical use, e.g., as a vaccine, in a subject. The pharmaceutical compositions of this invention are formulations that comprise a pharmacologically effective amount of a composition comprising, e.g., a nucleic acid, or vector, or cell of the invention, and a pharmaceutically acceptable carrier.

The term "promoter" is an array of nucleic acid control sequences that direct transcription of a nucleic acid. As used herein, a promoter includes necessary nucleic acid sequences near the start site of transcription, such as, in the case of a polymerase II type promoter, a TATA element. A promoter also optionally includes distal enhancer or repressor elements that can be located as much as several thousand base pairs from the start site of transcription. A "constitutive" promoter is active under most environmental and developmental conditions. An "inducible" promoter is active under environmental or developmental regulation. A "tissue specific" promoter is active in certain tissue types of an organism, but not in other tissue types from the same organism. The term "operably linked" refers to a functional linkage between a nucleic acid expression control sequence (such as a promoter, or array of transcription factor binding sites) and a second nucleic acid sequence, wherein the expression control sequence directs transcription of the nucleic acid corresponding to the second sequence.

The term "recombinant" refers to a polynucleotide synthesized or otherwise manipulated *in vitro* (e.g.*,* "recombinant polynucleotide"), to methods of using recombinant polynucleotides to produce gene products in cells or other biological systems, or to a polypeptide ("recombinant protein") encoded by a recombinant polynucleotide. For example, recombinant nucleic acid or polypeptide, as described herein, can be used to practice the methods of the invention. "Recombinant means" also encompass the ligation of nucleic acids having various coding regions or domains or promoter sequences from different sources into an expression cassette or vector for expression of, *e.g.,* inducible or constitutive expression of polypeptide coding sequences in the vectors used to practice this invention.

The term "self-replicating RNA replicon" refers to constructs based on RNA viruses, e.g., alphavirus genome RNAs (e.g., Sindbis virus, Semliki Forest virus, etc.), that have been engineered to allow expression of heterologous RNAs and proteins. These recombinant vectors are self-replicating (i.e., they are "replicons") and can be introduced into cells as naked RNA or DNA, as described in detail, below. In one embodiment, the self-replicating RNA replicon comprises a Sindbis virus self replicating RNA vector SINrep5, which is described in detail in U.S. Patent No. 5,217,879.

The term "systemic administration" refers to administration of a composition or drug, such as the DNA vaccines of the invention or chimeric nucleic acids and polypeptides described herein, in a manner that results in the introduction of the composition or drug into the circulatory system. The term "regional administration" refers to administration of a composition or drug into a specific anatomical space, such as intraperitoneal, intrathecal, subdural, or to a specific organ, and the like. For example, regional administration includes administration of the composition or drug into the hepatic artery for regional administration to the liver. The term "local administration" refers to administration of a composition or drug into a limited, or circumscribed, anatomic space, such as intratumoral injection into a tumor mass, subcutaneous injections, intramuscular injections, and the like. Any one of skill in the art would understand that local administration or regional administration may also result in entry of the composition or drug into the circulatory system.

### Generating and Manipulating of Nucleic Acids

The methods of the invention provide for the administration of nucleic acids encoding fusion proteins, as described herein. Recombinant fusion proteins can be synthesized *in vitro* or *in vivo.* Nucleic acids encoding these compositions can be in the form of "naked DNA" or they can be incorporated in plasmids, vectors, recombinant viruses (e.g., "replicons") and the like for *in vivo* or *ex vivo* administration. Nucleic acids and vectors of the invention can be made and expressed *in vitro* or *in vivo,* a variety of means of making and expressing these genes and vectors can be used. One of skill will recognize that desired gene activity may be obtained by modulating the expression or activity of the genes and nucleic acids (e.g., promoters) within vectors used to practice the invention. Any of the known methods described for increasing or decreasing expression or activity, or tissue specificity, of genes can be used for this invention. The invention can be practiced in conjunction with any method or protocol known in the art, which are well described in the scientific and patent literature.

### General Techniques

The nucleic acid sequences used to practice this invention, whether RNA, cDNA, genomic DNA, vectors, recombinant viruses or hybrids thereof, may be isolated from a variety of sources, genetically engineered, amplified, and/or expressed recombinantly. Any recombinant expression system can be used, including, in addition to bacterial cells, e.g., mammalian, yeast, insect or plant cell expression systems. Alternatively, these nucleic acids can be synthesized *in vitro* by well-known chemical synthesis techniques, as described in, e.g., Carruthers (1982) Cold Spring Harbor Symp. Quant. Biol. 47:411-418; Adams (1983) J. Am. Chem. Soc. 105:661; Belousov (1997) Nucleic Acids Res. 25:3440-3444; Frenkel (1995) Free Radic. Biol. Med. 19:373-380; Blommers (1994) Biochemistry 33:7886-7896; Narang (1979) Meth. Enzymol. 68:90; Brown (1979) Meth. Enzymol. 68:109; Beaucage (1981) Tetra. Lett. 22:1859; U.S. Patent No. 4,458,066. Double stranded DNA fragments may then be obtained either by synthesizing the complementary strand and annealing the strands together under appropriate conditions, or by adding the complementary strand using DNA polymerase with an appropriate primer sequence.

Techniques for the manipulation of nucleic acids, such as, e.g., generating mutations in sequences, subcloning, labeling probes, sequencing, hybridization and the like are well described in the scientific and patent literature, see, e.g., Sambrook, ed., MOLECULAR CLONING: A LABORATORY MANUAL (2ND ED.), Vols. 1-3, Cold Spring Harbor Laboratory, (1989); CURRENT PROTOCOLS IN MOLECULAR BIOLOGY, Ausubel, ed. John Wiley & Sons, Inc., New York (1997); LABORATORY TECHNIQUES IN BIOCHEMISTRY AND MOLECULAR BIOLOGY: HYBRIDIZATION WITH NUCLEIC ACID PROBES, Part I. Theory and Nucleic Acid Preparation, Tijssen, ed. Elsevier, N.Y (1993).

Nucleic acids, vectors, capsids, polypeptides, and the like can be analyzed and quantified by any of a number of general means well known to those of skill in the art. These include, e.g., analytical biochemical methods such as NMR, spectrophotometry, radiography, electrophoresis, capillary electrophoresis, high performance liquid chromatography (HPLC), thin layer chromatography (TLC), and hyperdiffusion chromatography, various immunological methods, e.g. fluid or gel precipitin reactions, immunodiffusion, immuno- electrophoresis, radioimmunoassays (RIAs), enzyme-linked immunosorbent assays (ELISAs), immuno-fluorescent assays, Southern analysis, Northern analysis, dot-blot analysis, gel electrophoresis (e.g., SDS-PAGE), RT-PCR, quantitative PCR, other nucleic acid or target or signal amplification methods, radiolabeling, scintillation counting, and affinity chromatography.

### Amplification of Nucleic Acids

Oligonucleotide primers can be used to amplify nucleic acids to generate fusion protein coding sequences used to practice the invention, to monitor levels of vaccine after *in vivo* administration (e.g., levels of a plasmid or virus), to confirm the presence and phenotype of activated CTLs, and the like. The skilled artisan can select and design suitable oligonucleotide amplification primers using known sequences, e.g., those encoding a carboxy terminal fragment of a heat shock protein (HSP), an Flt-3 ligand (FL), a cytoplasmic translocation domain of a *Pseudomonas* exotoxin A (ETA dII), or a granulocyte-macrophage-colony stimulating factor (GM-CSF) sequence. Amplification methods are also well known in the art, and include, *e.g.,* polymerase chain reaction, PCR (PCR PROTOCOLS, A GUIDE TO METHODS AND APPLICATIONS, ed. Innis, Academic Press, N.Y. (1990) and PCR STRATEGIES (1995), ed. Innis, Academic Press, Inc., N.Y., ligase chain reaction (LCR) (see, e.g., Wu (1989) Genomics 4:560; Landegren (1988) Science 241:1077; Barringer (1990) Gene 89:117); transcription amplification (see, e.g., Kwoh (1989) Proc. Natl. Acad. Sci. USA 86:1173); and, self-sustained sequence replication (see, e.g., Guatelli (1990) Proc. Natl. Acad. Sci. USA 87:1874); Q Beta replicase amplification (see, e.g., Smith (1997) J. Clin. Microbiol. 35:1477-1491), automated Q-beta replicase amplification assay (see, e.g., Burg (1996) Mol. Cell. Probes 10:257-271) and other RNA polymerase mediated techniques *(e.g.,* NASBA, Cangene, Mississauga, Ontario); see also Berger (1987) Methods Enzymol. 152:307-316; Sambrook; Ausubel; U.S. Patent Nos. 4,683,195 and 4,683,202; Sooknanan (1995) Biotechnology 13:563-564.

### Cloning and construction of expression cassettes

Expression cassettes, including plasmids, recombinant viruses (e.g., RNA viruses like the replicons described below) and other vectors encoding the fusion proteins described herein are used to express these polypeptides *in vitro* and *in vivo.* Recombinant nucleic acids expressed by a variety of conventional techniques, well described in the scientific and patent literature. See, e.g., Roberts (1987) Nature 328:731; Schneider (1995) Protein Expr. Purif. 6435:10; Sambrook, Tijssen or Ausubel. Plasmids, vectors, etc. can be isolated from natural sources, obtained from such sources as ATCC or GenBank libraries, or prepared by synthetic or recombinant methods.

The nucleic acids used to practice the invention can be stably or transiently expressed in cells (e.g., episomal expression systems). Selection markers can be incorporated to confer a selectable phenotype on transformed cells. For example, selection markers can code for episomal maintenance and replication such that integration into the host genome is not required. For example, the marker may encode antibiotic resistance (e.g., chloramphenicol, kanamycin, G418, bleomycin, hygromycin) or herbicide resistance (e.g., chlorosulfuron or Basta) to permit selection of those cells transformed with the desired DNA sequences (see, e.g., Blondelet- Rouault (1997) Gene 190:31 S-317; Aubrecht (1997) J. Pharmacol. Exp. Ther. 281:992-997).

### In Vivo Nucleic Acid Administration

In one embodiment, the chimeric nucleic acids of the invention are cloned into expression cassettes (e.g., plasmids or other vectors, viruses) that can transfect or infect cells (such as human or other mammalian cells) *in vitro, ex vivo* and/or *in vivo.* Any approach can be used, e.g., lipid or liposome based gene delivery (see, e.g., Mannino (1988) BioTechniques 6:682-691; U.S. Pat No. 5,279,833), replication-defective retroviral vectors with desired exogenous sequence as part of the retroviral genome (see, e.g., Miller (1990) Mol. Cell. Biol. 10:4239; Kolberg (1992) J. NIH Res. 4:43; Cornetta (1991) Hum. Gene Ther. 2: 215). See also, e.g., Zhang (1996) Cancer Metastasis Rev. 15:385-401; Anderson, Science (1992) 256: 808-813; Nabel (1993) TIBTECH 11: 211-217; Mitani (1993) TIBTECH 11: 162-166; Mulligan (1993) Science, 926-932; Dillon (1993) TIBTECH 11: 167-175; Miller (1992) Nature 357: 455-460.

Expression cassettes can also be derived from viral genomes. Vectors which may be employed include recombinantly modified enveloped or non-enveloped DNA and RNA viruses, e.g., from baculoviridiae, parvoviridiae, picornoviridiae, herpesveridiae, poxviridae, adenoviridiae, picornnaviridiae or alphaviridae. Chimeric vectors may also be employed which exploit advantageous merits of each of the parent vector properties (See e.g., Feng (1997) Nature Biotechnology 15:866-870). Such viral genomes may be modified by recombinant DNA techniques to include the tumor suppressor gene and may be engineered to be replication deficient, conditionally replicating or replication competent. The vectors can be replication deficient or conditionally replicating. Vectors can be derived from adenoviral, adeno-associated viral or retroviral genomes. Retroviral vectors can include those based upon murine leukemia virus (MuLV), gibbon ape leukemia virus (GaLV), Simian Immuno deficiency virus (SIV), human immuno deficiency virus (HIV), and combinations thereof. See, e.g., Buchscher (1992) J. Virol. 66(5) 2731-2739; Johann (1992) J. Virol. 66 (5):1635-1640 (1992); Sommerfelt (1990) Virol. 176:58-59; Wilson (1989) J. Virol. 63:2374-2378; Miller (1991) J. Virol. 65:2220-2224. Adeno-associated virus (AAV)-based vectors can also used to transduce cells, e.g., for the *in vitro* production of nucleic acids and peptides, and in *in vivo* and *ex vivo* therapy procedures. See, Okada (1996) Gene Ther. 3:957-964; West (1987) Virology 160:38-47; Carter (1989) U.S. Patent No. 4,797,368; Carter et al. WO 93/24641 (1993); Kotin (1994) Human Gene Therapy 5:793-801; Muzyczka (1994) J. Clin. Invst. 94:1351, for an overview of AAV vectors.

### In vivo administration using self-replicating RNA replicons

In addition to the above-described expression vectors and recombinant viruses, self-replicating RNA replicons can also be used to infect cells or tissues or whole organisms with a fusion protein-expressing nucleic acids of the invention. Thus, the invention also incorporates RNA viruses, e.g., alphavirus genome RNAs (e.g., Sindbis virus; Semliki Forest virus; Venezuelan equine encephalitis virus, and the like), that have been engineered to allow expression of heterologous RNAs and proteins. High levels of expression of heterologous sequences, e.g., fusion proteins of the invention, are achieved when the viral structural genes are replaced by the heterologous coding sequences.

These recombinant RNAs are self-replicating (i.e., they are "replicons") and can be introduced into cells as naked RNA or DNA. However, they require *trans* complementation to be packaged and released from cells as infectious virion particles. The defective helper RNAs contain the cis-acting sequences required for replication as well as an RNA promoter which drives expression of polypeptide-encoding open reading frames. In cells co-transfected with both the replicon and defective helper RNAs, viral nonstructural proteins translated from the replicon RNA allow replication and transcription of the defective helper RNA to produce the virion's structural proteins. See, e.g., Bredenbeek (1993) J. Virol. 67:6439-6446.

RNA replicon vaccines may be derived from alphavirus vectors, such as Sindbis virus (family *Togaviridae)* (see, e.g., Xiong (1989) Science 243:1188-1191), Semliki Forest virus (see, e.g., Ying (1999) Nat. Med. 5:823-827) or Venezuelan equine encephalitis virus (see, e.g., Pushko (1997) Virology 239:389-401) vectors. These vaccines are self-replicating and self-limiting and may be administered as either RNA or DNA, which is then transcribed into RNA replicons in transfected cells or *in vivo* (see, e.g., Berglund (1998) Nat. Biotechnol. 16:562-565). Self-replicating RNA infects a diverse range of cell types and allows the expression of the antigen of interest at high levels (see, e.g., Huang (1996) Curr. Opin. Biotechnol. 7:531-535). Additionally, self-replicating RNA eventually causes lysis of transfected cells because viral replication is toxic to infected host cells (see, e.g., Frolov (1996) J. Virol. 70:1182-1190). These vectors therefore do not raise the concern associated with naked DNA vaccines of integration into the host genome. This is particularly important for vaccine development targeting proteins that are potentially oncogenic; such as the adenoviral E7 protein.

In one embodiment, the self-replicating RNA replicon comprises a Sindbis virus self-replicating RNA vector SINrep5, as described in detail by, e.g., Bredenbeek (1993) J. Virol. 67:6439-6446; see also, e.g., Herrmann (1998) Biochem. Biophys. Res. Commun. 253:524-531.

### Formulation and Administration of Pharmaceutical Compositions

In dated methods of the invention, polypeptides, nucleic acids, expression cassettes, cells, and particles, are administered to an individual as pharmacological compositions in amounts sufficient to generate an antigen-specific immune response (e.g., a CTL response) in the individual.

Pharmaceutically acceptable carriers and formulations for nucleic acids, peptides and polypeptides are known to the skilled artisan and are described in detail in the scientific and patent literature, see e.g., the latest edition of Remington's Pharmaceutical Science, Maack Publishing Company, Easton, PA ("Remington's"), Banga; Putney (1998) Nat. Biotechnol. 16:153-157; Patton (1998) Biotechniques 16:141-143; Edwards (1997) Science 276: 1868-1871; U.S. Patent Nos. 5,780,431; 5,770,700; 5,770,201.

The nucleic acids and polypeptides used in the methods relating to the invention can be delivered alone or as pharmaceutical compositions by any means known in the art, *e.g.*, systemically, regionally, or locally; by intraarterial, intrathecal (IT), intravenous (IV), parenteral, intra-pleural cavity, topical, oral, or local administration, as subcutaneous, intratracheal (e.g., by aerosol) or transmucosal (e.g., buccal, bladder, vaginal, uterine, rectal, nasal mucosa). Actual methods for delivering compositions will be known or apparent to those skilled in the art and are described in detail in the scientific and patent literature, see *e.g.,* Remington's.

The pharmaceutical compositions can be administered by any protocol and in a variety of unit dosage forms depending upon the method and route and frequency of administration, whether other drugs are being administered, the individual's response, and the like. Dosages for typical nucleic acid, peptide and polypeptide pharmaceutical compositions are well known to those of skill in the art. Such dosages are typically advisorial in nature and are adjusted depending on a variety of factors, *e.g.,* the initial responses *(e.g.,* amount of antigen-specific CTLs generated, tumor shrinkage, and the like), the particular therapeutic context, patient health and tolerance. The amount of pharmaceutical composition adequate to generate the desired response is defined as a "therapeutically effective dose." The dosage schedule and amounts effective for this use, *i.e.*, the "dosing regimen," will depend upon a variety of factors, including, e.g., the diseases or conditions to be treated or prevented by the immunization, the general state of the patient's health, the patient's physical status, age, pharmaceutical formulation and concentration of pharmaceutical composition, and the like. The dosage regimen also takes into consideration pharmacokineties, *i.e.,* the pharmaceutical composition's rate of absorption, bioavailability, metabolism, clearance, and the like, see, e.g., Remington. Dosages can be determined empirically, e.g., by assessing the abatement or amelioration of symptoms, or, by objective criteria, e.g., measuring levels of antigen-specific CTLs. As noted above, a single or multiple administrations can be administered depending on the dosage and frequency as required and tolerated by the patient. The pharmaceutical compositions can be administered alone or in conjunction with other therapeutic treatments, or, as prophylactic immunization. Kits

The invention relates to kits that contain the pharmaceutical compositions of the invention, as described above, to practice the methods of the invention. In alternative embodiments, the kits can contain recombinant or synthetic chimeric polypeptides of the invention; or, the nucleic acids encoding them, e.g., in the form of naked DNA (e.g., plasmids), viruses (e.g. alphavirus-derived "replicons" including Sindbis virus replicans) and the like. The kit can contain instructional material teaching methodologies, e.g., means to administer the compositions used to practice the invention, means to inject or infect cells or patients or animals with the nucleic acids or polypeptides of the invention, means to monitor the resultant immune response (e.g., CTL response) and assess the reaction of the individual to which the compositions have been administered, and the like.

### EXAMPLES

The following examples are offered to illustrate, but not to limit the claimed invention.

### Example 1: Enhancement of DNA Vaccine Potency by Linkage of an Antigen Gene to an HSP70 Gene and Treatment of Tumors In Vivo

The following example describes studies that demonstrate that the compositions and methods of the invention are effective for enhancing antigen-specific cytotoxic T lymphocyte (CTL) responses and treating tumors *in vivo.*

Using HPV-16 E7 as a model antigen, the data described herein indicate that a DNA vaccine which contains a polynucleotide encoding a chimeric polypeptide with an heat shock protein, cytoplasmic translocation portion, and an antigen in the form of a class I MHC epitope resulted in potent immunity specific for the epitope. The effect of linkage to *Mycobacterium tuberculosis* heat shock protein 70 (HSP70) on the potency of antigen-specific immunity generated by naked DNA vaccines was evaluated.

*M. tuberculosis* HSP70 dramatically enhances the potency of HPV-16 E7-expressing DNA vaccines. DNA vaccines with HSP70 fused to HPV-16 E7 elicited strong E7-specific cellular immunity (at least 30-fold increase in the E7-specific CD8⁺ T cells precursor frequencies) and generated significant CD8⁺ T cell-dependent preventive and therapeutic effects against HPV-16 E7-expressing murine tumors.

The data indicated that HSP70 preferentially enhances CD8⁺ T cell responses of E7 DNA vaccines. In contrast, CD4⁺ T cell responses were not detectably enhanced by HSP70 linkage. This was demonstrated by a failure to induce detectable IFN-γ-expressing CD4+ T cells by flow cytometry and a failure to induce E7-specific antibodies. DNA vaccines encoding E7-HSP70 fusion genes increased the frequency of E7-specific CD8+ T cells by 40-fold relative to vaccines containing the wild type E7 gene (in the absence of HSP70 gene sequences). More importantly, the addition of HSP70 gene sequences to a conventional DNA vaccine (e.g., one encoding the antigen alone) converted a less effective vaccine into one with significant potency against established E7-expressing tumors. Surprisingly, E7-HSP70 fusion vaccines exclusively targeted CD8+ T cells; immunologic and antitumor effects were completely CD4 independent. These results indicate that fusion of HSP70 to an antigen gene greatly enhances the potency of DNA vaccines via CD8 dependent pathways.

Human papilloma virus (HPV)-derived antigens were chosen because HPVs, particularly HPV-16, are associated with most cervical cancers. The HPV oncogenic proteins, E6 and E7, are important in the induction and maintenance of cellular transformation and co-expressed in most HPV-containing cervical cancers. Vaccines or immunotherapies targeting E7 and/or E6 proteins can prevent and treat HPV-associated cervical malignancies. The data indicate that linking antigen-encoding polynucleotides, e.g., full-length E7-encoding sequences, to HSP-encoding sequences enhances the potency of DNA vaccines. DNA vaccines containing wild type HPV-16 E7 with DNA vaccines containing full-length E7 fused to *Mycobacterium tuberculosis* HSP70 were evaluated for their immune response generation and their ability to protect animals against the HPV-16 E7-expressing murine tumors. Linking DNA encoding E7 to DNA encoding HSP70 dramatically increases expansion and activation of E7-specific CD8+ T cells, completely bypassing the CD4 arm. This enhanced CD8 response results in potent antitumor immunity against established tumors.

The following materials and methods were used to generate the data described below.

*Plasmid DNA Constructs and Preparation:* DNA fragment encoding *Mycobacterium tuberculosis* HSP70 is known in the art (GENBANK Accession No. Z95324 AL123456; nucleotides 10633-12510 encode HSP70).

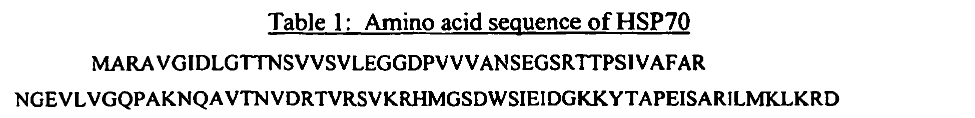

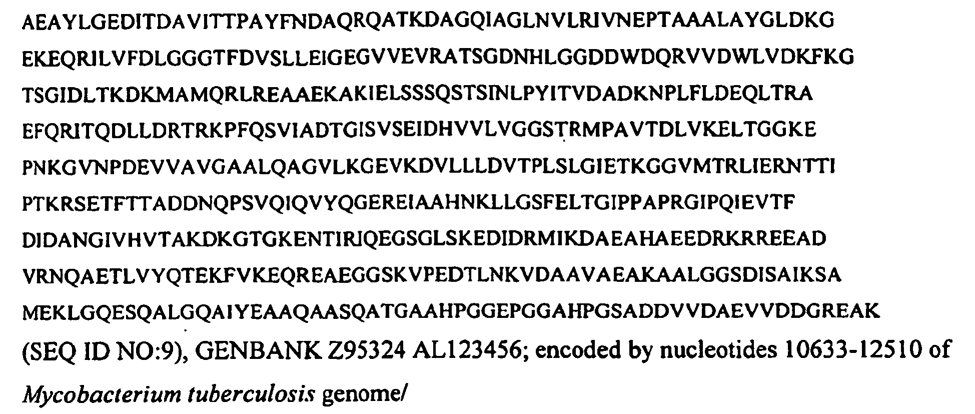

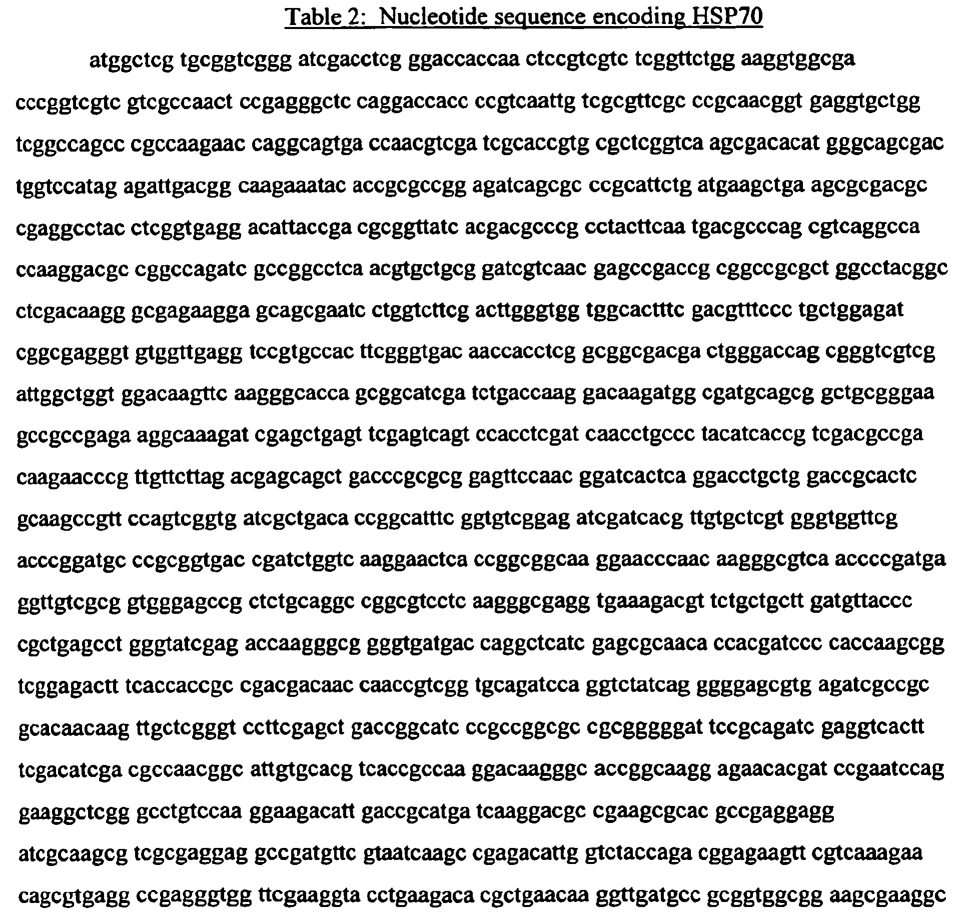

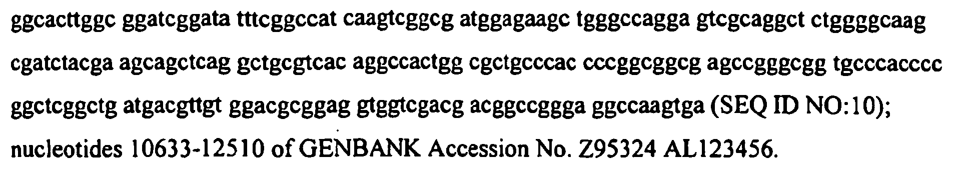

nucleotides 10633-12510 of GENBANK Accession No. Z95324 AL123456.

For the generation of HSP-expressing plasmid (pcDNA3-HSP), HSP70-encoding DNA was subcloned from pKS70 into the unique BamHI and HindIII cloning sites of the pcDNA3.1 (-) expression vector (Invitrogen, Carlsbad, CA) downstream of the cytomegalovirus promoter. For the generation of HPV-16 E7-expressing plasmid (pcDNA3-E7), E7 DNA was amplified by PCR using primers designed to generate BamHI and HindIII restriction sites at the 5' and 3' ends of the amplified fragments respectively. The amplified E7 DNA was then cloned into the unique BamHI and HindIII cloning sites of the pcDNA3.1. For the generation of E7-HSP70 chimera (pcDNA-E7-HSP70), E7 DNA was amplified by PCR using primers designed to generate BamHI restriction sites at both 5' and 3' ends of the amplified fragments. The E7 DNA was then subcloned to the 5' end of pcDNA3-HSP. The accuracy of these constructs was confirmed by DNA sequencing. Plasmid DNA with HSP, E7 or E7-HSP70 gene insert and the "empty" plasmid vector were transfected into subcloning efficient DH5 (TM cells; Life Technologies, USA). The DNA was then amplified and purified using double CsCI purification (BioServe Biotechnologies, Laurel, Maryland). The integrity of plasmid DNA and the absence of Escherichia coli DNA or RNA were checked in each preparation using 1% agarose gel electrophoresis. DNA concentration was determined by the optical density measured at 260 nm. The presence of the inserted E7 fragment was confirmed by restriction enzyme digestion and gel electrophoresis. Reticulum and calreticulum polypeptides (and polynucleotides encoding them) are a useful alternative to the HSP component of the immunogenic composition.

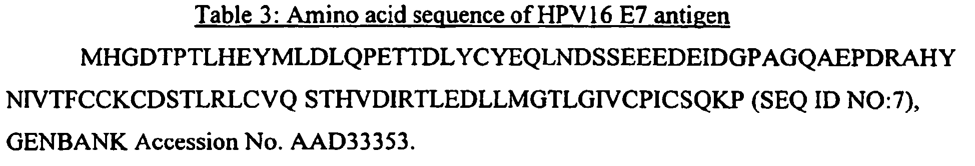

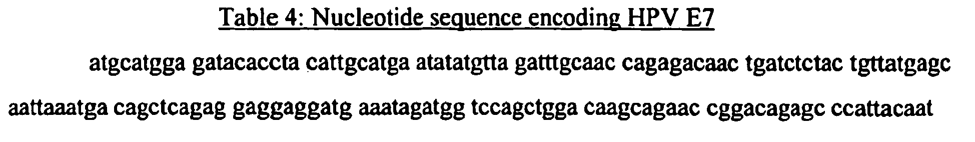

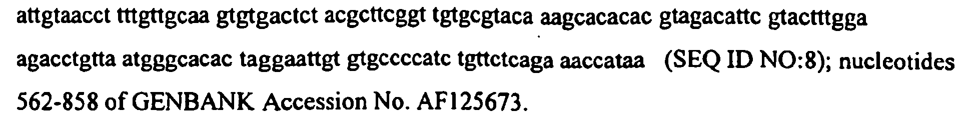

*DNA Vaccination:* Gene gun particle-mediated DNA vaccination was performed using a helium-driven gene gun (Bio-Rad, Hercules, CA, according to the standard protocols. Briefly, DNA coated gold particles were prepared by combining 25 mg of 1.6 µm gold microcarriers (Bio-rad, Hercules, CA) and 100 µl of 0.05 M spermidine (Sigma, St, Louis, MO). Plasmid DNA (50 µg) and 1.0 M CaCl₂ (100 µl) were added sequentially to the microcarriers while mixing by vortex. This mixture was allowed to precipitate at room temperature (RT) for 10 minutes. The microcarrier/DNA suspension was then centrifuged (10,000 r.p.m. for 5 sec) and washed 3 times in fresh absolute ethanol before resuspending in 3 ml of polyvinylpyrrolidone (0.1 mg/ml) (Bio-rad, Hercules, CA) in absolute ethanol. The solution was then loaded into tubing and allowed to settle for 4 min. The ethanol was gently removed and the microcarrier/DNA suspension was evenly attached to the inside surface of the tubing by rotating the tube. The tube was then dried by 0.4 liters per minute of flowing nitrogen gas. The dried tubing coated with microcarrier/DNA was then cut to 0.5-inch cartridges and stored in a capped dry bottle at 4□C. As a result, each cartridge contained 1 µg of plasmid DNA and 0.5 mg of gold. The DNA coated gold particles (1 µg DNA/bullet) were delivered to the shaved abdominal region of the mice using a helium-driven gene gun (Bio-rad, Hercules, CA) with a discharge pressure of 400 p.s.i.

*ELISPOTAssay:* A standard ELISPOT assay was used to detect HPV-16 E7-specific CD8+ T cell. The 96-well filtration plates (Millipore, Bedford, MA) were coated with 10 µg/ml rat anti-mouse IFN-γ antibody (clone R4-6A2, Pharmingen, San Diego, CA) in 50 µl of phosphate-buffered saline (PBS). After overnight incubation at 4°C, the wells were washed and blocked with culture medium containing 10% fetal bovine serum. Different concentrations of fresh isolated spleen cells from each vaccinated mice group, starting from 1x10⁶ /well, were added to the well along with 15 U/ml interleukin-2. Cells were incubated at 37°C for 24 hours either with or without 1 µg/ml E7 specific H-2D^{b} CTL epitope (E7, residues 49-57; SEQ ID NO:5). After culture, the plate was washed and then the cells were incubated with 5 µg/ml biotinylated IFN-γ antibody (clone XMG 1.2, Pharmingen) in 50 µl in PBS at 4°C overnight. After washing six times, 1.25 µg/ml avidin-alkaline phosphatase (Sigma, St. Louis, MO) in 50 µl PBS, were added and incubated for 2 hours at room temperature. After washing, spots were developed by adding 50 µl BCIP/NBT solution (Boehringer Mannheim, Indianapolis, IN) and incubated at room temperature for 1 hr. The spots were counted using a dissecting microscope.

*Intracytoplasmic Cytokine Staining and Flow Cytometry:* Splenocytes from naive or vaccinated groups of mice were incubated either with the E7 peptide (residues 49-57; SEQ ID NO:5) that contains MHC class I epitope or the E7 peptide (residues 30-67; SEQ ID NO:6) that contains MHC class II peptide. Methods of determining which residues of a protein or polypeptide represent a MHC class I antigenic epitope are well known in the art. The E7 peptide was added at a concentration of 2 µg/ml for 20 hours. To detect E7-specific CD8⁺ T cell precursors and E7-specific CD4⁺T helper cell responses, CD8⁺ CTL epitopes residues 49-57 and residues 30-67 were used, respectively. Golgistop^{™} (Pharmigen, San Diego, CA) was added 6 hours before harvesting the cells from the culture. Cells were then washed once in FACSCAN buffer and stained with phycoerythrin (PE)-conjugated monoclonal rat anti-mouse CD8 or CD4 antibody (Pharmingen, San Diego, CA). Cells were subjected to intracellular cytokine staining using the Cytofix/Cytoperm^{™} kit according to the manufacturer's instructions (Pharmingen). FITC-conjugated anti-IFN-γ or anti-IL-4 antibodies and the immunoglobulin isotype control antibody (rat IgG1) were all purchased from Pharmingen. Analysis was done on a Becton-Dickenson FACScan^{™} with CELLQuest^{™} software (Becton Dickson Immunocytometry System, Mountain View, CA).

*ELISA for Cytokines:* Splenocytes (4x 10⁶) were harvested 2 weeks after the last vaccination and cultured with 10 µg/ml E7 protein in a total volume of 2 ml of RPMI 1640, supplemented with 10% (vol/vol) fetal bovine serum, 50 units/ml penicillin/streptomycin, 2mM L-glutamine, 1 mM sodium pyruvate, 2mM nonessential amino acids in a 24-well tissue culture plate for 72 hours. The supernatants were harvested and assayed for the presence of IFN-γ and IL-4 using ELISA kits (Endogen) according to the manufacturer's protocol.

*Anti-E7 ELISA:* The anti-HPV 16 E7 antibodies in the sera were determined by a standard direct ELISA. A 96 microwell plate was coated with 100 µl 10 µg/ml bacteria-derived HPV-16 E7 proteins and incubated at 4°C overnight. The wells were then blocked with PBS containing 20% fetal bovine serum. Sera were prepared from the mice on day 14 post-immunization, serially diluted in PBS, added to the ELISA wells, and incubated on 37°C for 2 hr. After washing with PBS containing 0.05% Tween-20, the plate was incubated with 1/2000 dilution of a peroxidase-conjugated rabbit anti-mouse IgG antibody (Zymed, San Francisco, CA) at room temperature for one hour. The plate was washed 6 times, developed with TMB (Pierce, Rockford, IL), and stopped with 1M H₂SO₄. The ELISA plate was read with a standard ELISA reader at 450 nm.

*Murine Tumor Cell Line:* Murine tumor cell line TC-1 was grown and maintained using standard tissue culture methods. Growth of tumor cell lines and formation of tumors by transformed cells in mice is an art-recognized model of human cancer. HPV-16 E6, E7 and ras oncogene were used to transform primary C57BL/6 mice lung epithelial cells. The cells were grown in RPMI 1640, supplemented with 10% (vol/vol) fetal bovine serum, 50 units/ml penicillin/ streptomycin, 2mM L-glutamine, 1mM sodium pyruvate, 2mM nonessential amino acids and 0.4 mg/ml G418 at 370C with 5% CO₂. On the day of tumor challenge, TC-1 cells were harvested by trypsinization, washed twice with 1X Hanks buffered salt solution and finally resuspended in 1X Hanks buffered salt solution to the designated concentration for injection.

*Mice:* Six to eight-week-old male C57BL/6 mice were purchased from the National Cancer Institute (Frederick, Maryland).

*In Vivo Tumor Protection:* Mice (5 per group) were vaccinated via gene gun with 2 µg of HSP DNA, E7 DNA, E7-HSP70 DNA, or the empty plasmid without insert. One week later, the mice were boosted with the same regimen as the first vaccination. Another set of mice (5 per group) was vaccinated once (without further booster). One week after the last vaccination, mice were subcutaneously challenged with 5×10⁴ cells/mouse TC-1 tumor cells in the right leg and then monitored twice a week.

*In Vivo Tumor Treatment:* The tumor cells and the DNA vaccines were prepared as described above. The mice were subcutaneously challenged with 2×10⁴ cells/mouse TC-1 tumor cells in the right legs. Three days after challenge with TC-1 tumor cells, mice were given 2 µg of HSP DNA, E7 DNA, E7-HSP70 DNA, or the empty plasmid without insert via gene gun. One week later, these mice were boosted with the same regimen as the first vaccination. Another set of mice (5 per group) were vaccinated once without further booster after the tumor challenges. Mice were monitored twice a week.

*In Vivo Antibody Depletion: In vivo* antibody depletions were carried out using standard methods. C57BL/6 mice were vaccinated with 2 µg E7-HSP70 DNA via gene gun, boosted one week later, and challenged with 5×10⁴ cells/mouse TC-1 tumor cells. Depletions were started one week prior to tumor challenge. A CD4-specific monoclonal antibody, e.g., MAb GK1.5, was used for CD4 depletion; a CD8-specific antibody e.g., MAb 2.43, was used for CD8 depletion, and an antibody which binds to NK cells, e.g., MAb PK136, was used for NK1.1 depletion. Flow cytometry analysis revealed that the greater than 95% of the appropriate lymphocytes subset were depleted with normal level of other subsets. Depletion was terminated on day 40 after tumor challenge.

*Vaccination with E7-HSP70 Fusion DNA Enhances E7-Specific CD8⁺ T Cell-Mediated Immune Responses:* CD8⁺ T lymphocytes are one of the most crucial components among antitumor effectors. To determine the E7-specific CD8⁺ T cell precursor frequencies generated by E7-HSP70 DNA vaccines, enzyme-linked immunospot (ELISPOT) assays and intracellular cytokine stains were used. Both ELISPOT assay and intracellular cytokine staining are sensitive functional assays used to measure IFN-γ production at the single-cell level, which can thus be applied to quantify antigen-specific CD8⁺ T cells. As shown in Fig. 1A, 435 IFN-γ spot-forming CD8⁺ T cells specific for the immunodominant D^{b}-restricted E7 (49-57; SEQ ID NO:5) peptide were detected per 10⁶ splenocytes derived from the E7-HSP70 DNA vaccinated mice, compared to only 14 E7(49-57)-specific IFN-γ spot-forming CD8⁺ T cells/10⁶ splenocytes derived from the E7 DNA vaccinated mice. Subtracting the background produced by the pcDNA-3 vector alone (3 Spots/10⁶ splenocytes) yielded 11 and 432 E7 (49-57)-specific IFN-γ spot-forming CD8+ T cells for the E7 and E7-HSP70 DNA vaccines, respectively. Similarly, the quantity of E7 (49-57)-specific CD8⁺ T cell precursors can also be determined by flow cytometry analysis using double staining for CD8 and intracellular IFN-γ. Values from this assay (Fig. 1B) correlated closely with the ELISPOT results presented in Fig. 1A. As shown in Fig. 1B, mice vaccinated with E7-HSP70 DNA generated the highest number of E7-specific IFN-γ⁺ CD8⁺ T cell precursors (around 726/10⁶ splenocytes) by flow cytometry analysis, whereas mice vaccinated with E7 DNA showed no signal above the vector-vaccinated controls. These data indicate that DNA encoding a MHC class I-restricted epitope of E7 operably linked to DNA encoding HSP70 or a fragment thereof led to at least a 30-fold increase in the E7-specific CD8⁺ T cell precursor frequencies.

*Vaccination with E7-HSP70 Fusion DNA does not Enhance E7-Specific CD4⁺ T Cell-Mediated Immune Responses:* To determine E7-specific CD4⁺ T precursor cells and the cytokine profiles generated by E7-HSP70 DNA vaccines, double staining for CD4 surface marker and intracellular IFN-γ or IL-4 on splenocytes from immunized mice was carried out, followed by flow cytometry analysis. The splenocytes from immunized mice were cultured in vitro with E7 peptide (residues 30-67; SEQ ID NO:6) overnight and stained for both CD4 and intracellular IFN-γ. The E7 peptide (residues 30-67; SEQ ID NO:6) contains a major T helper epitope in the E7 open reading frame protein of HPV-16. The percentage of IFN-γ secreting CD4⁺ T cells was analyzed using flow cytometry. As shown in Fig. 2, mice vaccinated with E7-HSP70 DNA generated a similar number of CD4⁺ IFN-γ⁺ double positive cells compared to mice vaccinated with wild-type E7 DNA or vector alone. Mice vaccinated with E7 DNA mixed with HSP70 plasmid generated slightly higher CD4⁺ IFN-γ⁺ double positive cells, but there is no statistically significant difference in the CD4⁺ IFN-γ⁺ double positive cell numbers between each vaccinated group.

As a positive control for the ability of CD4 vs. intracellular IFN-γ staining to detect E7-specific CD4+T cells, analysis of mice vaccinated with a DNA vaccine expressing Sig/E7/LAMP-1, which targets E7 to the MHC class II compartment, demonstrated a 3 fold increase in the CD4⁺ IFN-γ⁺ double positive cells relative to mice vaccinated with E7 DNA or control plasmid. IL-4 secreting E7-specific CD4⁺ T cell in mice vaccinated with various DNA vaccines were also analyzed. No significant CD4⁺ IL-4⁺ double-positive cells could be identified in the mice that received E7-HSP70 DNA, E7 DNA mixed with HSP70 DNA, wild type E7 DNA, empty plasmid DNA, or no vaccination. MICK-2 IL-4 secreting cells (Pharmingen, San Diego, CA) were used as positive controls to ensure the success of intracytoplasmic IL-4 staining for this study. These data indicate that the DNA vaccines of the invention which contain DNA encoding all or part of a heat shock protein induce a CD8⁺ immune response to the epitope encoded by the vaccine independent of a CD4⁺ immune response.

*Vaccination with E7-HSP70 Fusion DNA dose not Generate E7-Specific Antibodies:* The quantity of anti-HPV 16 E7 antibodies in the sera of the vaccinated mice was determined by a direct enzyme-linked immunoabsorbent assay (ELISA) 2 weeks after the last vaccination. No anti-E7 antibodies could be detected in the sera of mice of any vaccinated group (Fig. 3). The commercial anti-E7 monoclonal antibody (Zymed, San Francisco, CA) and sera from mice vaccinated with vaccinia virus containing the Sig/E7/LAMP-1 chimera were used as positive controls to ensure the success of anti-E7 ELISA for this study. This result is consistent with the complete absence of apparent E7-specific CD4 stimulation by either E7 DNA or E7-HSP70 DNA vaccines.

*Vaccination with E7-HSP70 Fusion DNA Enhances Protection Against the Growth of Tumors:* To determine whether vaccination with the E7-HSP70 DNA construct protects mice against E7-expressing tumors, two *in vivo* tumor protection experiments were performed using different doses of DNA vaccines. For the first experiment, mice were vaccinated with 2 µg naked DNA/mouse via gene gun and boosted with the same dose one week later. For the second experiment, mice were vaccinated with 2 µg naked DNA/mouse via gene gun without further booster. The mice were then challenged with 5x10⁴ TC-1/-mouse subcutaneously in the right leg 7 days after the last vaccination. For the mice receiving vaccination with booster, 100% of those receiving E7-HSP70 DNA vaccination remained tumor-free 60 days after TC-1 challenge, while only 40% of mice receiving E7 DNA (in the absence of HSP-encoding DNA) vaccination remained tumor-free. In contrast, all of the unvaccinated mice and mice receiving empty plasmid or HSP DNA developed tumor growth within 15 days after tumor challenge (Fig. 4A). For the mice receiving vaccination once without booster, 100% of those receiving E7-HSP70 DNA vaccination remained tumor-free 60 days after TC-1 challenge, whereas all of the unvaccinated mice and mice receiving empty plasmid, HSP70 DNA or E7 DNA developed tumor growth within 15 days after tumor challenge (Fig. 4B).

To determine whether the antitumor effect generated by E7-HSP70 DNA is long-lasting, these tumor free animals were rechallenged with the 5×10⁴ TC-1 cells/mouse subcutaneously in the left leg. No tumor growth was observed up to 30 days after tumor rechallenge (Figs. 4A-B). In summary, these results indicated that a DNA construct encoding a MHC class I-restricted antigenic epitope operably linked to DNA encoding a HSP polypeptide, e.g, E7-HSP70 fusion DNA, significantly enhances the antitumor immunity against the growth of a tumor which express the class I-restricted epitope, e.g., TC-1 tumors. The antitumor effect was observed even when tested under more stringent conditions (no booster vaccine administered). The antitumor immunity generated by E7-HSP70 DNA construct was long lasting.

*Therapeutic Vaccination with E7-HSP70 Fusion DNA Cures Mice with Established E7-Expressing Tumors:* To test the efficacy of DNA vaccines in eradicating established TC-1 tumors, *in vivo* tumor treatment experiments were performed using different doses of DNA vaccines. TC-1 cells were first injected into C57BL/6 mice subcutaneously at a dose of 2×10⁴ cells per mouse in the right leg. Three days later, each mouse was treated with 2 µg of either control plasmid DNA, HSP70 DNA, wild type E7 DNA, or E7-HSP70 DNA intradermally via gene gun. For the first experiment, mice were boosted with the same vaccine dose 7 days after priming. For the second experiment, mice did not receive further booster after priming. As shown in Fig. 5A, for mice receiving boosted DNA vaccination, TC-1 tumor was eliminated from 80% of mice receiving the E7-HSP70 DNA vaccination, whereas all of the unvaccinated mice and mice receiving empty plasmid, HSP70 DNA or E7 DNA developed tumor growth within 20 days after tumor challenge. For the mice receiving vaccination once without booster, 60% of those receiving E7-HSP70 DNA vaccination remained tumor-free 70 days after TC-1 challenge, whereas all of the unvaccinated mice and mice receiving empty plasmid, HSP70 DNA, or E7 DNA developed tumor growth within 20 days after tumor challenge (Fig. 5B). In summary, these results showed that vaccination with wild-type E7 DNA (in the absence of DNA encoding an HSP polypeptide) failed to eradicate the previously-inoculated E7-expressing tumors in mice, while vaccination with DNA encoding E7 operably linked to DNA encoding an HSP polypeptide, e.g., E7-HSP70 DNA, eradicated the established E7-expressing tumors. These data indicated that E7-HSP70 DNA significantly enhanced antitumor immunity, and that the presence of DNA encoding an HSP polypeptide in a DNA vaccine construct enhances the antitumor immunity generated by the DNA vaccine compared to the level of immunity generated by a conventional DNA vaccine (i.e., one without DNA encoding an HSP polypeptide).

*Fusion of DNA encoding an antigenic epilope to HSP70-encoding sequences is Required for the Generation of Antitumor Immunity:* To determine whether fusion of E7 to HSP70 was required for antitumor immunity, E7-HSP70 DNA was compared to E7-DNA mixed with HSP70 DNA and the ability to generate the antitumor immunity measured. To ensure that both E7 DNA and HSP70 DNA were delivered into the same cell, the E7 DNA was mixed with HSP70 DNA before the bullet preparation. Thus, a single bullet would contain both E7 DNA and HSP70 DNA. Immunological assays and tumor protection experiments (without vaccination booster) were carried out as described above. The data indicated that only E7-HSP70 DNA enhanced the E7-specific CD8+ T cell-mediated immune responses. Mixing HSP70 DNA with E7 DNA did not enhance the CD8+ T cell-mediated immune responses. For the tumor protection experiment, all of the mice vaccinated with E7-HSP70 DNA remained tumor-free 60 days after tumor challenge. In contrast, all of the unvaccinated mice or mice vaccinated with E7 DNA mixed with HSP70 DNA developed tumor growth within 15 days after tumor challenge (Fig. 6). Fusion of E7 DNA to HSP70 DNA was essential for the generation of antitumor immunity. These results indicate that DNA encoding a MHC class I-restricted antigenic epitope must be operably linked to DNA encoding an APC-binding epitope and/or DNA encoding a cytoplasmic translocation polypeptide.

*CD8⁺ T Cells But Not CD4+ T cells or NK cells Are Essential for the Antitumor Effect Generated by DNA Vaccine with E7 Fused to HSP70:* To determine the subset of lymphocytes that are important for the rejection of E7-positive tumor cells, *in vivo* antibody depletion experiments were performed. The antibody depletion was started one week before the tumor challenge and terminated on day 40 after tumor challenge. As shown in Fig. 7, all naive mice and all of the mice depleted of CD8⁺ T cells grew tumors within 14 days after tumor challenge. In contrast, all of the non-depleted mice and all of the mice depleted of CD4⁺ T cells or NK1.1 cells remained tumor-free 50 days after tumor challenge. These results indicate that DNA vaccine activated CD8⁺ T cells in a CD4-independent fashion, and that these CD8⁺ T cells are essential for the antitumor immunity generated by E7-HSP70 DNA vaccine.

*The role of HSP in stimulating CD8⁺ T cells:* While the invention is not limited by any particular mechanism, the HSP70 domain of the chimeric nucleic acid and DNA vaccines of the invention may be acting as a "chaperone" to the antigen, e.g., it may be a chaperone to move the antigenic peptide to the appropriate cell type to optimize induction of CD8⁺ T cells. Ballistic DNA delivery introduces DNA directly into dermal precursors. The E7-HSP70 DNA-transfected dendritic cells (DCs) expressed HSP70. HSP70 is a cytosolic HSP that plays multiple roles in protein folding, transport, and degradation. It is involved in processing MHC class I restricted antigens. HSP fusion products encoded by the DNA vaccines thus may be targeted more efficiently for proteasomal processing.

CD8⁺ T cells may be the key players in gene gun-mediated E7-HPS70 DNA vaccination. The data described herein showed that CD8⁺ T cells are required in the effector phase of antitumor immunity. In contrast, depletion of CD4⁺ or NK1.1⁺ cells did not decrease the antitumor immunity generated byE7-HSP70 DNA. The finding is in contrast to approaches using protein-based HSP vaccines, which showed that CD4⁺ and CD8⁺ T cells and NK cells are required in the effector phases of antitumor immunity using gp96 preparations from tumor cells.

HSP complexes taken up by professional APC may be involved in introducing HSP-associated peptides into the MHC-I antigen presentation pathway. Only activated B cells and mononuclear cells may be taking up HSP70, while activated T cells may not be transporting HSP70. Though the receptor-mediated uptake of HSP is important for HSP/peptide complex protein vaccines, it is unlikely to play a role in the gene gun-mediated E7-HSP70 DNA vaccines. The recombinant E7-HSP70 fusion gene encoded by the DNA vaccine was sent directly into DCs via gene gun, bypassing the need for receptor-mediated endocytosis. Cross-priming of APCs may also occur because E7-HSP70 might be released from other cell types, such as keratinocytes (which were also transfected by gene gun vaccination), and then enter the DCs via receptor-mediated endocytosis.

The requirement of the fusion of E7 to HSP70 suggests that E7 is becoming more immunogenic possibly because of the addition of HSP70 T cell epitopes. Vaccination with HSP70 DNA expands the pool of HSP70-reactive T cells that may exist in an individual (due to prior exposure to pathogens). These HSP70-reactive T cells may be exerting a strong helper effect reacting to conjugated peptides; this may have been the mechanisms that led to an increase in the E7-specific CD8+ T cell precursor frequency detected by ELISPOT assay and by intracellular cytokine staining observed in the mice vaccinated with E7-HSP70 fusion gene, but not in the mice vaccinated with E7 plasmid mixed with HSP70 plasmid.

HSPs may directly activate T cells *in vivo* and *in vitro* via an antigen-independent mechanism. In the absence of antigenic peptides, HSP can induce the secretion of TNF-α and IFN-γ of the antigen-specific CTL clones. Human HSP-60 can act as a danger signal to the innate immune system and can induce a T helper 1 (TH1) proinflammatory response. However, a significant increase in the numbers of E7-specific CD4+ T cells was not observed in mice vaccinated with HSP70 DNA alone or E7 plasmid mixed with HSP70 plasmids. γδ T cells stimulated by the HSP component of the DNA vaccine may also participate in the antitumor effect generated by E7-HSP70 DNA vaccines.

While E7-HSP70 generates potent CD8+ T cell responses through enhanced MHC class I presentation, other constructs that target antigen to MHC class II presentation pathways may provide enhanced CD4+ T cell responses. Administration of vaccines that directly enhance MHC class I and class II restricted pathways may be co-administered. For example, a DNA construct in which the second DNA encodes a LAMP-1 endosomal/lysosomal targeting signal for enhancing the MHC class II presentation pathway of E7, e.g., chimeric Sig/E7/LAMP-1 DNA vaccine can be co-administered with the nucleic acids and vaccines of the invention to stimulate antigen-specific CD4⁺ T cells. The E7-HSP70 vaccine described herein in conjunction with a MHC class II-targeting vaccine such as Sig/E7/LAMP-1 may activate multiple arms of the immune system in a synergistic fashion, leading to significantly enhanced CD4+ and CD8+ T cell responses and potent antitumor effects.

These results demonstrate that an exemplary chimeric nucleic acid of the invention (a DNA fusion construct encoding a HSP70 and HPV-16 E7 fusion protein), in the form of a DNA vaccine administered by ballistics, can generate stronger E7-specific CD8+ T cell-mediated immune responses and antitumor effects against HPV-16 E7-expressing murine tumors than by DNA vaccines encoding the antigen E7 alone. These data therefore demonstrate that fusion of HSP70 sequences (and, as described herein, fusion of the carboxy-terminal HSP domain) to an antigen gene (or fragment thereof) greatly enhances the potency of DNA vaccines. This strategy is applicable to the prevention and treatment of pathologies, e.g., infectious diseases, and to tumors and cancer systems, particularly those with known tumor-specific antigens.

### Example 2: Enhancement of DNA Vaccine Potency by Linkage of DNA Sequence Encoding GM-CSF and Translocation Domain of a Pseudomonas Exotoxin A to an Antigen Gene

The following example describes studies that demonstrate that the compositions and methods described are effective for enhancing antigen-specific cytotoxic T lymphocyte (CTL) responses and treating tumors *in vivo.*

A chimeric nucleic acid encoding an immunogenic "fusion protein" composition was constructed, wherein the DNA encoded a GM-CSF (or a fragment thereof) domain (operably linked) to an antigen-encoding sequence. While the method is not limited by any particular mechanism, the resulting fusion protein may have enhanced targeting of the antigen contained therein to cells expressing GM-CSF receptors, such as professional APCs and their precursors. The presence of DNA encoding an ETA polypeptide, e.g, domain II of *Pseudomonas* exotoxin A (ETA(dII) (SEQ ID NO:3), may mediate translocation of the antigen from endosomal/lysosomal compartments to the cytoplasm to enhance MHC class I presentation.

A DNA chimera (GM-ETA(dII)-E7) was made by fusing DNA sequences encoding GM-CSF and ETA(DII) to the gene of a model antigen, E7. An art-recognized mouse model of human cancer was used to evaluate the immunogenicity and anti-tumor activity elicited by the construct. Immunization of mice using the GM-ETA(dII)-E7 chimera DNA vaccine enhanced E7-specific CD8+ and CD4+ T cell immune responses and effective anti-tumor protection and treatment against an E7-expressing murine tumor cell line (TC-1) through intramuscular or intradermal injection. This dramatic anti-tumor effect was not observed in DNA vaccines encoding other constructs, particularly GM-E7, ETA(dII)-E7, or GM-ETA(dII) The DNA vaccination strategy described herein is useful for antigen specific immunotherapy for cancers and infection by pathogenic microorganisms.

*Improvement of MHC class I presentation of exogenous antigen is a strategy for enhancing DNA vaccine potency that may be tested using domain II of Pseudomonas exotoxin A (ETA(dII)):* ETA is a toxin derived from the bacterium *Pseudomonas aeruginosa* that blocks protein synthesis. Domain II, or ETA(dII), allows translocation from endosomal/lysosomal compartments to the cytoplasm, which leads to enhancement of MHC class I presentation of exogenous antigen. Since domain I is responsible for LDL-binding and domain III has the toxic capability of binding with ADP-ribosyl transferase, these unwanted portions of ETA are replaced or deleted. The data described below was generated using the DNA chimera (GM-ETA(dII)-E7). HPV-16 E7 was used as the model antigen because it is a clinically relevant an oncogenic protein that is widely expressed and well-characterized in HPV-associated malignancies. GM-CSF DNA can be incorporated into various APCs, inducing enhancement of dendritic cell (DC) maturation. By linkage with antigen, GM-CSF may also enhance targeting of the antigen to cells expressing GM-CSF receptor (GM-CSF-R), such as professional APCs and their precursors. ETA(dII) linked with antigen in a DNA form may be able to translocate antigen from the endosomal/lysosomal compartment to the cytoplasm, leading to enhanced MHC class I presentation. Vaccination with a chimeric DNA construct designed according to the GM-ETA(dII)-E7 example provide the necessary potency for activation of both MHC class I and II-restricted pathways, leading to enhanced E7-specific immune responses and anti-tumor effects.

The following materials and methods were used to generate the data described below.

*Plasmid DNA Constructs and Preparation:* DNA fragment encoding murine GM-CSF was obtained by PCR with 5'end primer, 5'-GGGGAGATCTGGATGTGGCTGCAG AATTTA-3' (SEQ ID NO:11) and 3'end primer (EcoRI), 5'- GGGAGAATTCTTTTGG ACTGGTTTTTTGCAT-3' (SEQ ID NO:12). cDNA generated from GM-CSF-transduced B-16 melanoma cell was used as temple for PCR.

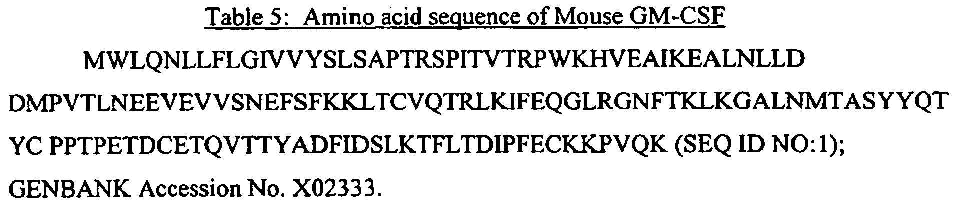

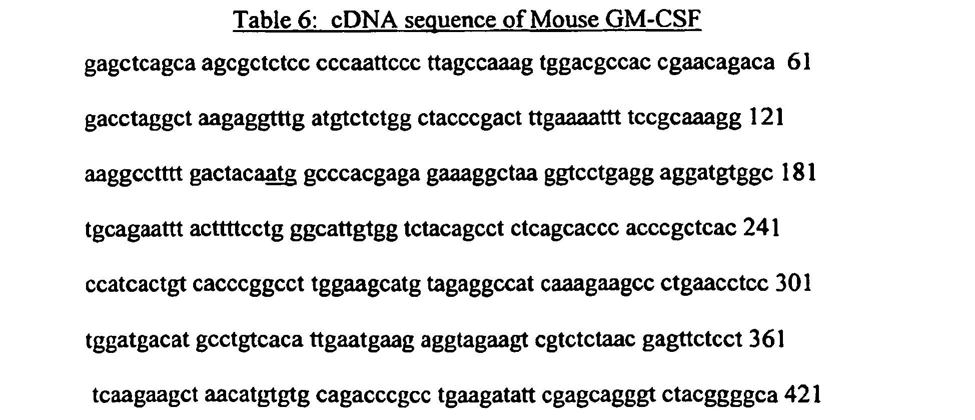

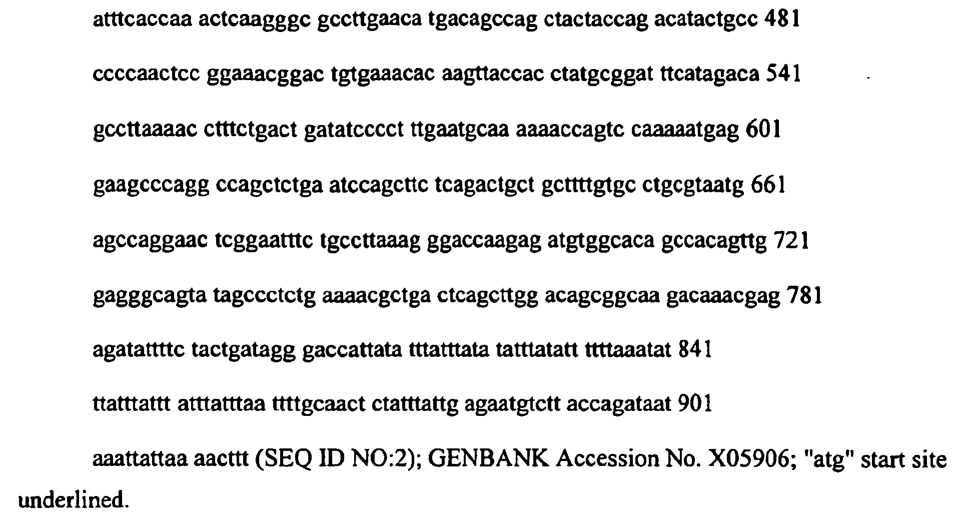

The amplified products were subsequently cloned into BgIII and EcoRI sites of pSP73 vector (Promega) to generate pSP73-GM. The same strategy was employed to make a DNA construct using human GM-CSF sequences (GENBANK Accession M11220). Alternatively, the first DNA encodes all or an APC-binding fragment of Flt-3 ligand (FL), CTLA-4, 4-1BB, CD40 ligand, or TNF receptor. The amino acid sequences and nucleotide sequences of these proteins are readily available to those skilled in the art. Determining whether a give polypeptide or protein fragment binds to an antigen presenting cell (APC) (e.g., a macrophage or DC) is also well known in the art. For example, the polypeptide (as well as a control polypeptide known not to bind to an APC) is labeled with a detectable marker and incubated with an APC. After the cells are washed, detection of the label on or in APC's (compared to the control) indicates that the polypeptide has an APC binding domain or sequence.

The DNA fragment containing the domain II of ETA was generated using PCR with 5' end primer, 5'-GGGAGAATTCTCGCCTGTCACTTTCCCGAG-3' (SEQ ID NO:13) and 3' end primer, 5□-GGAAGGATCCAGTTCTGCGTGCCGCGGG-3' (SEQ ID NO:14).

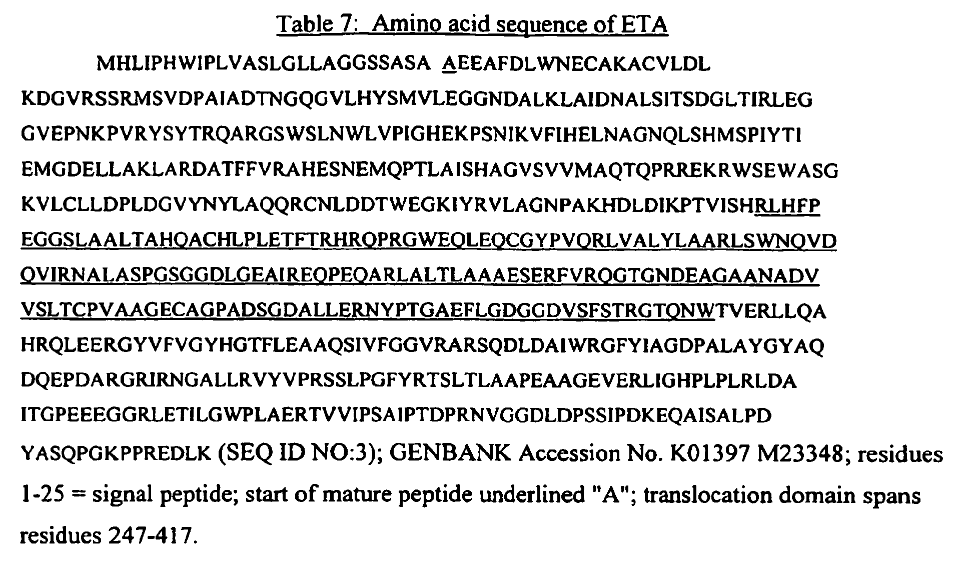

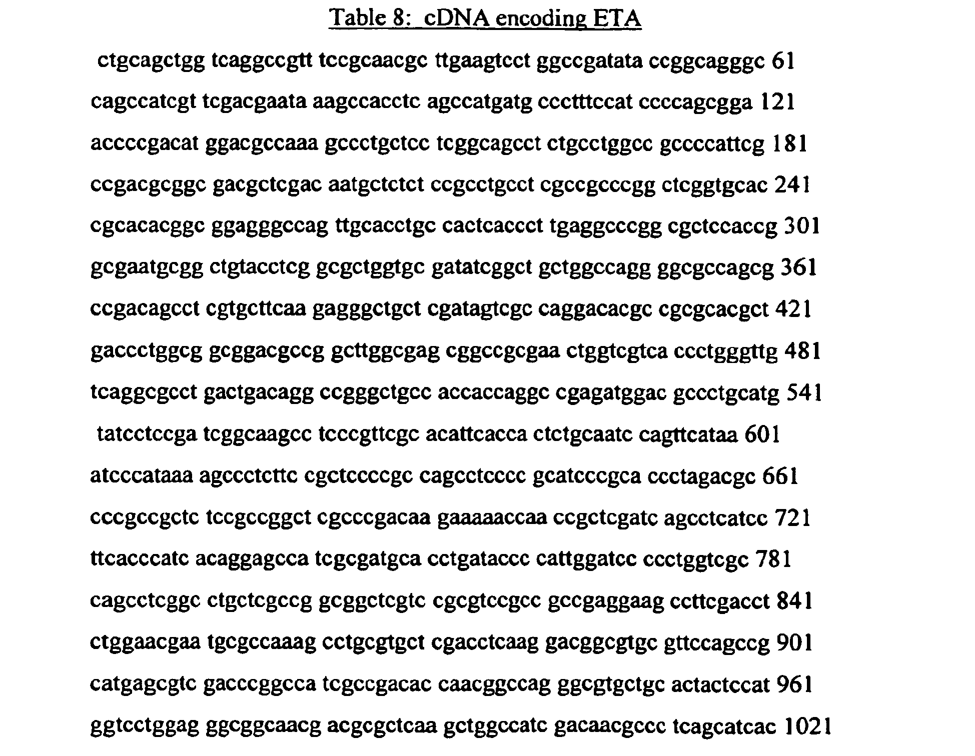

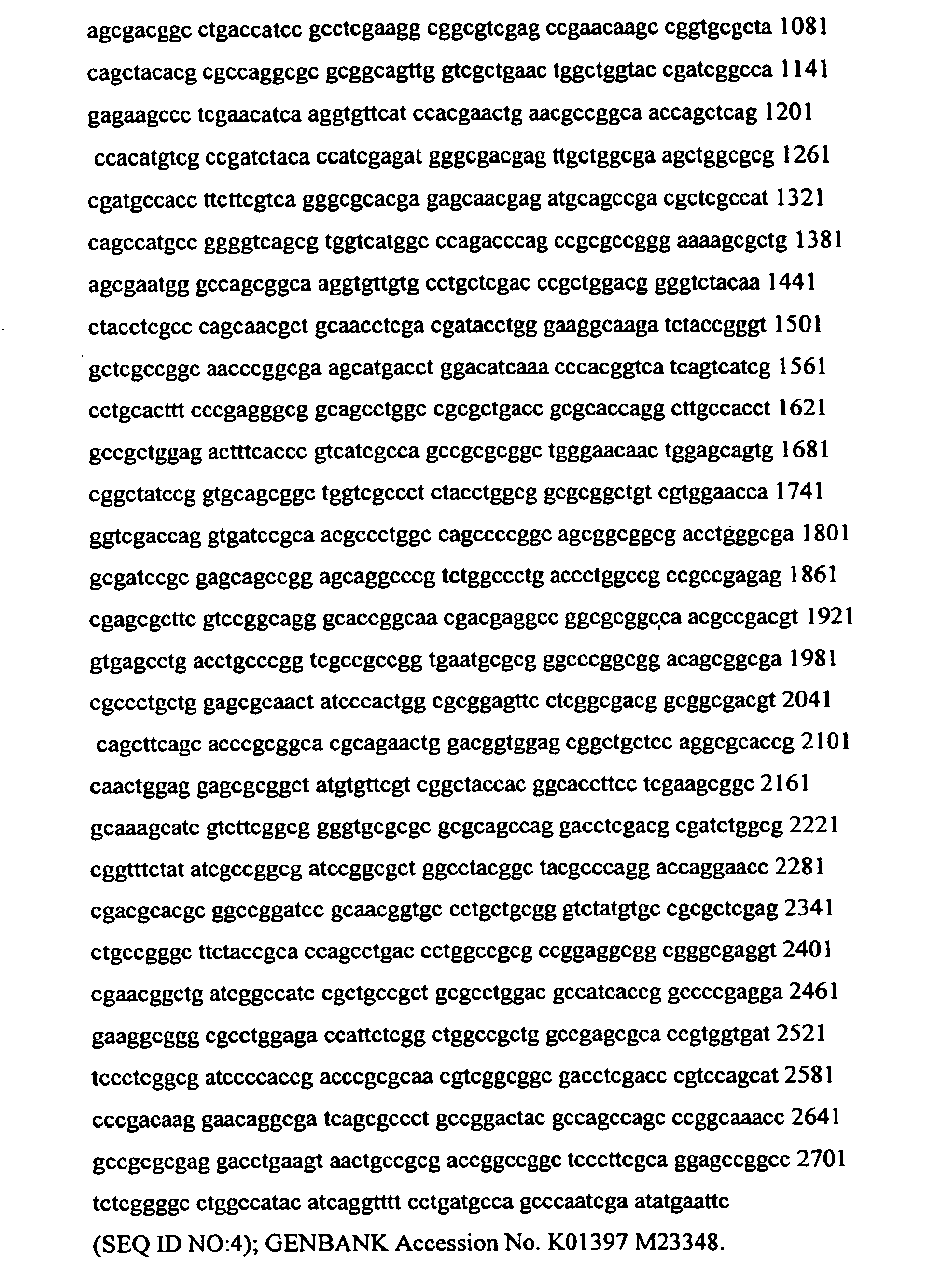

The amplified products were subsequently cloned into EcoRI and BamHI sites of pSP73-GM to generate pSP73-GM-ETA(dII). The DNA fragment containing HPV-16 E7 was synthesized by PCR with 5' end primer, 5'-GGGAGGATCCTCATGCATGGAGATAC ACCT-3' (SEQ **I**D NO:15) and 3' end primer, 5'-GGGGAAGCTTATCCTGAGAACAGA TGGGG-3' (SEQ ID N0:16). The plasmid pCMV neoBamHI-E7 was used as temple for the PCR reaction. The amplified products were further cloned into BamHI and HindIII sites of pSP73-GM-ETA(dII) to generate pSP73-GM-ETA(dII)-E7. The sequence of GM-ETA(dII)-E7 was confirmed by DNA sequencing. The pcDNA3.1-GM-ETA(dII)-E7 was obtained by digesting pSP73-GM-ETA(dII)-E7 with BgIII and HindII restriction enzymes and cloned the isolated GM-ETA(dII)-E7 fragment into the unique BamHI and HindIII cloning sites of the pcDNA3.1(-) expression vector (Invitrogen, Carlsbad, CA) downstream of the cytomegalovirus promoter. The pcDNA3.1-GM-ETA(dII) was generated by digesting pSP73-GM-ETA(dII)-E7 with BgIII and BamHI restriction enzymes and cloned the isolated GM-ETA(dII) fragment into the unique BamHI cloning site of the pcDNA3.1(-). The pcDNA3.1-ETA(dII)-E7 was obtained by digesting pSP73-GM-ETA(dII)-E7 with EcoRI and HindIII restriction enzymes and cloned the isolated ETA(dII)-E7 fragment into the unique EcoRI and HindIII cloning sites of the pcDNA3.1(-). The pcDNA3.1-GM was generated by digesting pSP73-GM-ETA(dII)-E7 with BgIII and BamHI restriction enzymes and cloned the isolated murine GM-CSF fragment into the unique BamHI cloning site of the pcDNA3.1 (-). The pcDNA3.1-ETA(dII) was obtained by digesting pSP73-GM-ETA(dII)-E7 with EcoRI and BamHI restriction enzymes and cloned the isolated ETA(dII) fragment into the unique EcoRI and BamHI cloning sites of the pcDNA3. 1(-). The pcDNA3.1-E7 was generated by digesting pSP73-GM-ETA(dII)-E7 with BamHI and HindIII restriction enzymes and cloned the isolated E7 fragment into the unique BamHI and HindIII cloning sites of the pcDNA3.1 (-). For the generation of pcDNA3.1-GM-E7, The E7 fragment was synthesized by PCR with a 5' end primer, 5'-CCCAGATCTAATCATGCATG -3' (SEQ ID NO:17) and 3' end primer, 5'-GGGGAAGCTTATCCTGAGAACAGATGGGG-3' (SEQ ID NO:18). The amplified E7 products were digested with BgIII and HindIII and further cloned into BamHI and HindIII sites of pSP73-GM. The GM-E7 fragment was isolated from pSP73-GM-E7 by digestion with BgIII and HindIII and cloned into the unique BamHI and HindII sites of pcDNA3.1(-).

The accuracy of these constructs was confirmed by DNA sequencing. Plasmid DNA with GM, ETA(dII), E7, GM-ETA(dII), ETA(DII)-E7 , GM-E7 or GM-ETA(DII)-E7 gene insert and the "empty" plasmid vector were transfected into subcloning efficient DH5(TM cells (Life Technologies, USA). The DNA was then amplified and purified using double CsCl purification (BioServe Biotechnologies, Laurel, Maryland). The integrity of plasmid DNA and the absence of Escherichia coli DNA or RNA were checked in each preparation using 1% agarose gel electrophoresis. DNA concentration was determined by the optical density measured at 260 nm. The presence of the inserted E7 fragment was confirmed by restriction enzyme digestion and gel electrophoresis.

*DNA Vaccination:* Gene gun particle-mediated DNA vaccination was performed using a helium-driven gene gun (Bio-Rad, Hercules, CA) according to standard protocols and as described above.

*Intracytoplasmic Cytokine Staining and Flow Cytometry:* Splenocytes from naïve or vaccinated groups of mice were incubated either with the E7 peptide (residues 49-57; SEQ ID NO:5) that contains MHC class I epitope or the E7 peptide (residues 30-67) that contains MHC class II peptide. The E7 peptide was added at a concentration of 2 µg/ml for 20 hours. To detect E7-specific CD8⁺ T cell precursors and E7-specific CD4⁺T helper cell responses, CD8⁺ CTL epitopes residues 49-57 and residues 30-67 were used, respectively. Golgistop (Pharmigen, San Diego, CA) was added 6 hours before harvesting the cells from the culture. Cells were then washed once in FACSCAN buffer and stained with phycoerythrin (PE)-conjugated monoclonal rat anti-mouse CD8 or CD4 antibody (Pharmingen, San Diego, CA). Cells were subjected to intracellular cytokine staining using the Cytofix/Cytoperm kit according to the manufacturer's instructions (Pharmingen). FITC-conjugated anti-IFN-γ or anti-IL-4 antibodies and the immunoglobulin isotype control antibody (rat IgG1) were all purchased from Pharmingen. Analysis was done on a Becton-Dickenson FACScan with CELLQuest software (Becton Dickson Immunocytometry System, Mountain View, CA).

*Mice and Murine Tumor Cells:* Mice and the production and maintenance of tumor cells such as TC-1 cells is described above in Example 1.

*In Vivo Tumor Protection:* For the tumor protection experiment, mice (5 per group) were vaccinated via gene gun with 2 µg of plasmid DNA with GM, ETA(dII), E7, GM-ETA(dII), ETA(DII)-E7 , GM-E7 or GM-ETA(DII)-E7 gene insert and the □empty□ plasmid vector. One week later, the mice were boosted with the same regimen as the first vaccination. Another set of mice (5 per group) were vaccinated once (without further booster). One week after the last vaccination, mice were subcutaneously challenged with 5x10⁴ cells/mouse TC-1 tumor cells in the right leg and then monitored twice a week.

*In Vivo Antibody Depletion:* Methods for *in vivo* antibody depletions are described above. Briefly, C57BL/6 mice were vaccinated with 2 µg GM-ETADII-E7 DNA via gene gun, boosted one week later, and challenged with 5 x 10⁴ cells/mouse TC-1 tumor cells. Depletions were started one week prior to tumor challenge. MAb GK1.5 was used for CD4 depletion, MAb 2.43 was used for CD8 depletion, and MAb PK136 was used for NK1.1 depletion. Flow cytometry analysis revealed that the greater than 95% of the appropriate lymphocytes subset were depleted with normal level of other subsets. Depletion was terminated on day 40 after tumor challenge.

*Generation and Characterization of GM-ETA(dII)-E7 Fusion DNA Vaccine:* A schematic diagram showing constructs of GM-ETA(dII)-E7, GM-ETA(dII), ETA(dII)-E7, GM-E7, GM-CSF, ETA(dII), and E7 is demonstrated in Fig. 8.

*Vaccination with GM-ETA(dII)-E7 Fusion DNA Enhances E7-Specific CD8⁺ T Cell-Mediated Immune Responses:* CD8⁺ T lymphocytes are one of the most crucial components among antitumor effectors. To determine the E7-specific CD8⁺ T cell precursor frequencies generated by GM-ETA(DII)-E7 DNA vaccines, intracellular cytokine stains were used. The quantity of E7(49-57)-specific CD8+ T cell precursors was determined by flow cytometry analysis using double staining for CD8 and intracellular IFN-γ. Values from this assay correlated closely with the ELISPOT results presented. As shown in Fig. 9, mice vaccinated with GM-ETA(dIl)-E7 DNA generated the highest number of E7-specific IFN-γ⁺ CD8⁺ T cell precursors by flow cytometry analysis, whereas mice vaccinated with E7, GM-CSF, ETA (dII), GM-ETA(dII), GM-E7, or ETA-E7 DNA showed no signal above the vector-vaccinated controls.

*Vaccination with GM-ETA(dII)-E7 Fusion DNA also Enhances E7-Specific CD4⁺ T Cell-Mediated Immune Responses:* To determine E7-specific CD4⁺ T precursor cells and the cytokine profiles generated by GM-ETA(dII)-E7 DNA vaccines, double staining was performed for CD4 surface marker and intracellular IFN-γ on splenocytes from immunized mice, followed by flow cytometry analysis. The splenocytes from immunized mice were cultured in vitro with E7 peptide (residues 30-67 of SEQ ID NO:7) overnight and stained for both CD4 and intracellular IFN-γ. The E7 peptide (residues 30-67 of SEQ ID NO:7) contains a major T helper epitope in the E7 open reading frame protein of HPV-16. Methods for determining T helper cell epitopes within the polypeptide sequence of a protein or peptide are well known in the art.

The percentage of IFN-γ secreting CD4⁺ T cells was analyzed using flow cytometry. As shown in Fig. 10, mice vaccinated with GM-ETA(dII)-E7 DNA generated the greater number of CD4⁺ IFN-γ ⁺ double positive cells compared to mice vaccinated with E7 DNA, GM-CSF, ETA (dII), GM-ETA(dII), ETA-E7 DNA or vector alone. Mice vaccinated with GM-ETA(dII)-E7 DNA generated similar level of E7-specific CD4⁺ IFN-γ ⁺ double positive cells. These results indicate GM-CSF plays an important role in enhancing IFN-γ secreting E7-specific CD4⁺ T cells in vaccinated mice. These results also demonstrate that DNA vaccines containing GM-CSF-coding sequence operably linked to DNA encoding a target antigen are more potent than those which contain antigen-encoding sequences alone.

*Vaccination with GM-ETA(dII)-E7 Fusion DNA Enhances Protection of Mice Against the Growth of TC-I Tumors:* To determine whether vaccination with the GM-ETA(DII)-E7 DNA construct protects mice against E7-expressing tumors, *in vivo* tumor protection experiments were performed using various DNA vaccines. Mice were vaccinated with 2 µg naked DNA/mouse via gene gun and boosted with the same dose one week later. The mice were then challenged with 5×10⁴ TC-1/mouse subcutaneously in the right leg 7 days after the last vaccination. 100% of those mice receiving GM-ETA(DII)-E7 DNA vaccination remained tumor-free 30 days after TC-1 challenge, while all of the unvaccinated mice and mice receiving E7, GM-CSF, ETA (dII), GM-ETA(dII), GM-E7, ETA-E7 DNA or empty plasmid developed tumor growth within 15 days after tumor challenge (Fig. 11). To determine whether the antitumor effect generated by GM-ETA(DII)-E7 DNA is long-lasting, these tumor free animals were re-challenged with the 5 x 10⁴ TC-1 cells/mouse subcutaneously in the left leg. No tumor growth was observed up to 30 days after tumor rechallenge. These results indicated that GM-ETA(DII)-E7 fusion DNA significantly enhances the antitumor immunity against the growth of TC-1 tumors and that the antitumor immunity generated by GM-ETA(DII)-E7 DNA is long lasting.

*CD8⁺ T Cells Are Essential for the Antitumor Effect Generated by GM-ETA (dII)-E7 DNA Vaccine:* To determine the subset of lymphocytes that are important for the rejection of E7-positive tumor cells*, in vivo* antibody depletion experiments were carried out. The antibody depletion was started one week before the tumor challenge and terminated on the day indicated in Fig. 12 after tumor challenge. As shown in Fig. 12, all naive mice and all of the mice depleted of CD8⁺ T cells grew tumors within 14 days after tumor challenge. In contrast, all of the non-depleted mice and all of the mice depleted of CD4+ T cells or NK1.1 cells remained tumor-free days after tumor challenge. These results indicate that CD8⁺ T cells are essential for the antitumor immunity generated by GM-ETAdII-E7 DNA vaccine.

The data described herein demonstrate that a chimeric nucleic acid of the invention, a DNA construct, encoding a chimeric polypeptide with an APC-binding domain, a cytoplasmic translocation domain, and an antigenic epitope, induces a potent immune response specific for the encoded antigenic epitope. In a mouse tumor, the results showed that fusion of the GM-CSF (GM) gene, the ETA(dII) gene, and the HPV-16 E7 gene in a chimera generated enhanced E7-specific CD8+ and CD4+ T cell-mediated immune responses and antitumor effects against HPV-16 E7-expressing murine tumors.

Immunization of mice using the GM-ETA(dII)-E7 chimera was capable of mediating E7-specific immune responses and effective anti-tumor protection and treatment against an E7-expressing murine tumor cell line (TC-1) through intramuscular or intradermal injection. This dramatic anti-tumor effect was not observed in DNA vaccines encoding other constructs, particularly GM-E7, ETA(dII)-E7, or GM-ETA(dII). Furthermore, greater CD8+ and CD4+ T cell responses were observed in mice receiving GM-ETA(dII)-E7 than in mice receiving other constructs. These results indicated that the GM-ETA(dII)-E7 chimeric DNA represents a novel strategy for enhancing the cell mediated response for a selected antigen, e.g., a tumor antigen in cancer immunotherapy, or, an antigen against a pathogen for treating or preventing pathology associated with that pathogen. The results also demonstrate that the components of the vaccine are important in the generation of potent E7-specific immune responses and anti-tumor effects.

The results indicated that the GM-CSF gene was an important component in GM-ETA(dII)-E7 fusion protein, demonstrated by the comparison of GM-ETA(dII)-E7 and ETA(dII)-E7 DNA constructs. The ETA(dII)-E7 construct lacked the GM-CSF component of GM-ETA(dII)-E7, which may account for the lack of enhanced E7-specific CD8+ or CD4+ T cell-mediated immune responses and anti-tumor effects.

GM-CSF induces production of granulocytes and APCs and acts as an immunostimulatory signal to dendritic cells (DCs). Furthermore, GM-CSF targets professional APCs and promotes their differentiation, since APCs possess the GM-CSF receptor. While the invention is not limited by any particular mechanism, the GM-ETA(dII)-E7 fusion protein may be inducing changes in MHC and co-stimulatory molecule expression on DCs and other APCs due to the presence of the GM-CSF domain, resulting in enhanced humoral and cell-mediated immune responses that were not observed in ETA(dII)-E7. These observations indicated that the immune-enhancing effect of the GM-CSF domain on dendritic cells is a crucial component to the generation of immune responses and an anti-tumor effect by the GM-ETA(dII)-E7 fusion protein.

While the method is not limited by any particular mechanism, the antigen-specific immunotherapy approach may be targeting the surface molecules of DCs, which allows antigen to be directed into DCs for processing and presentation. Other polypeptides that can be used as the APC-binding component of the immunogenic composition include Flt-3 ligand (FL), CD40 ligand, TNFα, and 41BB. DNAs encoding such polypeptides are useful as the first DNA component of the immunogenic composition or DNA vaccine of the invention. Flt3 ligand (Flt3-1 or FL) may by augmenting the function and quantity of dendritic cells *in vivo*. Flt3 (fms-like tyrosine kinase 3) is a murine tyrosine kinase receptor known in the art, and Flt3 has been used to identified and subsequently clone the corresponding ligand, Flt3-L (Hannum et al., 1994, Nature. 368: 643-648; Maraskovsky et al., J. of Experimental Med., (1996) 184:1953-1962; 199638). CD40 ligand (CD154) is also useful; it may be enhancing vaccine potency because of its effect on DCs. TNF-α represents another strategy for enhancing vaccine potency; TNF-α may be promoting the migration of DCs to lymphoid tissues and differentiation and development of Langerhans cells. Another strategy for enhancing vaccine potency is the use of 4-1BBL, a cytokine of the tumor necrosis factor family that binds to 4-1BB (CD137). 4-1BBL expression on DCs followed by interaction with 4-1BB provides secondary costimulatory signals to T cells for initiating proliferation, independent of signaling through the CD28 receptor. These strategies for enhancing vaccine potency employ DC surface molecules in a manner similar to GM-CSF in GM-ETA(dII)-E7, allowing for targeting of antigen to DCs and consequent induction of DC differentiation and enhancement of antigen processing and presentation.

*Diptheria, Clostridium, Botulinum* (tetanus), *Bacillus, Yersinia, Vibrio cholerae* (cholera) or *Bordetella pertussis* toxins represent bacteria-derived molecules that may have therapeutic applications. Such molecules may function in a manner similar to that of the ETA domain in the fusion polypeptides encoded by the DNA vaccines described. These domains may cause enhancement of MHC class I presentation through translocation of antigen from the endosomal/Iysosomal compartment to the cytosol (particularly if the toxic domain of the gene encoding the toxin is mutated or deleted).

Determining whether a give polypeptide or protein fragment has cytoplasmic translocation activity, and thus is within the scope of the invention as a domain within the fusion proteins of the invention, is determined as follows. Anthrax toxin lethal factor (LF) has been shown to translocate foreign proteins into the cytosol of eukaryotic cells, resembling the mechanism of *Diphtheria* toxin. *Yersiniae* cytotoxins YopE and YopH are useful as translocation domains and for internalization of antigens across the host cell membrane into the cytosol of macrophages. *Bordetella pertussis* adenylate toxin (CyaA) translocates directly through the cell membrane to the cytosol, thus enhancing MHC class I antigen handling. Thus, the strategies for enhancing vaccine potency employed by the fusion proteins and vaccines of the invention (e.g., the ETA(DU) domain in the GM-ETA(dII)-E7 fusion protein) may be similar to the biological mechanism of the translocation domains of these bacterial toxins. These domains may be allowing antigen to escape from the endosomal/lysosomal compartments to the cytosol, resulting in enhancement of MHC class I presentation.

### Example 3: Enhancement of DNA Vaccine Potency by Linkage of DNA Encoding the Translocation Domain of Pseudomonas Exotoxin A to an Antigen Gene

The following example describes studies that demonstrate that the compositions and methods described are effective for enhancing antigen-specific cytotoxic T lymphocyte (CTL) responses and treating tumors *in vivo.*

While the method is not limited by any particular mechanism, a DNA vaccine encoding a fusion protein comprising an ETA domain and the HPV-16 E7 antigen may lead to the distribution of E7 to a greater number of antigen presenting cells (APCs) than a vaccine without ETA-encoding sequences, particularly for nucleic acid administered intradermally via gene gun. The polypeptide domain ETA(dII) may be releasing protein for uptake by other cells.

Immunological assays revealed that vaccination with E7 fused to ETA(dII) led to a 30-fold increase in E7-specific CD8+ T cell precursor frequencies with no significant E7-specific CD4 or antibody enhancement. *In vitro* studies indicated that cells transfected with ETA(dD)-E7 DNA presented E7 antigen through the MHC class I pathway in a more efficient manner than cells transfected with wild-type E7 DNA. Furthermore, bone marrow-derived dendritic cells pulsed with ETA fusion protein presented E7 antigen through the MHC class I pathway in a more efficient manner than dendritic cells pulsed with wild-type E7 protein. The surprising enhancement of CD8+ T cell-mediated immunity led to potent subcutaneous tumor protection and treatment of lung metastases. These data suggest that the compositions and methods described may be enhancing antigen-specific DNA vaccine potency by increasing the spread of expressed antigen. For example, administering DNA vaccines encoding ETA-antigen fusion proteins or chimeras may be increasing the intracellular spread the antigen expressed in the fusion protein (relative to similar DNA vaccines encoding polypeptides comprising antigen but lacking the ETA domain).

### Example 4: Enhancement of DNA Vaccine Potency by Linkage of Antigen-Encoding Sequences to a FL (Flt-3 Ligand) Sequences

The following example describes studies that demonstrate that the compositions and methods described are effective for enhancing antigen-specific cytotoxic T lymphocyte (CTL) responses and treating tumors *in vivo.*

Flt3-ligand (FL) is an important cytokine in the generation of professional antigen presenting cells, particularly dendritic cells. While the method is not limited by any particular mechanism, a recombinant molecule in which an Flt3 ligand domain is linked to an antigen to which immunity is desired (a fusion protein comprising FL and an antigen) may be targeting the antigen to APCs, such as dendritic cells.

Using HPV-16 E7 as an antigen, the effect of Flt3-ligand (FL)-antigen constructs (Fig. 19) on the potency of antigen-specific immunity generated by!naked DNA vaccines was evaluated. Residues 1-189 of SEQ ID NO:25 are FL-derived, and residues 190-287 are E7-derived ("Z" in position 288 of SEQ ID NO:25 indicates a stop). DNA constructs encoding FL-E7 fusion polypeptides were administered intradermally via gene gun. Vaccines containing chimeric FL/E7 fusion genes were found to dramatically increase the frequency of E7-specific CD8+ T cells relative to vaccines containing the wild-type E7 gene in the absence of FL-encoding sequences.

*In vitro* studies indicated that cells transfected with FL/E7 DNA presented E7 antigen through the MHC class I pathway in a more efficient manner than cells transfected with wild-type E7 DNA. Furthermore, bone marrow-derived dendritic cells pulsed with FL/E7 fusion protein presented E7 antigen through the MHC class I pathway in a more efficient manner than dendritic cells pulsed with wild-type E7 protein. More importantly, this fusion converted a less effective vaccine into one with significant potency against established E7-expressing metastatic tumors. Interestingly, FL/E7 fusion vaccines mainly targeted CD8+ T cells and antitumor effects were completely CD4-independent. These data indicated that fusion of Flt3-ligand DNA to an antigen-encoding enhances the potency of DNA vaccines via CD8-dependent pathways.

### Example 5: Domains of HSP70 that Confer Enhancement of Antigen-Specific Immune Reponses

The following example describes studies that demonstrate that the chimeric nucleic acid of the invention encoding a chimeric polypeptide comprising a first polypeptide domain comprising a carboxy terminal fragment of a heat shock protein (HSP) is effective for enhancing antigen-specific cytotoxic T lymphocyte (CTL) responses and treating tumors *in vivo.*

The domain structure of *Mycobacterium tuberculosis* HSP70 was determined. At least three functional domains were identified: an ATPase domain (containing residues 1-356 of SEQ ID NO:9), substrate binding domain (SD; containing residues 357-516 of SEQ ID NO:9) and a carboxyterminal domain (CD; containing residues 517-625 of SEQ ID NO:9) (Fig. 16). The contribution of each domain of this HSP70 on the potency of naked DNA vaccines was determined. DNA encoding each domain was linked in frame, i.e., operably linked, to DNA encoding the papillomavirus antigen E7. E7-specific CD8 T cell immune responses were measured.

A standard gene gun DNA delivery method was used to vaccinate C57BL/6 mice intradermally with DNA vaccines containing (i) HSP70 gene; (ii) wild-type HPV-16 E7 gene; (iii) HPV-16 E7 gene fused to the ATPase domain of HSP70 (E7-ATP); (iv), HPV-16 E7 gene fused to the substrate binding domain (SD) of HSP70 (E7-SD); or (v) IIPV-16 E7 gene fused to the cytoplasmic domain (CD) of HSP70 (E7-CD). All of the chimeric E7/heat shock protein constructs were cloned into the multiple cloning sites of the pcDNA3.1 (-) expression vector downstream of the cytomegalovirus promoter (Fig. 17). The *in vivo* antitumor immune responses were analyzed by intracellular cytokine staining using E7-specific MHC class I peptide (RAHYNIVTF; amino acids 49-57 of SEQ ID NO:7) to assess the E7-specific CD8+T cell-mediated immunity.

Figure 18A is a representative FASCAN showing E7-specific CD8⁺ T cell precursor frequencies. Splenocytes from vaccinated mice were cultured *in vitro* with the E7 peptide (amino acids 49-57 of SEQ ID NO:7) overnight and were stained for both CD8 expression and intracellular IFN-γ. The number of IFN-γ secreting CD8+ T cell precursors in mice immunized with various DNA constructs (E7-HSP70, E7-ATP, E7-SD, E7-CD, or E7 alone) was analyzed using flow cytometry. Mice vaccinated with E7-HSP70 DNA generated the highest number of E7-specific CD8+ precursors. E7-CD generated the second highest quantity of E7-specific CD8+ precursors, comparable to E7-HSP70 alone (and greater than E7-SD, E7-ATP, and wild-type E7).

Mean number of IFN-γ-producing E7-specific CD8+ T cells is shown in Figure 18B. The number of cells was determined by flow cytometry in the presence (solid bars) and absence (open bars) of the E7 antigen peptide (residues 49-57 of SEQ ID NO:7). The data was expressed as mean number of CD8+ IFN-γ+ cells/3x10⁵ splenocytes ± SEM. The results indicate that when incorporated into a DNA vaccine construct, DNA encoding a cytoplasmic domain of HSP70 increases the potency of the DNA vaccine in generating immunity to the target antigen encoded by the vaccine. Moreover, the cytoplasmic domain accounts for most of the enhancement of antigen specific MHC class I restricted CD8+ T cell immune responses in DNA vaccines that contain full length HSP70.

For example, vaccination with E7 fused to full length HSP70 led to a 30-fold increase in E7-specific CD8+T cell precursor frequencies (approximately 365 per 3×10⁵ splenocytes) (Figs. 18A-B): Vaccination with E7 fused to the ATP domain of HSP70 generated slightly increased E7-specific CD8+T cell precursor frequencies (approximately 58 per 3x10⁵) splenocytes). Similarly, vaccination with E7 fused to the SD domain of HSP70 generated approximately 85 E7-specific CD8+T cell precursors per 3x10⁵ splenocytes. In contrast, vaccination with E7 fused to the CD domain of HSP70 generated approximately 296 E7-specific CD8+T cell precursors per 3x10⁵ splenocytes, an amount similar to that obtained from mice vaccinated with E7 fused to full length HSP70. These results indicated the CD domain of HSP70 confers most of the enhanced antigen-specific MHC class I restricted CD8+T cell immune responses in DNA vaccines.

These data demonstrate that DNA encoding a fusion of full-length HSP70 or the CD domain of HSP70 (in the absence of DNA encoding other portions of HSP70) to an antigenic peptide (e.g., HPV-16 E7) greatly enhances antigen-specific CD8+T cell immune responses to the antigen. The enhancement of immune responses was observed in constructs which lacked more N-terminal sequences in the region of the ATPase domain (e.g., residues 161-370 of SEQ ID NO:9) which were thought to be important in stimulating a CTL response. The data indicate that DNA vaccines containing constructs encoding the CD domain of HSP, even in the absence of other HSP sequences, enhanced antigen-specific MHC class I restricted CD8+T cell immune responses to a target antigen encoded by the DNA vaccine.

### Example 6: Administration of self replicating RNA viruses expressing the fusion proteins of the invention enhance generation of an antigen-specific cytotoxic T lymphocyte (CTL) responses

In one embodiment, the invention provides a self-replicating RNA replicon that can express a chimeric protein of the invention: a protein that comprises a first polypeptide domain comprising a heat shock polypeptide and a second polypeptide domain comprising at least one antigenic peptide. The following example describes studies that demonstrate that, using the methods of the invention, these constructs are effective for enhancing antigen-specific cytotoxic T lymphocyte (CTL) responses *in vivo.* As a model system, a fusion protein comprising HPV-16 E7 and *Mycobacterium tuberculosis* HSP70 was expressed *in vivo* in a Sindbis virus self-replicating RNA vector, SINrep5. The potency of antigen-specific immunity generated by this vector was determined. These results also demonstrate that fusion proteins comprising a heat shock polypeptide and an antigenic peptide expressed *in vivo* in a Sindbis virus self-replicating RNA vector are effective for enhancing antigen-specific CTL responses *in vivo.*

These experiments demonstrated that an RNA replicon vaccine containing E7/HSP70 fusion genes generated significantly higher E7-specific T cell-mediated immune responses than vaccines containing the wild type E7 gene in vaccinated mice. Furthermore, *in vitro* studies demonstrated that E7 antigen from E7/HSP70 RNA replicon-transfected apoptotic cells can be taken up by bone marrow-derived dendritic cells and presented more efficiently through the MHC class I pathway than wild-type E7 RNA replicon-transfected apoptotic cells. More importantly, the fusion of HSP70 to E7 converted a less effective vaccine into one with significant potency against E7-expressing tumors. This antitumor effect was dependent on NK cells and CD8⁺ T cells. These results indicated that fusion of HSP70 to an antigen gene greatly enhanced the potency of self-replicating RNA vaccines. These results demonstrated that a Sindbis RNA vaccine linking E7 with HSP70 dramatically increased expansion and activation of E7-specific CD8+ T cells and NK cells, completely bypassing the CD4 arm and resulting in potent anti-tumor immunity against E7-expressing tumors.

The mechanism of Sindbis RNA vaccine to promote the anti-tumor effect was further investigated. It was found that the Sindbis E7/HSP70 RNA vaccine could induce apoptotic death of host cells and promote dendritic cells to phagocytose these cells, dramatically increasing the expansion and activation of E7-specific CD8+ T cells. This enhanced CD8 response resulted in potent anti-tumor immunity against an E7-expressing tumor cell line.

HPV-16 E7 was chosen as a model antigen for vaccine development because HPVs, particularly HPV-16, are associated with most cervical cancers, as discussed above.

*Plasmid DNA Constructs and Preparation:* The vectors pcDNA3-HSP70, pcDNA3-E7, and pcDNA3-E7/HSP70 were made as described by Chen (2000) supra. The Sindbis virus RNA replicon vector, SINrep5 has been described by, e.g., Bredenbeek (1993) J. Virol. 67:6439-6446. Vectors SINrep5-HSP70, SINrepS-E7, and SINrep5-E7/HSP70 were made by isolating DNA fragments encoding *Mycobacterium tuberculosis* HSP70, HPV-16 E7 and chimeric E7/HSP70 by cutting pcDNA3-HSP70, pcDNA3-E7, and pcDNA3-E7/HSP70, respectively, with Xba I and Pme I restriction enzymes. Digested products were isolated using gels. These isolated DNA fragments were further cloned into the corresponding XabI and Pml I sites of the SINrep5 vector to generate SINrep5-HSP70, SINrep5-E7, and SINrep5-E7/HSP70 constructs. The accuracy of these constructs was confirmed by DNA sequencing.

*In Vitro RNA Preparation:* The generation of RNA transcripts from SINrep5-HSP70, SINrep5-E7, SINrep5-E7/HSP70 and SINrep5 was performed using the protocol described by Mandl (1998) Nature Med 4:1438-1440. SpeI was used to linearize DNA templates for the synthesis of RNA replicons from SINrep5-HSP70, SINrep5-E7, SINrep5-E7/HSP70 and SINrep5. RNA vaccines were transcribed *in vitro* and capped using SP6 RNA polymerase and capping analog from a standard *in vitro* transcription kit (Life Technologies, Rockville, MD) according to vendor's manual. After synthesis, DNA was removed by digestion with DNase I. Synthesized RNA was quantified and analyzed using denaturing formaldehyde agarose gels (see, e.g., Mandl (1998) supra). The purified RNA was divided into aliquots to be used for vaccination in animals and for transfection of a BHK21 cell line. The protein expression of the transcripts was assessed by transfection of the RNA into BHK21 cells using electroporation.

*Cell Lines:* Baby hamster kidney (BHK21) cells were obtained from the ATCC (Rockville, MD) and grown in Glasgow MEM supplemented with 5% FBS, 10% tryptose phosphate broth, 2 mM glutamine, and antibiotics. Cells were kept at 37°C in a humidified 5% CO2 atmosphere and were passaged every 2 days. The production and maintenance of TC-1 cells was done as described by Lin (1996) Cancer Res. 56:21-26. On the day of tumor challenge, TC-1 cells were harvested by trypsinization, washed twice with 1X Hanks buffered salt solution (HBSS), and finally resuspended in 1X HBSS to the designated concentration for injection.

*ELISA for E7 Protein Expression of SINrep5 RNA vaccines:* The expression of E7 protein from SINrep5-E7 and SINrep5-E7/HSP70 RNA was determined by an indirect ELISA method. The quantity of E7 protein was determined using cell lysates from SIN5rep-E7 or -E7/HSP70 transfected BHK21 cells. Briefly, ten million BHK21 cells were transfected with the 4 µg SINrep5, SINrep5-E7, SINrep5-HSP70 or SINrep5-E7/HSP70 RNA transcripts respectively via electroporation as described by Liljestrom (1991) J. Virol. 65:4107-4113. The transfected BHK21 cells were collected 16-20 hrs after electroporation. A 96-microwell plate was coated BHK 21 cell lysates that were transfected with various SINrep5 RNAs in a final volume of 100 µl, and were incubated at 4 °C overnight. The bacteria-derived HPV-16 E7 proteins were used as a positive control. The wells were then blocked with PBS containing 20% fetal bovine serum. Diluted anti-E7 Ab (Zymed, San Francisco, CA) were added to the ELISA wells, and incubated on 37 °C for 2 hr. After washing with PBS containing 0.05% Tween-20, the plate was incubated with 1/2000 dilution of a peroxidase-conjugated rabbit anti-mouse IgG antibody (Zymed, San Francisco, CA) at room temperature (RT) for one hour. The plate was washed, developed with 1-Step^{™} Turbo TMB-ELISA (Pierce, Rockford, IL), and stopped with 1M H₂SO₄. The ELISA plate was read with a standard ELISA reader at 450 nm. The quantity of E7 protein of the cell lysates was then calculated and determined by comparing with the standardized E7 protein.

*Mice:* 6 to 8-week-old female C57BL/6 mice from the National Cancer Institute (Frederick, MD) were purchased and kept in the oncology animal facility of the Johns Hopkins Hospital (Baltimore, MD). All animal procedures were performed according to approved protocols and in accordance with recommendations for the proper use and care of laboratory animals.

*RNA Vaccination:* All SINrep5 RNA vaccines were generated using *in vitro* transcription as described above. RNA concentration was determined by optical density measured at 260 nm. The integrity and quantity of RNA transcripts were further checked using denaturing gel electrophoresis. Mice were vaccinated intramuscularly with 10 µg of various SINrep5 RNAs in the right hind leg except for SINrep5-E7/HSP70, which was administered in 0.1, 1, and 10 µg quantities.

*ELISA for E7 Antibodies:* Anti-HPV 16 E7 antibodies in the sera were determined by a direct ELISA as described by Wu (1995) Proc. Natl. Acad. Sci. USA 92:11671-1165. A 96-microwell plate was coated with 100 µl 5 µg/ml bacteria-derived HPV-16 E7 proteins and incubated at 4 °C overnight. The wells were then blocked with PBS containing 20% fetal bovine serum. Sera were prepared from mice on day 14 post-immunization, serially diluted in PBS, added to the ELISA wells, and incubated on 37 °C for 2 hr. After washing with PBS containing 0.05% Tween-20, the plate was incubated with 1/2000 dilution of a peroxidase-conjugated rabbit anti-mouse IgG antibody (Zymed, San Francisco, CA) at RT for one hour. The plate was washed, developed with 1-Step^{™} Turbo TMB-ELISA (Pierce, Rockford, IL), and stopped with 1M H₂SO₄. The ELISA plate was read with a standard ELISA reader at 450 nm.

*Enzyme-Linked Immunoabsorbent Assay (ELISA) for INF-γ.* Splenocytes were harvested 2 weeks after vaccination and cultured with the E7 peptide (aa 49-57) containing MHC class I epitope (see, e.g., Feltkamp (1993) Eur. J. Immunol. 23:2242-2249) or the E7 peptide (aa 30-67) containing MHC class II peptide (see, e.g., Tindle (1991) Proc. Natl. Acad. Sci. USA 88:5887-5891), in a total volume of 2 ml of RPMI 1640, supplemented with 10% (vol/vol) fetal bovine serum, 50 units/ml penicillin and streptomycin, 2 mM L-glutamine, 1 mM sodium pyruvate, 2 mM nonessential amino acids in a 24-well tissue culture plate for 6 days. The supernatants were harvested and assayed for the presence of IFN-γ using ELISA kits (Endogen, Wobum, MA) according to the manufacturer's protocol.

*Cytotoxic T Lymphocyte (CTL) Assays:* CTL assays were performed in 96-well round-bottom plates as described by Corr (1999) J. Immunol. 163:4721-4727. Cytolysis was determined by quantitative measurements of lactate dehydrogenase (LDH) (see, e.g., Corr (1999) supra). Splenocytes were harvested 2 weeks after RNA vaccination and cultured with the E7 peptide (aa 49-57) in a total volume of 2 ml of RPMI 1640, supplemented with 10% (vol/vol) fetal bovine serum, 50 units/ml penicillin/ streptomycin, 2mM L-glutamine, 1mM sodium pyruvate, 2mM nonessential amino acids in a 24-well tissue culture plate for 6 days as effector cells. TC-1 tumor cells were used as target cells. The TC-1 cells mixed with splenocytes at various effector/target (E/T) ratios. After 5 hr incubation at 37°C, 50µl of the cultured media were collected to assess the amount of LDH in the cultured media according to the manufacturer's protocol of the CytoTox^{™} assay kits (Promega, Madison, WI). The percentage of lysis was calculated from the following equation: 100 X (A-B)/(C-D), where A is the reading of experimental-effector signal value, B is the effector spontaneous background signal value, C is maximum signal value from target cells, D is the target spontaneous background signal value.

*Intracytoplasmic Cytokine Staining and Flow Cytometry Analysis:* Splenocytes from naïve or vaccinated groups of mice were incubated with the E7 peptide (aa 30-67) that contains MHC class II peptide (Tindle (1999) supra) for detecting E7-specific CD4⁺ T helper cell precursors. The E7 peptide was added at a concentration of 10 µg/ml for 20 hours. Golgistop^{™} (PharMingen, San Diego, CA) was added 6 hours before harvesting the cells from the culture. Cells were then washed once in FACScan^{™} buffer and stained with phycoerythrin (PE)-conjugated monoclonal rat anti-mouse CD4 antibody (PharMingen, San Diego, CA). Cells were subjected to intracellular cytokine staining using the Cytofix/Cytoperm^{™} kit according to the manufacturer's instructions (PharMingen). FITC-conjugated anti-IFN-γ antibody and the immunoglobulin isotype control antibody (rat IgG1) were all purchased from PharMingen. Analysis was done on a Becton Dickinson FACScan^{™} with CELLQuest^{™} software (Becton Dickinson Immunocytometry System, Mountain View, CA).

*In Vivo Tumor Protection Experiments:* For the tumor protection experiment, mice (5 per group) were immunized intramuscularly (IM) with different doses of SINrep5-HSP70, SINrep5-E7, SINrep5-E7/HSP70, and empty SINrep5 RNA vaccines. 14 days after immunization, mice were injected intravenously (IV) with 1x10⁴ cells/mouse TC-1 tumor cells in the tail vein. Three weeks later, mice were euthanized. The lung weight and number of pulmonary nodules in each mouse was evaluated and counted by experimenters in a blinded fashion.

*In Vivo Antibody Depletion Experiments:* The procedure for *in vivo* antibody depletion has been described previously by, e.g., Lin (1996) supra; Wu (1995) J. Exp. Med. 182:1415-1421. In brief, mice were vaccinated with 1 µg self-replicating SINrep5-E7/HSP70 RNA intramuscularly and challenged with 1 x 10⁴ cells/mouse TC-1 tumor cells via tail vein injection. Depletions were started one week prior to tumor challenge. MAb GK1.5 (Dialynas (1983) J. Immunol. 131:2445) was used for CD4 depletion, MAb 2.43 Sarmiento (1980) J. Immunol. 125:2665) was used for CD8 depletion, and MAb PK136 (Koo (1986) J. Immunol. 137:3742) was used for NK1.1 depletion. Flow cytometry analysis revealed that >95% of the appropriate lymphocytes subset were depleted with a normal level of other subsets. Depletion was terminated on day 21 after tumor challenge.

*Cell Surface Marker Staining and Flow Cytometry Analysis:* Splenocytes removed from naive or vaccinated groups of mice were immediately treated with cell surface marker staining as described by Ji (1999) Human Gene Therapy 10:2727-2740. Cells were then washed once in FACSCAN^{™} buffer and stained with PE-conjugated monoclonal rat anti-mouse NK1.1 antibody and FITC-conjugated monoclonal rat anti-mouse CD3 antibody (Pharmingen, San Diego, CA). The population ofNK cells was stained with anti-NK1.1 antibody and not stained with anti-CD3 antibody. The percentages ofNK cells in mice immunized with various self-replicating RNA vaccines was analyzed using flow cytometry.

*Generation and Culture of Dendritic Cells (DCs) from Bone Marrow:* DCs were generated by culture of bone marrow cells in the presence of GM-CSF as described by Lu (2000) J. Exp. Med. 191:541-550. Briefly, bone marrow was collected from the tibias of mice. Erythrocytes were lysed, and the remaining cells were passed through a nylon mesh to remove small pieces of bone and debris. The cells were collected and 1x10⁶ cells/ml were placed in 24-well plates in RMPI 1640, supplemented with 5% FCS, 2mM β-mercaptoethanol, 1% non-essential amino acids, 100 U/ml penicillin and 100 µg/ml streptomycin (Life Technologies, Rockville, MD), and 100 U/ml GM-CSF (PharMingen, San Diego, CA). Two-thirds of the medium was replaced every 2 days, and non-adherent cells were harvested on day 7. The collected cells were characterized by flow cytometry analysis (FACS) for DC markers.

*Generation of E7-Specific CD8⁺ T Cell Lines:* Generation of E7-specfic CD8+ cell lines was done by immunizing female C57BL/6 (H-2b) mice by intraperitoneal (IP) injection of Sig/E7/LAMP-1 vaccinia. Splenocytes were harvested on day 8. For initial *in vitro* stimulation, splenocytes were pulsed with IL-2 at a concentration of 20 U/ml and 1 µM E7 peptide (amino acids 49-57) for 6 days. Propagation of the E7-specific CTL cell line was performed in 24-well plates by mixing (2 ml/well) 1x10⁶ splenocytes containing E7-specific CTLs with 3x10⁶ irradiated splenocytes and pulsing them with IL-2 at a concentration of 20 U/ml and 1 µM E7 peptide (amino acids 49-57). This procedure was repeated every 6 days. The specificity of the E7 CTL line was characterized by the CTL assay. Flow cytometry was performed to demonstrate the expression of the CD8 marker.

*In Vitro Cell Death Analysis:* Ten million BHK21 cells were transfected with 4 µg SINrep5, SINrep5-E7, SINrep5-HSP70 or SINrep5-E7/HSP70 RNA transcripts as mentioned earlier. Native BHK21 cells or BHK21 cells that were electroporated without SINrep5 RNA were used as controls. BHK21 cells were collected and assessed every 24 hr, until hour 72. The percentages of apoptotic and necrotic BHK21 cells were analyzed using annexin V apoptosis detection kits (PharMingen, San Diego, CA) according to the manufacturer's protocol, followed by flow cytometry analysis.

*CTL Assay Using DCs Pulsed with Apoptotis Cells as Target Cells:* CTL assays using DCs pulsed with apoptosis cells as target cells were performed using a protocol similar to that described by Albert (1998) Nature 392:86-89; Albert (1998) J. Exp. Med. 188:1359-1368; with modification. Briefly, 10 million BHK21 cells were transfected with 4 µg of various self-replicating SINrep5 RNAs via electroporation. BHK21 cells were collected 16-20 hr after electroporation. The levels of E7 protein expression in BHK21 cells transfected with SINrep5-E7, or SINrep5-E7/HSP70 RNA transcripts were similar, as determined by ELISA. 3x10⁵ transfected BHK21 cells were then co-incubated with 1x10⁵ of bone marrow-derived DCs at 37 °C for 48 hr. These prepared DCs were then used as target cells and the Db-restricted E7-specific CD8⁺ T cells were used as the effector cells. CTL assays were performed with effector cells and targets cells (1x10⁴ per well) mixed together at various ratios (1:1, 3:1, 9:1, and 27:1) in a final volume of 200 µl. After 5 h incubation at 37°C, 50 µl of the cultured media were collected to assess the amount of LDH in the cultured media as described above. DCs co-incubated with untransfected BHK21 cells, transfected BHK21 cells alone, untreated DCs alone, and CD8⁺ T cell line alone were included as negative controls.

*Construction and Characterization of Self-replicating RNA Constructs:* Generation of plasmid DNA constructs and subsequent preparation of self-replicating SINrep5 RNA constructs was performed as described above. The SINrep5 vector contains the genes encoding Sindbis virus RNA replicase and the SP6 promoter (see, e.g., Bredenbeek (1993) supra). The schematic diagram of SINrep5, SINrep5-HSP70, SINrep5-E7, SINrep5-E7/HSP70 DNA constructs was shown in Figure 20A. In addition, the schematic diagram of RNA transcript derived from these DNA constructs using SP6 RNA polymerase was shown in Figure 20B. A methylated M⁷G "cap" is located at the 5' end of the mRNA, followed by a sequence responsible for the self-replication (replicase), the gene of interest (i.e., an MHC class I peptide epitope, an E7, an HSP70, an E7/HSP70, or the like), and a polyadenylated tail (AAAA). An ELISA was performed to demonstrate the expression of E7 protein by BHK21 cells transfected with the various self-replicating RNA constructs. SINrepS-E7 and SINrep5-E7/HSP70 expressed similar amounts of E7 protein.

*Vaccination with Self-replicating SINrep5-E7*/*HSP70 RNA Enhances an E7-Specific Cytotoxic Immune Response:* CD8⁺ T lymphocytes are one of the most crucial effectors for inducing anti-tumor immunity. To determine the quantity of E7-specific CD8⁺ T cell responses generated by the SINrep5-E7/HSP70 RNA vaccine, CTL assays were used. Mice were immunized with various SINrep5 self-replicating RNA vaccines via intramuscular injection. Splenocytes and serum samples were collected after 14 days. To perform the cytotoxicity assay, splenocytes from the various self-replicating SINrep5 RNA vaccines were cultured with E7 peptide (aa 49-57) containing MHC class I epitope for 6 days as effector cells. TC-1 tumor cells were as target cells. The TC-1 cells mixed with splenocytes at various E/T (effector/target ratio). Cytolysis was determined by quantitative measurements of LDH. CTL assays shown here are from one representative experiment of two performed.

The self-replicating RNA E7/HSP70 vaccine generated significantly higher percentage of specific lysis as compared with the other RNA vaccines (*: P<0.001, one-way ANOVA). The self-replicating SINrepS-E7/HSP70 generated a significantly higher percentage of specific lysis compared to mice vaccinated with the other SINrep5 RNA vaccines (P<0.001, one-way ANOVA). The ability of SINrep5-E7/HSP70 RNA to generate specific lysis was found to be approximately 4 times that of self-replicating SINrep5-E7 RNA (32.7% versus 8.8%, E/T ratio 45/1, P<0.001).

*Vaccination with Self-replicating SINrep5-E7*/*HSP70 RNA Enhances E7-specific CD8⁺ T cells to Secrete High Levels of INFγ:* To determine the extent of the immunological response of E7-specific CD8⁺ T cells generated by self-replicating SINrep5-E7/HSP70 RNA, an ELISA was used to detect the concentration of INF-γ in the supernatant of cultured splenocytes. Mice were immunized with various self-replicating RNA vaccines via intramuscular injection. Splenocytes and serum samples were collected after 14 days. Splenocytes from the various self-replicating RNA vaccines were cultured *in vitro* with E7 peptide (aa 49-57) containing the MHC class I epitope (or without any peptide) for 6 days. As a negative control, an ELISA was also performed without peptide. Supernatants in the culture medium were collected to detect the INF-γ concentration using an ELISA.

Splenocytes from the self-replicating E7/HSP70 RNA group stimulated with E7 peptide (aa 49-57) secreted the highest concentration of INF- y compared to the other RNA vaccines (P<0.001, one-way ANOVA). These results also indicated that fusion of HSP70 to E7 significantly enhances INF-γ-secreting E7-specific CD8⁺ T cell activity. Thus, the CD8⁺ T cells could be induced by the MHC class I epitope of E7. Note: the splenocytes from the self-replicating E7/HSP70 RNA group stimulated with E7 peptide (aa 49-57) secreted the highest concentration of INF- y compared to the other RNA vaccines (*: P<0.001, one-way ANOVA).

*Vaccination with Self-replicating SINrep5-E7*/*HSP70 RNA Does Not Generate Significant E7-Specific CD4⁺ T Cell-Mediated Immune Responses:* To examine the generation of E7-specific CD4⁺T precursor cells and cytokine profiles by each of these RNA vaccines, we performed double staining for CD4 surface marker and intracellular IFN-γ on splenocytes obtained from immunized mice, followed by flow cytometry analysis. The splenocytes were cultured *in vitro* with E7 peptide (aa 30-67) overnight and stained for both CD4 and intracellular IFN-γ. The E7 peptide (aa 30-67) contains a major T helper epitope in the E7 open reading frame protein of HPV-16 (see, e.g., Tindle (1991) supra). The percentage of IFN-γ-secreting CD4⁺ T cells was analyzed using flow cytometry.

Mice vaccinated with SINrep5-E7/HSP70 RNA generated a similar number of CD4⁺ IFN-γ⁺ double positive cells compared to mice vaccinated with SINrep5-E7 RNA (15/3×10⁵ splenocytes versus 12/3x10⁵ splenocytes, p>0.05) or other RNA groups. There was no significant difference in the number of E7-specific CD4⁺ IFN-γ⁺ cells observed using flow cytometry staining among naive mice or mice vaccinated with empty plasmid, E7, HSP70, or E7/HSP70 RNA. Splenocytes from Sig/E7/LAMP-1 DNA vaccinated mice (Ji (1999) supra) were used as positive controls for intracellular IFN-γ staining for this study.

The quantity of anti-HPV 16 E7 antibodies in the sera of the vaccinated mice was determined using a direct enzyme-linked immunoabsorbent assay (ELISA) 2 weeks after vaccination at various dilutions (1:100, 1:500, 1:1000). SINrepS-E7/HSP70 did not generate higher titers of E7-specific antibodies in the sera of vaccinated mice compared to that generated by other RNA vaccine constructs.

*Vaccination with Self-Replicating SINrepS-E7*/*HSP70 RNA Protects Mice Against the Growth of TC-1 Tumors:* To determine whether vaccination with the self-replicating SINrep5-E7/HSP70 RNA protected mice against E7-expressing tumors, an *in vivo* tumor protection experiment was performed using different doses of SINrep5-E7/HSP70 RNA administered intramuscularly in the right hind leg. Mice were similarly vaccinated with 10 µg self-replicating SINrep5, SINrep5-HSP70, and SINrep5-E7 RNA. Different doses of self-replicating SINrep5-E7/HSP70 RNA including 0.1 µg, 1 µg and 10 µg were also injected into mice. One week after vaccination, mice were challenged with TC-1 tumor cells via intravenous tail vein injection at a dose of 2x10⁴ cells/mouse. Mice were monitored twice a week and sacrificed at day 21 after tumor challenge. The pulmonary nodules were assessed 21 days after tumor challenge. Lungs were dissected from the mice 35 days after vaccination with empty SINrep5 (10 µg), SINrep5-HSP70 (10 µg), SINrep5-E7 (10 µg), and SINrepS-E7/HSP70 RNA (0.1 µg, 1 µg, or 10 µg). The mean number of lung foci was used as a measurement of the effectiveness of the various self-replicating RNA vaccines at controlling HPV-16 E7-expressing tumor growth.

The mean pulmonary nodules of mice vaccinated with the self-replicating E7/HSP70 RNA vaccines (0.1 µg, 1 µg, and 10 µg) were much less compared to mice vaccinated with the other RNA vaccines (P<0.001, one-way ANOVA). These results demonstrated that self-replicating RNA SINrep5-E7/HSP70 vaccines protect mice from intravenous tumor challenge even at the low dosage of 0.1 µg while mice vaccinated with RNA from 10 µg SINrep5 without insert, 10 µg SINrep5-E7, or 10 µg SINrep5-HSP70 developed numerous lung nodules from TC-1 tumor challenge.

*CD8⁺ T Cells and NK cells Are Important for the Anti-tumor Effect Generated by Vaccination with SINrep5-E7*/*HSP70 RNA Vaccines:* To determine the types of lymphocytes that are important for protection against E7-expressing tumor cells, *in vivo* antibody depletion (of CD8⁺ T cells and NK cells) experiments were performed (the percentage of NK cells from the splenocytes of mice immunized with self-replicating RNA vaccines were higher than that without immunization and there was no significant difference between the percentage ofNK cells among the various self-replicating RNA vaccines). The antibody depletion was started one week before tumor challenge and terminated on day 21 after tumor challenge.

The mean pulmonary nodules from mice depleted of CD8⁺ T cells and NK1.1 cells were significantly higher than those of non-depleted group. Furthermore, depletion of NK1.1 cells resulted in a higher mean number of tumor lung nodules than CD8+ depleted mice.

In comparison, the mean pulmonary nodules from mice depleted of CD4⁺ T cells resembled results obtained from non-depleted mice, indicating that CD4⁺ T cells were not critical in generating this effect. These results suggest that CD8⁺ T cells are essential for the antigen-specific anti-tumor immunity generated by SINrep5-E7/HSP70 RNA vaccine and that NK cells, while not limited to the E7/HSP70 RNA vaccine, play an important role as well.

It was also investigated whether the NK cell effect was limited to the E7/HSP70 vaccines or if it was the result of the vector used. Flow cytometry analysis of CD3(-), NK1.1(+) cells revealed that their presence was markedly increased in all constructs (E7/HSP70, E7, HSP70, and control plasmid) relative to naïve mice, indicating that NK cells were important effectors of the anti-tumor effect that are not limited to the E7/HSP70 vaccines.

*Self-Replicating RNA Vaccines Induce Apoptosis:* RNA transcribed *in vitro* from various plasmid SINrep5 RNA vaccines were transfected into BHK21 cells via electroporation. Electroporated BHK 21 cells without RNA and untreated BHK21 cells were used as controls. The percentages of apoptotic and necrotic BHK21 cells were stained by annexin V-FITC and propidium iodide (PI) followed by flow cytometry analysis.

The percentages of apoptotic BHK21 cells revealed statistical declines when transfected with SINrep5 RNA vaccines, 24 hr to 72 hr after (representative with SIN5-E7/HSP70 70.3±3.6% for 24 hr, 49.3±4.2% for 48 hr, 18.0±3.1% for 72 hr, P<0.001, one-way ANOVA). BHK21 cells transfected with SINrep5 RNA vaccines generated higher percentages after 24, 48 or 72 hours later compared to the other two control groups. No statistical differences could be found in the apoptotic percentages of various SINrep5 RNA vaccines.

*Enhanced Presentation of E7 through the MHC Class I Pathway in Dendritic Cells Pulsed With Cells Transfected with SINrepS-E7*/*HSP70 RNA:* A potential mechanism for the enhanced E7-specific CD8⁺ T cell immune responses *in vivo* is the presentation of E7 through the MHC class I pathway by uptake of apoptotic bodies from cells expressing various E7 constructs, also called "cross-priming". A cross priming experiment was performed to characterize the MHC class I presentation of E7 in dendritic cells pulsed with apoptotic bodies from BHK21 cells transfected with various self-replicating RNA. As mentioned previously, BHK21 cells have been shown to have stable high transfection efficiency and similar E7 expression among cells transfected with different E7-containing self-replicating RNA. Transfected BHK21 cells were co-incubated with bone marrow-derived DCs. DCs were used as target cells while E7-specific CD8⁺ T cells served as effector cells. CTL assays with various Err ratios were performed.

DC target cells co-incubated with BHK21 cells transfected with SINrepS-E7/HSP70 RNA generated significantly higher percentages of specific lysis compared to DCs co-incubated with BHK21 cells transfected with SINtep5-E7 RNA (P<0.001). These results suggested that dendritic cells pulsed with apoptotic bodies containing E7/HSP70 fusion protein presented E7 antigen through the MHC class I pathway more efficiently than dendritic cells pulsed with apoptotic bodies containing wild-type E7 protein. Thus, the fusion of HSP70 to E7 enhanced E7-specific CD8⁺ T cell immune responses; and, while the invention is not limited by any particular mechanism, the enhancement was likely via "cross priming."

## Claims

1. An immunogenic composition comprising a nucleic acid which encodes a chimeric polypeptide comprising:
(a) a first polypeptide domain, that is a carboxy terminal fragment or domain of a heat shock protein (HSP) which has chaperone activity;
(b) a second polypeptide domain comprising an antigenic polypeptide;
wherein the nucleic acid encoding the second polypeptide domain is 5' to the nucleic acid encoding the first polypeptide domain, and the nucleic acid can stimulate or enhance a cellular immune response to the antigenic polypeptide on administration to a mammal.

2. A composition according to claim 1, which can stimulate or enhance the immunogenicity of the antigenic peptide.

3. A composition according to claim 1 or 2, wherein the HSP comprises HSP 70.

4. A composition according to claim 3, wherein the HSP is derived from *a Mycobacterium tuberculosis.*

5. A composition according to any of the preceding claims, wherein the nucleic acid can induce a cytotoxic T cell response.

6. A composition according to any of the preceding claims, wherein the HSP carboxy terminal fragment or domain is selected from:
(a) a sequence as set forth by residues 312 to 625 of SEQ ID NO: 9; and
(b) a sequence as set forth by residues 517 to 625 of SEQ ID NO: 9.

7. A composition according to any of the preceding claims, wherein the antigenic polypeptide comprises an MHC Class I-binding peptide epitope.

8. A composition according to claim 7, wherein the MHC Class I-binding peptide epitope is between 8 amino acid residues and 11 amino acid residues in length.

9. A composition according to any of the preceding claims, wherein the antigenic polypeptide is derived from a virus, is a tumor-specific or tumor-associated polypeptide, or is derived from a pathogen other than a virus.

10. A composition according to claim 9, wherein the virus is a human papovavirus, a human papilloma virus (HPV) or a human immunodeficiency virus (HIV).

11. A composition according to claim 10, wherein the virus is a human papilloma virus-16 (HPV-16).

12. A composition according to claim 11, wherein the antigenic polypeptide comprises the HPV-16 E6 or E7 polypeptide or an epitopic fragment thereof.

13. A composition according to claim 9, wherein the tumor-specific or tumor-associated polypeptide comprises an antigen expressed on the surface of a tumor cell.

14. A composition according to claim 13, wherein the polypeptide comprises a mutant p53, a MAGE-1 or a MAGE-3.

15. A composition according to claim 9, wherein the antigenic polypeptide is derived from an HIV-1, a bovine viral diarrhoea virus, a cytomegalovirus, an encephalitis virus, a hepatitis virus, a herpes simplex virus, an influenza virus or a pathogen that causes malaria.

16. A composition according to claim 15, wherein the pathogen that causes malaria is malaria peptide (NANP) 40.

17. A composition according to any of the preceding claims, wherein the chimeric polypeptide further comprises a third polypeptide domain comprising a cytoplasmic translocation polypeptide domain.

18. A composition according to claim 17, wherein the third polypeptide domain is a cytoplasmic translocation domain of a *Pseudomonas* exotoxin A (ETA).

19. A composition according to claim 18, wherein the third polypeptide domain comprises a sequence as set forth by residue 253 to residue 364 of SEQ ID NO: 3.

20. A composition according to claim 17, wherein the third polypeptide domain comprises a cytoplasmic translocation domain of a toxin derived from *Diphtheria, Clostridium, Botulinum, Bacillus, Yersinia, Vibrio cholerae* or *Bordetella pertussis.*

21. A composition according to any of claims 17 to 20, wherein the nucleic acid encoding the third polypeptide domain is located 5' to the nucleic acid encoding the second domain, or is located between the nucleic acids encoding the first and the second domains, or is located 3' to the nucleic acid encoding the first domain.

22. A composition according to any of the preceding claims further comprising regulatory nucleic acid sequences.

23. A composition according to claim 22, wherein the regulatory sequences are transcriptional regulatory sequences.

24. A composition according to any one of the preceding claims wherein the nucleic acid is comprised in an expression cassette.

25. A composition according to claim 24, wherein the expression cassette comprises an expression vector, a plasmid or a self-replicating RNA replicon.

26. A composition according to claim 25, wherein the self-replicating RNA replicon comprises a Sindbis virus self-replicating RNA vector.

27. A composition according to claim 25, wherein the RNA replicon comprises SINrep5.

28. A composition according to any one of claims 1 to 25, wherein the nucleic acid or expression cassette is in the form of naked DNA.

29. A composition according to any one of claims 1 to 28, wherein the composition is formulated with phospholipids to form a liposome or is suitable to be administered intradermally, and/or is a DNA vaccine.

30. A particle comprising a nucleic acid as contained in a composition of any one of claims 1 to 27.

31. A particle according to claim 30, which comprises a material suitable for particle bombardment.

32. A particle according to claim 31, wherein the material is gold.

33. A particle according to any of claims 30 or 32 which is suitable to be administered by a gene gun.

34. A nucleic acid as defined in any of claims 1 to 27, a composition according to any of claims 1 to 29 or a particle according to any of claims 30 to 33 for use in a method of treatment of the human or animal body by therapy.

35. Use of a nucleic acid as defined in any of claims 1 to 27, a composition according to any of claims 1 to 29 or a particle according to any of claims 30 to 33 in the manufacture of a medicament for inducing an immune response.

36. Use according to claim 35, wherein the induced immune response comprises a cytotoxic T cell response and/or the medicament is a vaccine.

37. Use according to claim 35 or 36, wherein the medicament is for vaccinating a mammal against:
(a) infection by a pathogen; or
(b) an antigen against which an immune response is desired.

38. Use according to claim 37, wherein the antigen is a tumor antigen.

## Patentansprüche

1. Immunogene Zusammensetzung, umfassend eine Nukleinsäure, die für ein chimäres Polypeptid codiert, umfassend:
(a) eine erste Polypeptiddomäne, die ein Carobxy-terminales Fragment oder Domäne eines Hitzeschockproteins (HSP) ist, welche eine Chaperon-Aktivität aufweist;
(b) eine zweite Polypeptid-Domäne, umfassend ein antigenes Polypeptid;
wobei die Nukleinsäure, die für die zweite Polypeptiddomäne codiert, 5' zu der Nukleinsäure ist, die für die erste Polypeptiddomäne codiert, und die Nukleinsäure eine zelluläre Immunantwort auf ein antigenes Polypeptid auf Verabreichung an einen Säuger hin stimulieren oder verstärken kann.

2. Zusammensetzung nach Anspruch 1, welche die Immunogenität des antigenen Peptids stimulieren oder verstärken kann.

3. Zusammensetzung nach Anspruch 1 oder 2, worin das HSP HSP 70 umfasst.

4. Zusammensetzung nach Anspruch 3, worin das HSP von *Mycobacterium tuberculosis* abgeleitet ist.

5. Zusammensetzung nach einem der vorangehenden Ansprüche, wobei die Nukleinsäure eine zytotoxische T-Zellantwort induzieren kann.

6. Zusammensetzung nach einem der vorangehenden Ansprüche, wobei das HSP-Carboxy-terminale Fragment oder -Domäne ausgewählt ist aus:
(a) einer Sequenz, wie durch Reste 312 bis 625 der SEQ ID NR. 9 dargestellt; und
(b) einer Sequenz, wie durch Reste 517 bis 625 der SEQ ID NR. 9 dargestellt.

7. Zusammensetzung nach einem der vorangehenden Ansprüche, worin das antigene Polypeptid ein MHC Klasse I-bindendes Peptidepitop umfasst.

8. Zusammensetzung nach Anspruch 7, worin das MHC Klasse I-bindende Peptidepitop zwischen 8 Aminosäure-Resten und 11 Aminosäure-Resten lang ist.

9. Zusammensetzung nach einem der vorangehenden Ansprüche, worin das antigene Polypeptid von einem Virus abgeleitet ist, ein Tumor-spezifisches oder Tumor-assoziiertes Polypeptid ist oder von einem anderen Pathogen als einem Virus abgeleitet ist.

10. Zusammensetzung nach Anspruch 9, worin das Virus ein humanes Papovavirus, ein humanes Papilloma-Virus (HPV) oder ein humanes Immundefizienz-Virus (HIV) ist.

11. Zusammensetzung nach Anspruch 10, worin das Virus ein humanes Papilloma-Virus-16 (HPV-16) ist.

12. Zusammensetzung nach Anspruch 11, worin das antigene Polypeptid das HPV-16 E6- oder E7-Polypeptid oder ein epitopes Fragment davon umfasst.

13. Zusammensetzung nach Anspruch 9, worin das Tumor-spezifische oder Tumorassoziierte Polypeptid ein Antigen umfasst, das an der Oberfläche einer Tumorzelle exprimiert wird.

14. Zusammensetzung nach Anspruch 13, worin das Polypeptid ein Mutanten-p53, ein MAGE-1 oder ein MAGE-3 umfasst.

15. Zusammensetzung nach Anspruch 9, worin das antigene Polypeptid von einem HIV-1, einem bovinen viralen Diarrhoe-Virus, ein Cytomegalovirus, einem Enzephalitis-Virus, einem Hepatitis-Virus, einem Herpes simplex-Virus, einem Influenza-Virus oder einem Pathogen, welches Malaria verursacht, abgeleitet ist.

16. Zusammensetzung nach Anspruch 15, worin das Pathogen, welches Malaria verursacht, Malariapeptid (NANP) 40 ist.

17. Zusammensetzung nach einem der vorangehenden Ansprüche, worin das chimäre Polypeptid außerdem eine dritte Polypeptiddomäne umfasst, umfassend eine zytoplasmatische Translokalisations-Polypeptiddomäne.

18. Zusammensetzung nach Anspruch 17, worin die dritte Polypeptiddomäne eine zytoplasmatische Translokalisationsdomäne eines Pseudomonas-Exotoxin A (ETA) ist.

19. Zusammensetzung nach Anspruch 18, worin die dritte Polypeptiddomäne eine Sequenz umfasst, wie dargestellt durch Rest 253 bis Rest 364 der SEQ ID NR. 3.

20. Zusammensetzung nach Anspruch 17, worin die dritte Polypeptiddomäne eine zytoplasmatische Translokalisationsdomäne eines Toxins, abgeleitet von *Diphtheria, Clostridium, Botulinum, Bacillus, Yersinia, Vibrio cholerae* oder *Bordetella pertussis,* umfasst.

21. Zusammensetzung nach einem der Ansprüche 17 bis 20, worin die Nukleinsäure, die für die dritte Polypeptiddomäne codiert, 5' zu der Nukleinsäure, die für die zweite Domäne codiert, lokalisiert ist, oder zwischen den Nukleinsäuren, die für die erste und die zweite Domäne codieren, lokalisiert ist, oder 3' zu der Nukleinsäure, die für die erste Domäne codiert, lokalisiert ist.

22. Zusammensetzung nach einem der vorangehenden Ansprüche, außerdem umfassend regulatorische Nukleinsäuresequenzen.

23. Zusammensetzung nach Anspruch 22, worin die regulatorischen Sequenzen transkriptionelle regulatorische Sequenzen sind.

24. Zusammensetzung nach einem der vorangehenden Ansprüche, worin die Nukleinsäure in einer Expressionskassette enthalten ist.

25. Zusammensetzung nach Anspruch 24, worin die Expressionskassette einen Expressionsvektor, ein Plasmid oder ein selbstreplizierendes RNA-Replikon umfasst.

26. Zusammensetzung nach Anspruch 25, worin das selbstreplizierende RNA-Replikon einen selbstreplizierenden RNA-Vektor von Sindbis-Virus umfasst.

27. Zusammensetzung nach Anspruch 25, worin das RNA-Replikon SINrep5 umfasst.

28. Zusammensetzung nach einem der Ansprüche 1 bis 25, worin die Nukleinsäure oder Expressionskassette in der Form einer nackten DNA vorliegt.

29. Zusammensetzung nach einem der Ansprüche 1 bis 28, wobei die Zusammensetzung mit Phospholipiden formuliert ist, um ein Liposom zu bilden, oder geeignet ist, um intradermal verabreicht zu werden, und/oder eine DNA-Vakzine ist.

30. Partikel, umfassend eine Nukleinsäure, wie in einer Zusammensetzung nach einem der Ansprüche 1 bis 27 enthalten.

31. Partikel nach Anspruch 30, welches ein für das Partikelbombardment geeignetes Material umfasst.

32. Partikel nach Anspruch 31, worin das Material Gold ist.

33. Partikel nach einem der Ansprüche 30 bis 32, welches zur Verabreichung mittels einer Genkanone geeignet ist.

34. Nukleinsäure nach einem der Ansprüche 1 bis 27, Zusammensetzung nach einem der Ansprüche 1 bis 29 oder Partikel nach einem der Ansprüche 30 bis 33, zur Verwendung bei einer Methode zur Behandlung des menschlichen oder tierischen Körpers durch Therapie.

35. Verwendung einer Nukleinsäure, wie in einem der Ansprüche 1 bis 27 definiert, einer Zusammensetzung nach einem der Ansprüche 1 bis 29 oder eines Partikels nach einem der Ansprüche 30 bis 33, in der Herstellung eines Medikaments zum Induzieren einer Immunantwort.

36. Verwendung nach Anspruch 35, wobei die induzierte Immunantwort eine zytotoxische T-Zellantwort umfasst und/oder das Medikament ein Impfstoff ist.

37. Verwendung nach Anspruch 35 oder 36, wobei das Medikament zur Impfung eines Säugers ist gegen:
(a) eine Infektion durch ein Pathogen; oder
(b) ein Antigen, gegen das eine Immunantwort gewünscht wird.

38. Verwendung nach Anspruch 37, wobei das Antigen ein Tumorantigen ist.

## Revendications

1. Composition immunogène comprenant un acide nucléique qui code pour un polypeptide chimérique comprenant:
(a) un premier domaine polypeptidique, qui est un fragment carboxy-terminal ou un domaine d'une protéine de choc thermique (HSP) qui a une activité de chaperonne ;
(b) un deuxième domaine polypeptidique comprenant un polypeptide antigénique ;
dans lequel l'acide nucléique codant pour le deuxième domaine polypeptidique est en 5' de l'acide nucléique codant pour le premier domaine polypeptidique, et l'acide nucléique peut stimuler ou favoriser une réponse immunitaire cellulaire au polypeptide antigénique à l'administration à un mammifère.

2. Composition selon la revendication 1, qui peut stimuler ou favoriser l'immunogénicité du peptide antigénique.

3. Composition selon la revendication 1 ou 2, dans laquelle la HSP comprend HSP 70.

4. Composition selon la revendication 3, dans laquelle la HSP est dérivée d'un *Mycobacterium tuberculosis.*

5. Composition selon l'une quelconque des revendications précédentes, dans laquelle l'acide nucléique peut induire une réponse cytotoxique des cellules T.

6. Composition selon l'une quelconque des revendications précédentes, dans laquelle le fragment carboxy-terminal ou domaine de HSP est choisi parmi:
(a) une séquence telle que représentée par les résidus 312 à 625 de la SEQ ID NO : 9 ; et
(b) une séquence telle que représentée par les résidus 517 à 625 de la SEQ ID NO: 9.

7. Composition selon l'une quelconque des revendications précédentes, dans laquelle le polypeptide antigénique comprend un épitope peptidique de liaison au CMH de classe I.

8. Composition selon la revendication 7, dans laquelle l'épitope peptidique de liaison au CMH de classe I a une longueur comprise entre 8 résidus d'acides aminés et 11 résidus d'acides aminés.

9. Composition selon l'une quelconque des revendications précédentes, dans laquelle le polypeptide antigénique est dérivé d'un virus, est un polypeptide spécifique d'une tumeur ou associé à une tumeur, ou est dérivé d'un pathogène autre qu'un virus.

10. Composition selon la revendication 9, dans laquelle le virus est un papovavirus humain, un virus du papillome humain (VPH) ou un virus de l'immunodéficience humaine (VIH).

11. Composition selon la revendication 10, dans laquelle le virus est un virus du papillome humain de type 16 (VPH-16).

12. Composition selon la revendication 11, dans laquelle le polypeptide antigénique comprend le polypeptide E6 ou E7 de VPH-16 ou un fragment épitopique de celui-ci.

13. Composition selon la revendication 9, dans laquelle le polypeptide spécifique d'une tumeur ou associé à une tumeur comprend un antigène exprimé à la surface d'une cellule tumorale.

14. Composition selon la revendication 13, dans laquelle le polypeptide comprend un mutant p53, un MAGE-1 ou un MAGE-3.

15. Composition selon la revendication 9, dans laquelle le polypeptide antigénique est dérivé d'un VIH-1, d'un virus de la diarrhée virale bovine, d'un cytomégalovirus, d'un virus de l'encéphalite, d'un virus de l'hépatite, d'un virus de l'herpès simplex, d'un virus de l'influenza ou d'un pathogène responsable du paludisme.

16. Composition selon la revendication 15, dans laquelle le pathogène responsable du paludisme est le peptide du paludisme (NANP) 40.

17. Composition selon l'une quelconque des revendications précédentes, dans laquelle le polypeptide chimérique comprend en outre un troisième domaine polypeptidique comprenant un domaine polypeptidique de translocation cytoplasmique.

18. Composition selon la revendication 17, dans laquelle le troisième domaine polypeptidique est un domaine de translocation cytoplasmique d'une exotoxine A de *Pseudomonas* (ETA).

19. Composition selon la revendication 18, dans laquelle le troisième domaine polypeptidique comprend une séquence telle que représentée par les résidus 253 à 364 de la SEQ ID NO :3.

20. Composition selon la revendication 17, dans laquelle le troisième domaine polypeptidique comprend un domaine de translocation cytoplasmique d'une toxine dérivée de *Diphtheria, Clostridium, Botulinum, Bacillus, Yersinia, Vibrio cholerae* ou *Bordetella pertussis.*

21. Composition selon l'une quelconque des revendications 17 à 20, dans laquelle l'acide nucléique codant pour le troisième domaine polypeptidique est situé en 5' de l'acide nucléique codant pour le deuxième domaine, ou est situé entre les acides nucléiques codant pour le premier et le deuxième domaine, ou est situé en 3' de l'acide nucléique codant pour le premier domaine.

22. Composition selon l'une quelconque des revendications précédentes comprenant en outre des séquences d'acide nucléique régulatrices.

23. Composition selon la revendication 22, dans laquelle les séquences régulatrices sont des séquences régulatrices transcriptionnelles.

24. Composition selon l'une quelconque des revendications précédentes dans laquelle l'acide nucléique est compris dans une cassette d'expression.

25. Composition selon la revendication 24, dans laquelle la cassette d'expression comprend un vecteur d'expression, un plasmide ou un réplicon d'ARN auto-réplicable.

26. Composition selon la revendication 25, dans laquelle le réplicon d'ARN auto-réplicable comprend un vecteur d'ARN auto-réplicable du virus Sindbis.

27. Composition selon la revendication 25, dans laquelle le réplicon d'ARN comprend SINrep5.

28. Composition selon l'une quelconque des revendications 1 à 25, dans laquelle l'acide nucléique ou la cassette d'expression est sous la forme d'ADN nu.

29. Composition selon l'une quelconque des revendications 1 à 28, dans laquelle la composition est préparée avec des phospholipides pour former un liposome ou est adaptée pour être administrée par voie intradermique, et/ou est un vaccin à ADN.

30. Une particule comprenant un acide nucléique tel que contenu dans une composition selon l'une quelconque des revendications 1 à 27.

31. Une particule selon la revendication 30, qui comprend un matériau adapté au bombardement de particules.

32. Une particule selon la revendication 31, dans laquelle le matériau est l'or.

33. Particule selon l'une quelconque des revendications 30 ou 32 qui est adaptée pour être administrée par un canon à gènes.

34. Acide nucléique tel que défini selon l'une quelconque des revendications 1 à 27, une composition selon l'une quelconque des revendications 1 à 29 ou une particule selon l'une quelconque des revendications 30 à 33 destinés à l'utilisation dans une méthode de traitement du corps humain ou animal par thérapie.

35. Utilisation d'un acide nucléique tel que défini selon l'une quelconque des revendications 1 à 27, d'une composition selon l'une quelconque des revendications 1 à 29 ou d'une particule selon l'une quelconque des revendications 30 à 33 dans la fabrication d'un médicament pour induire une réponse immunitaire .

36. Utilisation selon la revendication 35, dans laquelle la réponse immunitaire induite comprend une réponse cytotoxique des cellules T et/ou le médicament est un vaccin.

37. Utilisation selon la revendication 35 ou 36, dans laquelle le médicament est destiné à la vaccination d'un mammifère contre:
(a) une infection par un pathogène ; ou
(b) un antigène contre lequel une réponse immunitaire est souhaitée.

38. Utilisation selon la revendication 37, dans laquelle l'antigène est un antigène tumoral.


### SEQUENCE LISTING

<110>Johns Hopkins University School of Medecine
<120> CHIMERIC IMMUNOGENIC COMPOSITIONS AND NUCLEIC ACIDS ENCODING THEM
<130> 02240-169351
<140>PCT/US00/041422
   <141>2000-10-20
<150> US 09/501,097
   <151>2000-02-09
<160< 25
<170> FastSeQ for Windows Version 4.0
<210>1
   <211< 141
   <212> PRT
   <213> mouse
<400>
<210> 2
   <211< 916
   <212> DNA
   <213> mouse
<400> 2
<210> 3
   <211> 638
   <212> PRT
   <213> Pseudomonas
<400> 3
   Met His Leu Ile Pro His Trp Ile Pro Leu Val Ala Ser Leu Gly Leu
    1               5                  10                  15
   Leu Ala Gly Gly Ser Ser Ala Ser Ala Ala Glu Glu Ala Phe Asp Leu
               20                  25                  30
   Trp Asn Glu Cys Ala Lys Ala Cys Val Leu Asp Leu Lys Asp Gly Val
           35                  40                  45
   Arg Ser Ser Arg Met Ser Val Asp Pro Ala Ile Ala Asp Thr Asn Gly
       50                  55                  60
   Gln Gly Val Leu His Tyr Ser Met Val Leu Glu Gly Gly Asn Asp Ala
   65                  70                  75                  80
   Leu Lys Leu Ala Ile Asp Asn Ala Leu Ser Ile Thr Ser Asp Gly Leu
                   85                  90                  95
   Thr Ile Arg Leu Glu Gly Gly Val Glu Pro Asn Lys Pro Val Arg Tyr
               100                 105                 110
   Ser Tyr Thr Arg Gln Ala Arg Gly Ser Trp Ser Leu Asn Trp Leu Val
           115                 120                 125
   Pro Ile Gly His Glu Lys Pro Ser Asn Ile Lys Val Phe Ile His Glu
       130                 135                 140
   Leu Asn Ala Gly Asn Gln Leu Ser His Met Ser Pro Ile Tyr Thr Ile
   145                 150                 155                 160
   Glu Met Gly Asp Glu Leu Leu Ala Lys Leu Ala Arg Asp Ala Thr Phe
                   165                 170                 175
   Phe Val Arg Ala His Glu Ser Asn Glu Met Gln Pro Thr Leu Ala Ile
               180                 185                 190
   Ser His Ala Gly Val Ser Val Val Met Ala Gln Thr Gln Pro Arg Arg
           195                 200                 205
   Glu Lys Arg Trp Ser Glu Trp Ala Ser Gly Lys Val Leu Cys Leu Leu
       210                 215                 220
   Asp Pro Leu Asp Gly Val Tyr Asn Tyr Leu Ala Gln Gln Arg Cys Asn
   225                 230                 235                 240
   Leu Asp Asp Thr Trp Glu Gly Lys Ile Tyr Arg Val Leu Ala Gly Asn
                   245                 250                 255
   Pro Ala Lys His Asp Leu Asp Ile Lys Pro Thr Val Ile Ser His Arg
               260                 265                 270
   Leu His Phe Pro Glu Gly Gly Ser Leu Ala Ala Leu Thr Ala His Gln
           275                 280                 285
   Ala Cys His Leu Pro Leu Glu Thr Phe Thr Arg His Arg Gln Pro Arg
       290                 295                 300
   Gly Trp Glu Gln Leu Glu Gln Cys Gly Tyr Pro Val Gln Arg Leu Val
   305                 310                 315                 320
   Ala Leu Tyr Leu Ala Ala Arg Leu Ser Trp Asn Gln Val Asp Gln Val
                   325                 330                 335
   Ile Arg Asn Ala Leu Ala Ser Pro Gly Ser Gly Gly Asp Leu Gly Glu
               340                 345                 350
   Ala Ile Arg Glu Gln Pro Glu Gln Ala Arg Leu Ala Leu Thr Leu Ala
           355                 360                 365
   Ala Ala Glu Ser Glu Arg Phe Val Arg Gln Gly Thr Gly Asn Asp Glu
       370                 375                 380
   Ala Gly Ala Ala Asn Ala Asp Val Val Ser Leu Thr Cys Pro Val Ala
   385                 390                 395                 400
   Ala Gly Glu Cys Ala Gly Pro Ala Asp Ser Gly Asp Ala Leu Leu Glu
                   405                 410                 415
   Arg Asn Tyr Pro Thr Gly Ala Glu Phe Leu Gly Asp Gly Gly Asp Val
               420                 425                 430
   Ser Phe Ser Thr Arg Gly Thr Gln Asn Trp Thr Val Glu Arg Leu Leu
           435                 440                 445
   Gln Ala His Arg Gln Leu Glu Glu Arg Gly Tyr Val Phe Val Gly Tyr
       450                 455                 460
   His Gly Thr Phe Leu Glu Ala Ala Gln Ser Ile Val Phe Gly Gly Val
   465                 470                 475                 480
   Arg Ala Arg Ser Gln Asp Leu Asp Ala Ile Trp Arg Gly Phe Tyr Ile
                   485                 490                 495
   Ala Gly Asp Pro Ala Leu Ala Tyr Gly Tyr Ala Gln Asp Gln Glu Pro
               500                 505                 510
   Asp Ala Arg Gly Arg Ile Arg Asn Gly Ala Leu Leu Arg Val Tyr Val
           515                 520                 525
   Pro Arg Ser Ser Leu Pro Gly Phe Tyr Arg Thr Ser Leu Thr Leu Ala
       530                 535                 540
   Ala Pro Glu Ala Ala Gly Glu Val Glu Arg Leu Ile Gly His Pro Leu
   545                 550                 555                 560
   Pro Leu Arg Leu Asp Ala Ile Thr Gly Pro Glu Glu Glu Gly Gly Arg
                   565                 570                 575
   Leu Glu Thr Ile Leu Gly Trp Pro Leu Ala Glu Arg Thr Val Val Ile
               580                 585                 590
   Pro Ser Ala Ile Pro Thr Asp Pro Arg Asn Val Gly Gly Asp Leu Asp
           595                 600                 605
   Pro Ser Ser Ile Pro Asp Lys Glu Gln Ala Ile Ser Ala Leu Pro Asp
       610                 615                 620
   Tyr Ala Ser Gln Pro Gly Lys Pro Pro Arg Glu Asp Leu Lys
   625                 630                 635
<210> 4
   <211> 2760
   <212> DNA
   <213> Pseudomonas
<400> 4
   ctgcagctgg tcaggccgtt tccgcaacgc ttgaagtcct ggccgatata ccggcagggc     60
   cagccatcgt tcgacgaata aagccacctc agccatgatg ccctttccat ccccagcgga    120
   accccgacat ggacgccaaa gccctgctcc tcggcagcct ctgcctggcc gccccattcg    180
   ccgacgcggc gacgctcgac aatgctctct ccgcctgcct cgccgcccgg ctcggtgcac    240
   cgcacacggc ggagggccag ttgcacctgc cactcaccct tgaggcccgg cgctccaccg    300
   gcgaatgcgg ctgtacctcg gcgctggtgc gatatcggct gctggccagg ggcgccagcg    360
   ccgacagcct cgtgcttcaa gagggctgct cgatagtcgc caggacacgc cgcgcacgct    420
   gaccctggcg gcggacgccg gcttggcgag cggccgcgaa ctggtcgtca ccctgggttg    480
   tcaggcgcct gactgacagg ccgggctgcc accaccaggc cgagatggac gccctgcatg    540
   tatcctccga tcggcaagcc tcccgttcgc acattcacca ctctgcaatc cagttcataa    600
   atcccataaa agccctcttc cgctccccgc cagcctcccc gcatcccgca ccctagacgc    660
   cccgccgctc tccgccggct cgcccgacaa gaaaaaccaa ccgctcgatc agcctcatcc    720
   ttcacccatc acaggagcca tcgcgatgca cctgataccc cattggatcc ccctggtcgc    780
   cagcctcggc ctgctcgccg gcggctcgtc cgcgtccgcc gccgaggaag ccttcgacct    840
   ctggaacgaa tgcgccaaag cctgcgtgct cgacctcaag gacggcgtgc gttccagccg    900
   catgagcgtc gacccggcca tcgccgacac caacggccag ggcgtgctgc actactccat    960
   ggtcctggag ggcggcaacg acgcgctcaa gctggccatc gacaacgccc tcagcatcac   1020
   cagcgacggc ctgaccatcc gcctcgaagg cggcgtcgag ccgaacaagc cggtgcgcta   1080
   cagctacacg cgccaggcgc gcggcagttg gtcgctgaac tggctggtac cgatcggcca   1140
   cgagaagccc tcgaacatca aggtgttcat ccacgaactg aacgccggca accagctcag   1200
   ccacatgtcg ccgatctaca ccatcgagat gggcgacgag ttgctggcga agctggcgcg   1260
   cgatgccacc ttcttcgtca gggcgcacga gagcaacgag atgcagccga cgctcgccat   1320
   cagccatgcc ggggtcagcg tggtcatggc ccagacccag ccgcgccggg aaaagcgctg   1380
   gagcgaatgg gccagcggca aggtgttgtg cctgctcgac ccgctggacg gggtctacaa   1440
   ctacctcgcc cagcaacgct gcaacctcga cgatacctgg gaaggcaaga tctaccgggt   1500
   gctcgccggc aacccggcga agcatgacct ggacatcaaa cccacggtca tcagtcatcg   1560
   cctgcacttt cccgagggcg gcagcctggc cgcgctgacc gcgcaccagg cttgccacct   1620
   gccgctggag actttcaccc gtcatcgcca gccgcgcggc tgggaacaac tggagcagtg   1680
   cggctatccg gtgcagcggc tggtcgccct ctacctggcg gcgcggctgt cgtggaacca   1740
   ggtcgaccag gtgatccgca acgccctggc cagccccggc agcggcggcg acctgggcga   1800
   agcgatccgc gagcagccgg agcaggcccg tctggccctg accctggccg ccgccgagag   1860
   cgagcgcttc gtccggcagg gcaccggcaa cgacgaggcc ggcgcggcca acgccgacgt   1920
   ggtgagcctg acctgcccgg tcgccgccgg tgaatgcgcg ggcccggcgg acagcggcga   1980
   cgccctgctg gagcgcaact atcccactgg cgcggagttc ctcggcgacg gcggcgacgt   2040
   cagcttcagc acccgcggca cgcagaactg gacggtggag cggctgctcc aggcgcaccg   2100
   ccaactggag gagcgcggct atgtgttcgt cggctaccac ggcaccttcc tcgaagcggc   2160
   gcaaagcatc gtcttcggcg gggtgcgcgc gcgcagccag gacctcgacg cgatctggcg   2220
   cggtttctat atcgccggcg atccggcgct ggcctacggc tacgcccagg accaggaacc   2280
   cgacgcacgc ggccggatcc gcaacggtgc cctgctgcgg gtctatgtgc cgcgctcgag   2340
   cctgccgggc ttctaccgca ccagcctgac cctggccgcg ccggaggcgg cgggcgaggt   2400
   cgaacggctg atcggccatc cgctgccgct gcgcctggac gccatcaccg gccccgagga   2460
   ggaaggcggg cgcctggaga ccattctcgg ctggccgctg gccgagcgca ccgtggtgat   2520
   tccctcggcg atccccaccg acccgcgcaa cgtcggcggc gacctcgacc cgtccagcat   2580
   ccccgacaag gaacaggcga tcagcgccct gccggactac gccagccagc ccggcaaacc   2640
   gccgcgcgag gacctgaagt aactgccgcg accggccggc tcccttcgca ggagccggcc   2700
   ttctcggggc ctggccatac atcaggtttt cctgatgcca gcccaatcga atatgaattc   2760

<210> 5
   <211> 9
   <212> PRT
   <213> human papillomavirus
<400> 5
   Arg Ala His Tyr Asn Ile Val Thr Phe
    1               5
<210> 6
   <211> 38
   <212> PRT
   <213> human papillomavirus
<210> 7
   <211> 98
   <212> PRT
   <213> human papillomavirus
<210> 8
   <211> 297
   <212> DNA
   <213> human papillomavirus
<400> 8
   atgcatggag atacacctac attgcatgaa tatatgttag atttgcaacc agagacaact      60
   gatctctact gttatgagca attaaatgac agctcagagg aggaggatga aatagatggt     120
   ccagctggac aagcagaacc ggacagagcc cattacaata ttgtaacctt ttgttgcaag     180
   tgtgactcta cgcttcggtt gtgcgtacaa agcacacacg tagacattcg tactttggaa     240
   gacctgttaa tgggcacact aggaattgtg tgccccatct gttctcagaa accataa        297
<210> 9
   <211> 625
   <212> PRT
   <213>Mycobacterium tuberculosis
<400> 9
   Met Ala Arg Ala Val Gly Ile Asp Leu Gly Thr Thr Asn Ser Val Val
    1               5                  10                  15
   Ser Val Leu Glu Gly Gly Asp Pro Val Val Val Ala Asn Ser Glu Gly
               20                  25                  30
   Ser Arg Thr Thr Pro Ser Ile Val Ala Phe Ala Arg Asn Gly Glu Val
           35                  40                  45
   Leu Val Gly Gln Pro Ala Lys Asn Gln Ala Val Thr Asn Val Asp Arg
       50                  55                  60
   Thr Val Arg Ser Val Lys Arg His Met Gly Ser Asp Trp Ser Ile Glu
   65                  70                  75                  80
   Ile Asp Gly Lys Lys Tyr Thr Ala Pro Glu Ile Ser Ala Arg Ile Leu
                   85                  90                  95
   Met Lys Leu Lys Arg Asp Ala Glu Ala Tyr Leu Gly Glu Asp Ile Thr
               100                 105                 110
   Asp Ala Val Ile Thr Thr Pro Ala Tyr Phe Asn Asp Ala Gln Arg Gln
           115                 120                 125
   Ala Thr Lys Asp Ala Gly Gln Ile Ala Gly Leu Asn Val Leu Arg Ile
       130                 135                 140
   Val Asn Glu Pro Thr Ala Ala Ala Leu Ala Tyr Gly Leu Asp Lys Gly
   145                 150                 155                 160
   Glu Lys Glu Gln Arg Ile Leu Val Phe Asp Leu Gly Gly Gly Thr Phe
                   165                 170                 175
   Asp Val Ser Leu Leu Glu Ile Gly Glu Gly Val Val Glu Val Arg Ala
               180                 185                 190
   Thr Ser Gly Asp Asn His Leu Gly Gly Asp Asp Trp Asp Gln Arg Val
           195                 200                 205
   Val Asp Trp Leu Val Asp Lys Phe Lys Gly Thr Ser Gly Ile Asp Leu
       210                 215                 220
   Thr Lys Asp Lys Met Ala Met Gln Arg Leu Arg Glu Ala Ala Glu Lys
   225                 230                 235                 240
   Ala Lys Ile Glu Leu Ser Ser Ser Gln Ser Thr Ser Ile Asn Leu Pro
                   245                 250                 255
   Tyr Ile Thr Val Asp Ala Asp Lys Asn Pro Leu Phe Leu Asp Glu Gln
                   260                 265                 270
   Leu Thr Arg Ala Glu Phe Gln Arg Ile Thr Gln Asp Leu Leu Asp Arg
           275                 280                 285
   Thr Arg Lys Pro Phe Gln Ser Val Ile Ala Asp Thr Gly Ile Ser Val
       290                 295                 300
   Ser Glu Ile Asp His Val Val Leu Val Gly Gly Ser Thr Arg Met Pro
   305                 310                 315                 320
   Ala Val Thr Asp Leu Val Lys Glu Leu Thr Gly Gly Lys Glu Pro Asn
                   325                 330                 335
   Lys Gly Val Asn Pro Asp Glu Val Val Ala Val Gly Ala Ala Leu Gln
                   340                 345                 350
   Ala Gly Val Leu Lys Gly Glu Val Lys Asp Val Leu Leu Leu Asp Val
           355                 360                 365
   Thr Pro Leu Ser Leu Gly Ile Glu Thr Lys Gly Gly Val Met Thr Arg
       370                 375                 380
   Leu Ile Glu Arg Asn Thr Thr Ile Pro Thr Lys Arg Ser Glu Thr Phe
   385                 390                 395                 400
   Thr Thr Ala Asp Asp Asn Gln Pro Ser Val Gln Ile Gln Val Tyr Gln
                   405                 410                 415
   Gly Glu Arg Glu Ile Ala Ala His Asn Lys Leu Leu Gly Ser Phe Glu
                   420                 425                 430
   Leu Thr Gly Ile Pro Pro Ala Pro Arg Gly Ile Pro Gln Ile Glu Val
           435                 440                 445
   Thr Phe Asp Ile Asp Ala Asn Gly Ile Val His Val Thr Ala Lys Asp
       450                 455                 460
   Lys Gly Thr Gly Lys Glu Asn Thr Ile Arg Ile Gln Glu Gly Ser Gly
   465                 470                 475                 480
   Leu Ser Lys Glu Asp Ile Asp Arg Met Ile Lys Asp Ala Glu Ala His
                   485                 490                 495
   Ala Glu Glu Asp Arg Lys Arg Arg Glu Glu Ala Asp Val Arg Asn Gln
                   500                 505                 510
   Ala Glu Thr Leu Val Tyr Gln Thr Glu Lys Phe Val Lys Glu Gln Arg
           515                 520                 525
   Glu Ala Glu Gly Gly Ser Lys Val Pro Glu Asp Thr Leu Asn Lys Val
       530                 535                 540
   Asp Ala Ala Val Ala Glu Ala Lys Ala Ala Leu Gly Gly Ser Asp Ile
   545                 550                 555                 560
   Ser Ala Ile Lys Ser Ala Met Glu Lys Leu Gly Gln Glu Ser Gln Ala
                   565                 570                 575
   Leu Gly Gln Ala Ile Tyr Glu Ala Ala Gln Ala Ala Ser Gln Ala Thr
                   580                 585                 590
   Gly Ala Ala His Pro Gly Gly Glu Pro Gly Gly Ala His Pro Gly Ser
           595                 600                 605
   Ala Asp Asp Val Val Asp Ala Glu Val Val Asp Asp Gly Arg Glu Ala
       610                 615                 620
   Lys
   625
<210> 10
   <211> 1878
   <212> DNA
   <213> Mycobacterium tuberculosis
<400> 10
   atggctcgtg cggtcgggat cgacctcggg accaccaact ccgtcgtctc ggttctggaa      60
   ggtggcgacc cggtcgtcgt cgccaactcc gagggctcca ggaccacccc gtcaattgtc     120
   gcgttcgccc gcaacggtga ggtgctggtc ggccagcccg ccaagaacca ggcagtgacc     180
   aacgtcgatc gcaccgtgcg ctcggtcaag cgacacatgg gcagcgactg gtccatagag     240
   attgacggca agaaatacac cgcgccggag atcagcgccc gcattctgat gaagctgaag     300
   cgcgacgccg aggcctacct cggtgaggac attaccgacg cggttatcac gacgcccgcc     360
   tacttcaatg acgcccagcg tcaggccacc aaggacgccg gccagatcgc cggcctcaac     420
   gtgctgcgga tcgtcaacga gccgaccgcg gccgcgctgg cctacggcct cgacaagggc     480
   gagaaggagc agcgaatcct ggtcttcgac ttgggtggtg gcactttcga cgtttccctg     540
   ctggagatcg gcgagggtgt ggttgaggtc cgtgccactt cgggtgacaa ccacctcggc     600
   ggcgacgact gggaccagcg ggtcgtcgat tggctggtgg acaagttcaa gggcaccagc     660
   ggcatcgatc tgaccaagga caagatggcg atgcagcggc tgcgggaagc cgccgagaag     720
   gcaaagatcg agctgagttc gagtcagtcc acctcgatca acctgcccta catcaccgtc     780
   gacgccgaca agaacccgtt gttcttagac gagcagctga cccgcgcgga gttccaacgg     840
   atcactcagg acctgctgga ccgcactcgc aagccgttcc agtcggtgat cgctgacacc     900
   ggcatttcgg tgtcggagat cgatcacgtt gtgctcgtgg gtggttcgac ccggatgccc     960
   gcggtgaccg atctggtcaa ggaactcacc ggcggcaagg aacccaacaa gggcgtcaac    1020
   cccgatgagg ttgtcgcggt gggagccgct ctgcaggccg gcgtcctcaa gggcgaggtg    1080
   aaagacgttc tgctgcttga tgttaccccg ctgagcctgg gtatcgagac caagggcggg    1140
   gtgatgacca ggctcatcga gcgcaacacc acgatcccca ccaagcggtc ggagactttc    1200
   accaccgccg acgacaacca accgtcggtg cagatccagg tctatcaggg ggagcgtgag    1260
   atcgccgcgc acaacaagtt gctcgggtcc ttcgagctga ccggcatccc gccggcgccg    1320
   cgggggattc cgcagatcga ggtcactttc gacatcgacg ccaacggcat tgtgcacgtc    1380
   accgccaagg acaagggcac cggcaaggag aacacgatcc gaatccagga aggctcgggc    1440
   ctgtccaagg aagacattga ccgcatgatc aaggacgccg aagcgcacgc cgaggaggat    1500
   cgcaagcgtc gcgaggaggc cgatgttcgt aatcaagccg agacattggt ctaccagacg    1560
   gagaagttcg tcaaagaaca gcgtgaggcc gagggtggtt cgaaggtacc tgaagacacg    1620
   ctgaacaagg ttgatgccgc ggtggcggaa gcgaaggcgg cacttggcgg atcggatatt    1680
   tcggccatca agtcggcgat ggagaagctg ggccaggagt cgcaggctct ggggcaagcg    1740
   atctacgaag cagctcaggc tgcgtcacag gccactggcg ctgcccaccc cggcggcgag    1800
   ccgggcggtg cccaccccgg ctcggctgat gacgttgtgg acgcggaggt ggtcgacgac    1860
   ggccgggagg ccaagtga                                                  1878
<210> 11
   <211> 30
   <212> DNA
   <213> synthetic
<400> 11
   ggggagatct ggatgtggct gcagaattta 30
<210> 12
   <211> 31
   <212> DNA
   <213> synthetic
<400> 12
   gggagaattc ttttggactg gttttttgca t 31
<210> 13
   <211> 30
   <212> DNA
   <213> synthetic
<400> 13
   gggagaattc tcgcctgtca 30
<210> 14
   <211> 28
   <212> DNA
   <213> synthetic
<400> 14
   ggaaggatcc agttctgcgt gccgcggg 28
<210> 15
   <211> 30
   <212> DNA
   <213> synthetic
<400> 15
   gggaggatcc tcatgcatgg agatacacct 30
<210> 16
   <211> 29
   <212> DNA
   <213> synthetic
<400> 16
   ggggaagctt atcctgagaa cagatgggg 29
<210> 17
   <211> 20
   <212> DNA
   <213> synthetic
<400> 17
   cccagatcta atcatgcatg 20
<210> 18
   <211> 29
   <212> DNA
   <213> synthetic
<400> 18
   ggggaagctt atcctgagaa cagatgggg 29
<210> 19
   <211> 2172
   <212> DNA
   <213> human papillomavirus/Mycobacterium tuberculosis
<400> 19
   atgcatggag atacacctac attgcatgaa tatatgttag atttgcaacc agagacaact      60
   gatctctact gttatgagca attaaatgac agctcagagg aggaggatga aatagatggt     120
   ccagctggac aagcagaacc ggacagagcc cattacaata ttgtaacctt ttgttgcaag     180
   tgtgactcta cgcttcggtt gtgcgtacaa agcacacacg tagacattcg tactttggaa     240
   gacctgttaa tgggcacact aggaattgtg tgccccatct gttctcaagg atccatggct     300
   cgtgcggtcg ggatcgacct cgggaccacc aactccgtcg tctcggttct ggaaggtggc     360
   gacccggtcg tcgtcgccaa ctccgagggc tccaggacca ccccgtcaat tgtcgcgttc     420
   gcccgcaacg gtgaggtgct ggtcggccag cccgccaaga accaggcagt gaccaacgtc     480
   gatcgcaccg tgcgctcggt caagcgacac atgggcagcg actggtccat agagattgac     540
   ggcaagaaat acaccgcgcc ggagatcagc gcccgcattc tgatgaagct gaagcgcgac     600
   gccgaggcct acctcggtga ggacattacc gacgcggtta tcacgacgcc cgcctacttc     660
   aatgacgccc agcgtcaggc caccaaggac gccggccaga tcgccggcct caacgtgctg     720
   cggatcgtca acgagccgac cgcggccgcg ctggcctacg gcctcgacaa gggcgagaag     780
   gagcagcgaa tcctggtctt cgacttgggt ggtggcactt tcgacgtttc cctgctggag    840
   atcggcgagg gtgtggttga ggtccgtgcc acttcgggtg acaaccacct cggcggcgac    900
   gactgggacc agcgggtcgt cgattggctg gtggacaagt tcaagggcac cagcggcatc    960
   gatctgacca aggacaagat ggcgatgcag cggctgcggg aagccgccga gaaggcaaag   1020
   atcgagctga gttcgagtca gtccacctcg atcaacctgc cctacatcac cgtcgacgcc   1080
   gacaagaacc cgttgttctt agacgagcag ctgacccgcg cggagttcca acggatcact   1140
   caggacctgc tggaccgcac tcgcaagccg ttccagtcgg tgatcgctga caccggcatt   1200
   tcggtgtcgg agatcgatca cgttgtgctc gtgggtggtt cgacccggat gcccgcggtg   1260
   accgatctgg tcaaggaact caccggcggc aaggaaccca acaagggcgt caaccccgat   1320
   gaggttgtcg cggtgggagc cgctctgcag gccggcgtcc tcaagggcga ggtgaaagac   1380
   gttctgctgc ttgatgttac cccgctgagc ctgggtatcg agaccaaggg cggggtgatg    1440
   accaggctca tcgagcgcaa caccacgatc cccaccaagc ggtcggagac tttcaccacc   1500
   gccgacgaca accaaccgtc ggtgcagatc caggtctatc agggggagcg tgagatcgcc  1560
   gcgcacaaca agttgctcgg gtccttcgag ctgaccggca tcccgccggc gccgcggggg   1620
   attccgcaga tcgaggtcac tttcgacatc gacgccaacg gcattgtgca cgtcaccgcc   1680
   aaggacaagg gcaccggcaa ggagaacacg atccgaatcc aggaaggctc gggcctgtcc   1740
   aaggaagaca ttgaccgcat gatcaaggac gccgaagcgc acgccgagga ggatcgcaag   1800
   cgtcgcgagg aggccgatgt tcgtaatcaa gccgagacat tggtctacca gacggagaag   1860
   ttcgtcaaag aacagcgtga ggccgagggt ggttcgaagg tacctgaaga cacgctgaac   1920
   aaggttgatg ccgcggtggc ggaagcgaag gcggcacttg gcggatcgga tatttcggcc   1980
   atcaagtcgg cgatggagaa gctgggccag gagtcgcagg ctctggggca agcgatctac   2040
   gaagcagctc aggctgcgtc acaggccact ggcgctgccc accccggcgg cgagccgggc   2100
   ggtgcccacc ccggctcggc tgatgacgtt gtggacgcgg aggtggtcga cgacggccgg   2160
   gaggccaagt ga                                                       2172
<210> 20
   <211> 723
   <212> PRT
   <213> human papillomavirus/Mycobacterium tuberculosis
<400> 20
   Met His Gly Asp Thr Pro Thr Leu His Glu Tyr Met Leu Asp Leu Gln
    1               5                  10                  15
   Pro Glu Thr Thr Asp Leu Tyr Cys Tyr Glu Gln Leu Asn Asp Ser Ser
               20                  25                  30
   Glu Glu Glu Asp Glu Ile Asp Gly Pro Ala Gly Gln Ala Glu Pro Asp
           35                  40                  45
   Arg Ala His Tyr Asn Ile Val Thr Phe Cys Cys Lys Cys Asp Ser Thr
       50                  55                  60
   Leu Arg Leu Cys Val Gln Ser Thr His Val Asp Ile Arg Thr Leu Glu
   65                  70                  75                  80
   Asp Leu Leu Met Gly Thr Leu Gly Ile Val Cys Pro Ile Cys Ser Gln
                   85                  90                  95
   Gly Ser Met Ala Arg Ala Val Gly Ile Asp Leu Gly Thr Thr Asn Ser
               100                 105                 110
   Val Val Ser Val Leu Glu Gly Gly Asp Pro Val Val Val Ala Asn Ser
           115                 120                 125
   Glu Gly Ser Arg Thr Thr Pro Ser Ile Val Ala Phe Ala Arg Asn Gly
       130                 135                 140
   Glu Val Leu Val Gly Gln Pro Ala Lys Asn Gln Ala Val Thr Asn Val
   145                 150                 155                 160
   Asp Arg Thr Val Arg Ser Val Lys Arg His Met Gly Ser Asp Trp Ser
                   165                 170                 175
   Ile Glu Ile Asp Gly Lys Lys Tyr Thr Ala Pro Glu Ile Ser Ala Arg
               180                 185                 190
   Ile Leu Met Lys Leu Lys Arg Asp Ala Glu Ala Tyr Leu Gly Glu Asp
           195                 200                 205
   Ile Thr Asp Ala Val Ile Thr Thr Pro Ala Tyr Phe Asn Asp Ala Gln
       210                 215                 220
   Arg Gln Ala Thr Lys Asp Ala Gly Gln Ile Ala Gly Leu Asn Val Leu
   225                 230                 235                 240
   Arg Ile Val Asn Glu Pro Thr Ala Ala Ala Leu Ala Tyr Gly Leu Asp
                   245                 250                 255
   Lys Gly Glu Lys Glu Gln Arg Ile Leu Val Phe Asp Leu Gly Gly Gly
               260                 265                 270
   Thr Phe Asp Val Ser Leu Leu Glu Ile Gly Glu Gly Val Val Glu Val
           275                 280                 285
   Arg Ala Thr Ser Gly Asp Asn His Leu Gly Gly Asp Asp Trp Asp Gln
       290                 295                 300
   Arg Val Val Asp Trp Leu Val Asp Lys Phe Lys Gly Thr Ser Gly Ile
   305                 310                 315                 320
   Asp Leu Thr Lys Asp Lys Met Ala Met Gln Arg Leu Arg Glu Ala Ala
                   325                 330                 335
   Glu Lys Ala Lys Ile Glu Leu Ser Ser Ser Gln Ser Thr Ser Ile Asn
               340                 345                 350
   Leu Pro Tyr Ile Thr Val Asp Ala Asp Lys Asn Pro Leu Phe Leu Asp
           355                 360                 365
   Glu Gln Leu Thr Arg Ala Glu Phe Gln Arg Ile Thr Gln Asp Leu Leu
       370                 375                 380
   Asp Arg Thr Arg Lys Pro Phe Gln Ser Val Ile Ala Asp Thr Gly Ile
   385                 390                 395                 400
   Ser Val Ser Glu Ile Asp His Val Val Leu Val Gly Gly Ser Thr Arg
                   405                 410                 415
   Met Pro Ala Val Thr Asp Leu Val Lys Glu Leu Thr Gly Gly Lys Glu
               420                 425                 430
   Pro Asn Lys Gly Val Asn Pro Asp Glu Val Val Ala Val Gly Ala Ala
           435                 440                 445
   Leu Gln Ala Gly Val Leu Lys Gly Glu Val Lys Asp Val Leu Leu Leu
       450                 455                 460
   Asp Val Thr Pro Leu Ser Leu Gly Ile Glu Thr Lys Gly Gly Val Met
   465                 470                 475                 480
   Thr Arg Leu Ile Glu Arg Asn Thr Thr Ile Pro Thr Lys Arg Ser Glu
                   485                 490                 495
   Thr Phe Thr Thr Ala Asp Asp Asn Gln Pro Ser Val Gln Ile Gln Val
               500                 505                 510
   Tyr Gln Gly Glu Arg Glu Ile Ala Ala His Asn Lys Leu Leu Gly Ser
           515                 520                 525
   Phe Glu Leu Thr Gly Ile Pro Pro Ala Pro Arg Gly Ile Pro Gln Ile
       530                 535                 540
   Glu Val Thr Phe Asp Ile Asp Ala Asn Gly Ile Val His Val Thr Ala
   545                 550                 555                 560
   Lys Asp Lys Gly Thr Gly Lys Glu Asn Thr Ile Arg Ile Gln Glu Gly
                   565                 570                 575
   Ser Gly Leu Ser Lys Glu Asp Ile Asp Arg Met Ile Lys Asp Ala Glu
               580                 585                 590
   Ala His Ala Glu Glu Asp Arg Lys Arg Arg Glu Glu Ala Asp Val Arg
           595                 600                 605
   Asn Gln Ala Glu Thr Leu Val Tyr Gln Thr Glu Lys Phe Val Lys Glu
       610                 615                 620
   Gln Arg Glu Ala Glu Gly Gly Ser Lys Val Pro Glu Asp Thr Leu Asn
   625                 630                 635                 640
   Lys Val Asp Ala Ala Val Ala Glu Ala Lys Ala Ala Leu Gly Gly Ser
                   645                 650                 655
   Asp Ile Ser Ala Ile Lys Ser Ala Met Glu Lys Leu Gly Gln Glu Ser
               660                 665                 670
   Gln Ala Leu Gly Gln Ala Ile Tyr Glu Ala Ala Gln Ala Ala Ser Gln
           675                 680                 685
   Ala Thr Gly Ala Ala His Pro Gly Gly Glu Pro Gly Gly Ala His Pro
   690 695 700
   Gly Ser Ala Asp Asp Val Val Asp Ala Glu Val Val Asp Asp Gly Arg
   705 710 715 720
   Glu Ala Lys
<210> 21
   <211> 1263
   <212> DNA
   <213> Mouse/Pseudomonas
<400> 21
   atgtggctgc agaatttact tttcctgggc attgtggtct acagcctctc agcacccacc 60
   cgctcaccca tcactgtcac ccggccttgg aagcatgtag aggccatcaa agaagccctg 120
   aacctcctgg atgacatgcc tgtcacattg aatgaagagg tagaagtcgt ctctaacgag 180
   ttctccttca agaagctaac atgtgtgcag acccgcctga agatattcga gcagggtcta 240
   cggggcaatt tcaccaaact caagggcgcc ttgaacatga cagccagcta ctaccagaca 300
   tactgccccc caactccgga aacggactgt gaaacacaag ttaccaccta tgcggatttc 360
   atagacagcc ttaaaacctt tctgactgat atcccctttg aatgcaaaaa accagtccaa 420
   aagaattctc gcctgcactt tcccgagggc ggcagcctgg ccgcgctgac cgcgcaccag 480
   gcttgccacc tgccgctgga gactttcacc cgtcatcgcc agccgcgcgg ctgggaacaa 540
   ctggagcagt gcggctatcc ggtgcagcgg ctggtcgccc tctacctggc ggcgcggctg 600
   tcgtggaacc aggtcgacca ggtgatccgc aacgccctgg ccagccccgg cagcggcggc 660
   gacctgggcg aagcgatccg cgagcagccg gagcaggccc gtctggccct gaccctggcc 720
   gccgccgaga gcgagcgctt cgtccggcag ggcaccggca acgacgaggc cggcgcggcc 780
   aacgccgacg tggtgagcct gacctgcccg gtcgccgccg gtgaatgcgc gggcccggcg 840
   gacagcggcg acgccctgct ggagcgcaac tatcccactg gcgcggagtt cctcggcgac 900
   ggcggcgacg tcagcttcag cacccgcggc acgcagaact ggatcctcat gcatggagat 960
   acacctacat tgcatgaata tatgttagat ttgcaaccag agacaactga tctctactgt 1020
   tatgagcaat taaatgacag ctcagaggag gaggatgaaa tagatggtcc agctggacaa 1080
   gcagaaccgg acagagccca ttacaatatt gtaacctttt gttgcaagtg tgactctacg 1140
   cttcggttgt gcgtacaaag cacacacgta gacattcgta ctttggaaga cctgttaatg 1200
   ggcacactag gaattgtgtg ccccatctgt tctcaggata agcttggctg ttttggcgga 1260
   tga 1263
<210> 22
   <211> 420
   <212> PRT
   <213> Mouse/Pseudomonas
<400> 22
   Met Trp Leu Gln Asn Leu Leu Phe Leu Gly Ile Val Val Tyr Ser Leu
    1               5                  10                  15
   Ser Ala Pro Thr Arg Ser Pro Ile Thr Val Thr Arg Pro Trp Lys His
               20                  25                  30
   Val Glu Ala Ile Lys Glu Ala Leu Asn Leu Leu Asp Asp Met Pro Val
           35                  40                  45
   Thr Leu Asn Glu Glu Val Glu Val Val Ser Asn Glu Phe Ser Phe Lys
       50                  55                  60
   Lys Leu Thr Cys Val Gln Thr Arg Leu Lys Ile Phe Glu Gln Gly Leu
   65                  70                  75                  80
   Arg Gly Asn Phe Thr Lys Leu Lys Gly Ala Leu Asn Met Thr Ala Ser
                   85                  90                  95
   Tyr Tyr Gln Thr Tyr Cys Pro Pro Thr Pro Glu Thr Asp Cys Glu Thr
               100                 105                 110
   Gln Val Thr Thr Tyr Ala Asp Phe Ile Asp Ser Leu Lys Thr Phe Leu
           115                 120                 125
   Thr Asp Ile Pro Phe Glu Cys Lys Lys Pro Val Gln Lys Asn Ser Arg
       130                 135                 140
   Leu His Phe Pro Glu Gly Gly Ser Leu Ala Ala Leu Thr Ala His Gln
   145                 150                 155                 160
   Ala Cys His Leu Pro Leu Glu Thr Phe Thr Arg His Arg Gln Pro Arg
                   165                 170                 175
   Gly Trp Glu Gln Leu Glu Gln Cys Gly Tyr Pro Val Gln Arg Leu Val
               180                 185                 190
   Ala Leu Tyr Leu Ala Ala Arg Leu Ser Trp Asn Gln Val Asp Gln Val
           195                 200                 205
   Ile Arg Asn Ala Leu Ala Ser Pro Gly Ser Gly Gly Asp Leu Gly Glu
       210                 215                 220
   Ala Ile Arg Glu Gln Pro Glu Gln Ala Arg Leu Ala Leu Thr Leu Ala
   225                 230                 235                 240
   Ala Ala Glu Ser Glu Arg Phe Val Arg Gln Gly Thr Gly Asn Asp Glu
                   245                 250                 255
   Ala Gly Ala Ala Asn Ala Asp Val Val Ser Leu Thr Cys Pro Val Ala
               260                 265                 270
   Ala Gly Glu Cys Ala Gly Pro Ala Asp Ser Gly Asp Ala Leu Leu Glu
           275                 280                 285
   Arg Asn Tyr Pro Thr Gly Ala Glu Phe Leu Gly Asp Gly Gly Asp Val
       290                 295                 300
   Ser Phe Ser Thr Arg Gly Thr Gln Asn Trp Ile Leu Met His Gly Asp
   305                 310                 315                 320
   Thr Pro Thr Leu His Glu Tyr Met Leu Asp Leu Gln Pro Glu Thr Thr
                   325                 330                 335
   Asp Leu Tyr Cys Tyr Glu Gln Leu Asn Asp Ser Ser Glu Glu Glu Asp
               340                 345                 350
   Glu Ile Asp Gly Pro Ala Gly Gln Ala Glu Pro Asp Arg Ala His Tyr
           355                 360                 365
   Asn Ile Val Thr Phe Cys Cys Lys Cys Asp Ser Thr Leu Arg Leu Cys
       370                 375                 380
   Val Gln Ser Thr His Val Asp Ile Arg Thr Leu Glu Asp Leu Leu Met
   385                 390                 395                 400
   Gly Thr Leu Gly Ile Val Cys Pro Ile Cys Ser Gln Asp Lys Leu Gly
                   405                 410                 415
   Cys Phe Gly Gly
               420
<210> 23
   <211> 1842
   <212> DNA
   <213> Pseudomonas
<400> 23
   gccgaggaag ccttcgacct ctggaacgaa tgcgccaaag cctgcgtgct cgacctcaag      60
   gacggcgtgc gttccagccg catgagcgtc gacccggcca tcgccgacac caacggccag     120
   ggcgtgctgc actactccat ggtcctggag ggcggcaacg acgcgctcaa gctggccatc     180
   gacaacgccc tcagcatcac cagcgacggc ctgaccatcc gcctcgaagg cggcgtcgag     240
   ccgaacaagc cggtgcgcta cagctacacg cgccaggcgc gcggcagttg gtcgctgaac     300
   tggctggtac cgatcggcca cgagaagccc tcgaacatca aggtgttcat ccacgaactg     360
   aacgccggca accagctcag ccacatgtcg ccgatctaca ccatcgagat gggcgacgag     420
   ttgctggcga agctggcgcg cgatgccacc ttcttcgtca gggcgcacga gagcaacgag     480
   atgcagccga cgctcgccat cagccatgcc ggggtcagcg tggtcatggc ccagacccag     540
   ccgcgccggg aaaagcgctg gagcgaatgg gccagcggca aggtgttgtg cctgctcgac     600
   ccgctggacg gggtctacaa ctacctcgcc cagcaacgct gcaacctcga cgatacctgg     660
   gaaggcaaga tctaccgggt gctcgccggc aacccggcga agcatgacct ggacatcaaa     720
   cccacggtca tcagtcatcg cctgcacttt cccgagggcg gcagcctggc cgcgctgacc     780
   gcgcaccagg cttgccacct gccgctggag actttcaccc gtcatcgcca gccgcgcggc     840
   tgggaacaac tggagcagtg cggctatccg gtgcagcggc tggtcgccct ctacctggcg     900
   gcgcggctgt cgtggaacca ggtcgaccag gtgatccgca acgccctggc cagccccggc     960
   agcggcggcg acctgggcga agcgatccgc gagcagccgg agcaggcccg tctggccctg    1020
   accctggccg ccgccgagag cgagcgcttc gtccggcagg gcaccggcaa cgacgaggcc    1080
   ggcgcggcca acgccgacgt ggtgagcctg acctgcccgg tcgccgccgg tgaatgcgcg    1140
   ggcccggcgg acagcggcga cgccctgctg gagcgcaact atcccactgg cgcggagttc    1200
   ctcggcgacg gcggcgacgt cagcttcagc acccgcggca cgcagaactg gacggtggag    1260
   cggctgctcc aggcgcaccg ccaactggag gagcgcggct atgtgttcgt cggctaccac    1320
   ggcaccttcc tcgaagcggc gcaaagcatc gtcttcggcg gggtgcgcgc gcgcagccag     1380
   gacctcgacg cgatctggcg cggtttctat atcgccggcg atccggcgct ggcctacggc    1440
   tacgcccagg accaggaacc cgacgcacgc ggccggatcc gcaacggtgc cctgctgcgg    1500
   gtctatgtgc cgcgctcgag cctgccgggc ttctaccgca ccagcctgac cctggccgcg    1560
   ccggaggcgg cgggcgaggt cgaacggctg atcggccatc cgctgccgct gcgcctggac    1620
   gccatcaccg gccccgagga ggaaggcggg cgcctggaga ccattctcgg ctggccgctg    1680
   gccgagcgca ccgtggtgat tccctcggcg atccccaccg acccgcgcaa cgtcggcggc    1740
   gacctcgacc cgtccagcat ccccgacaag gaacaggcga tcagcgccct gccggactac    1800
   gccagccagc ccggcaaacc gccgcgcgag gacctgaagt aa                       1842
<210> 24
   <211> 613
   <212> PRT
   <213> Pseudomonas
<400> 24
   Ala Glu Glu Ala Phe Asp Leu Trp Asn Glu Cys Ala Lys Ala Cys Val
    1               5                   10                  15
   Leu Asp Leu Lys Asp Gly Val Arg Ser Ser Arg Met Ser Val Asp Pro
               20                  25                  30
   Ala Ile Ala Asp Thr Asn Gly Gln Gly Val Leu His Tyr Ser Met Val
           35                  40                  45
   Leu Glu Gly Gly Asn Asp Ala Leu Lys Leu Ala Ile Asp Asn Ala Leu
       50                  55                  60
   Ser Ile Thr Ser Asp Gly Leu Thr Ile Arg Leu Glu Gly Gly Val Glu
   65                  70                  75                  80
   Pro Asn Lys Pro Val Arg Tyr Ser Tyr Thr Arg Gln Ala Arg Gly Ser
                   85                  90                  95
   Trp Ser Leu Asn Trp Leu Val Pro Ile Gly His Glu Lys Pro Ser Asn
               100                 105                 110
   Ile Lys Val Phe Ile His Glu Leu Asn Ala Gly Asn Gln Leu Ser His
           115                 120                 125
   Met Ser Pro Ile Tyr Thr Ile Glu Met Gly Asp Glu Leu Leu Ala Lys
       130                 135                 140
   Leu Ala Arg Asp Ala Thr Phe Phe Val Arg Ala His Glu Ser Asn Glu
   145                 150                 155                 160
   Met Gln Pro Thr Leu Ala Ile Ser His Ala Gly Val Ser Val Val Met
                   165                 170                 175
   Ala Gln Thr Gln Pro Arg Arg Glu Lys Arg Trp Ser Glu Trp Ala Ser
               180                 185                 190
   Gly Lys Val Leu Cys Leu Leu Asp Pro Leu Asp Gly Val Tyr Asn Tyr
           195                 200                 205
   Leu Ala Gln Gln Arg Cys Asn Leu Asp Asp Thr Trp Glu Gly Lys Ile
       210                 215                 220
   Tyr Arg Val Leu Ala Gly Asn Pro Ala Lys His Asp Leu Asp Ile Lys
   225                 230                 235                 240
   Pro Thr Val Ile Ser His Arg Leu His Phe Pro Glu Gly Gly Ser Leu
                   245                 250                 255
   Ala Ala Leu Thr Ala His Gln Ala Cys His Leu Pro Leu Glu Thr Phe
               260                 265                 270
   Thr Arg His Arg Gln Pro Arg Gly Trp Glu Gln Leu Glu Gln Cys Gly
           275                 280                 285
   Tyr Pro Val Gln Arg Leu Val Ala Leu Tyr Leu Ala Ala Arg Leu Ser
       290                 295                 300
   Trp Asn Gln Val Asp Gln Val Ile Arg Asn Ala Leu Ala Ser Pro Gly
   305                 310                 315                 320
   Ser Gly Gly Asp Leu Gly Glu Ala Ile Arg Glu Gln Pro Glu Gln Ala
                   325                 330                 335
   Arg Leu Ala Leu Thr Leu Ala Ala Ala Glu Ser Glu Arg Phe Val Arg
               340                 345                 350
   Gln Gly Thr Gly Asn Asp Glu Ala Gly Ala Ala Asn Ala Asp Val Val
           355                 360                 365
   Ser Leu Thr Cys Pro Val Ala Ala Gly Glu Cys Ala Gly Pro Ala Asp
       370                 375                 380
   Ser Gly Asp Ala Leu Leu Glu Arg Asn Tyr Pro Thr Gly Ala Glu Phe
   385                 390                 395                 400
   Leu Gly Asp Gly Gly Asp Val Ser Phe Ser Thr Arg Gly Thr Gln Asn
                   405                 410                 415
   Trp Thr Val Glu Arg Leu Leu Gln Ala His Arg Gln Leu Glu Glu Arg
               420                 425                 430
   Gly Tyr Val Phe Val Gly Tyr His Gly Thr Phe Leu Glu Ala Ala Gln
           435                 440                 445
   Ser Ile Val Phe Gly Gly Val Arg Ala Arg Ser Gln Asp Leu Asp Ala
       450                 455                 460
   Ile Trp Arg Gly Phe Tyr Ile Ala Gly Asp Pro Ala Leu Ala Tyr Gly
   465                 470                 475                 480
   Tyr Ala Gln Asp Gln Glu Pro Asp Ala Arg Gly Arg Ile Arg Asn Gly
                   485                 490                 495
   Ala Leu Leu Arg Val Tyr Val Pro Arg Ser Ser Leu Pro Gly Phe Tyr
               500                 505                 510
   Arg Thr Ser Leu Thr Leu Ala Ala Pro Glu Ala Ala Gly Glu Val Glu
           515                 520                 525
   Arg Leu Ile Gly His Pro Leu Pro Leu Arg Leu Asp Ala Ile Thr Gly
       530                 535                 540
   Pro Glu Glu Glu Gly Gly Arg Leu Glu Thr Ile Leu Gly Trp Pro Leu
   545                 550                 555                 560
   Ala Glu Arg Thr Val Val Ile Pro Ser Ala Ile Pro Thr Asp Pro Arg
                   565                 570                 575
   Asn Val Gly Gly Asp Leu Asp Pro Ser Ser Ile Pro Asp Lys Glu Gln
               580                 585                 590
   Ala Ile Ser Ala Leu Pro Asp Tyr Ala Ser Gln Pro Gly Lys Pro Pro
           595                 600                 605
   Arg Glu Asp Leu Lys
       610
<210> 25
   <211> 286
   <212> PRT
   <213> Human papillomavirus/Mouse
<400> 25
   Met Thr Val Leu Ala Pro Ala Trp Ser Pro Asn Ser Ser Leu Leu Leu
   1               5                   10                  15
   Leu Leu Leu Leu Leu Ser Pro Cys Leu Arg Gly Thr Pro Asp Cys Tyr
               20                  25                  30
   Phe Ser His Ser Pro Ile Ser Ser Asn Phe Lys Val Lys Phe Arg Glu
           35                  40                  45
   Leu Thr Asp His Leu Leu Lys Asp Tyr Pro Val Thr Val Ala Val Asn
        50                  55                  60
   Leu Gln Asp Glu Lys His Cys Lys Ala Leu Trp Ser Leu Phe Leu Ala
    65                  70                  75                  80
   Gln Arg Trp Ile Glu Gln Leu Lys Thr Val Ala Gly Ser Lys Met Gln
                    85                  90                  95
   Thr Leu Leu Glu Asp Val Asn Thr Glu Ile His Phe Val Thr Ser Cys
               100                 105                 110
   Thr Phe Gln Pro Leu Pro Glu Cys Leu Arg Phe Val Gln Thr Asn Ile
           115                 120                 125
   Ser His Leu Leu Lys Asp Thr Cys Thr Gln Leu Leu Ala Leu Lys Pro
       130                 135                 140
   Cys Ile Gly Lys Ala Cys Gln Asn Phe Ser Arg Cys Leu Glu Val Gln
   145                 150                 155                 160
   Cys Gln Pro Asp Ser Ser Thr Leu Leu Pro Pro Arg Ser Pro Ile Ala
                   165                 170                 175
   Leu Glu Ala Thr Glu Leu Pro Glu Pro Arg Pro Arg Gln Met His Gly
               180                 185                 190
   Asp Thr Pro Thr Leu His Glu Tyr Met Leu Asp Leu Gln Pro Glu Thr
           195                 200                 205
   Thr Asp Leu Tyr Cys Tyr Glu Gln Leu Asn Asp Ser Ser Glu Glu Glu
       210                 215                 220
   Asp Glu Ile Asp Gly Pro Ala Gly Gln Ala Glu Pro Asp Arg Ala His
   225                 230                 235                 240
   Tyr Asn Ile Val Thr Phe Cys Cys Lys Cys Asp Ser Thr Leu Arg Leu
                   245                 250                 255
   Cys Val Gln Ser Thr His Val Asp Ile Arg Thr Leu Glu Asp Leu Leu
               260                 265                 270
   Met Gly Thr Leu Gly Ile Val Cys Pro Ile Cys Ser Gln Pro
           275                 280                 285